(19) Europäisches Patentamt
European Patent Office
Office européen des brevets

(11) EP 3 973 896 B1

(12) EUROPEAN PATENT SPECIFICATION

(45) Date of publication and mention of the grant of the patent:
**17.09.2025 Bulletin 2025/38**

(21) Application number: **20917786.4**

(22) Date of filing: **21.05.2020**

(51) International Patent Classification (IPC):
***A61B 17/34*** *(2006.01)* ***A61B 8/00*** *(2006.01)*
***A61B 34/20*** *(2016.01)* ***A61B 34/10*** *(2016.01)*

(52) Cooperative Patent Classification (CPC):
**A61B 8/12; A61B 8/085; A61B 8/4254; A61B 8/461; A61B 17/34; A61B 17/3403; A61B 34/20;** A61B 8/0841; A61B 8/0883; A61B 8/5223; A61B 2017/3413; A61B 2034/107; A61B 2034/2051; A61B 2090/378; A61B 2090/3784

(86) International application number:
**PCT/CN2020/091532**

(87) International publication number:
**WO 2021/155649 (12.08.2021 Gazette 2021/32)**

# (54) PUNCTURE NEEDLE POSITIONING SYSTEM AND COMPUTER PROGRAM

SYSTEM UND COMPUTER-PROGRAMM ZUR POSITIONIERUNG EINER PUNKTIONSNADEL

SYSTÈME ET PROGRAMME D'ORDINATEUR DE POSITIONNEMENT D'AIGUILLE DE PONCTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**04.02.2020 CN 202010079873**
**04.02.2020 CN 202010079870**
**04.02.2020 CN 202020151840 U**

(43) Date of publication of application:
**30.03.2022 Bulletin 2022/13**

(73) Proprietor: **Zhao, Tianli**
**Changsha, Hunan 410011 (CN)**

(72) Inventors:
- **ZHAO, Tianli**
**Changsha, Hunan 410011 (CN)**
- **LUO, Heng**
**Changsha, Hunan 410011 (CN)**
- **HU, Shijun**
**Changsha, Hunan 410011 (CN)**
- **XIAO, Jintao**
**Changsha, Hunan 410011 (CN)**
- **KE, Jianyuan**
**Changsha, Hunan 410011 (CN)**

(74) Representative: **Canzler & Bergmeier Patentanwälte Partnerschaft mbB**
**Despag-Straße 6**
**85055 Ingolstadt (DE)**

(56) References cited:
**CN-A- 106 175 836** **CN-A- 107 789 056**
**CN-A- 108 210 024** **CN-A- 108 294 825**
**CN-A- 109 394 317** **CN-A- 109 549 689**
**CN-A- 109 620 303** **CN-A- 109 805 991**
**CN-A- 109 907 801** **CN-A- 111 134 843**
**CN-A- 111 150 461** **JP-A- 2012 035 010**
**US-A1- 2009 137 907** **US-A1- 2010 298 704**
**US-A1- 2018 168 537** **US-B1- 6 216 029**
**US-B2- 9 597 054**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 973 896 B1

## Description

## FIELD OF TECHNOLOGY

The present invention relates to surgical positioning, in particular to a puncture needle positioning system and a computer-readable storage medium comprising instructions which, when executed by a computer, cause the computer to carry out a method for positioning a puncture needle.

## BACKGROUND TECHNOLOGY

With the highly advanced medical technology, interventional techniques have become the third effective clinical treatment after drug therapy and surgery. The interventional pathway is the key to successful treatment delivery. The ideal interventional pathway should be close to the lesion, convenient for manipulating the instruments, and at the same time less invasive. To facilitate bleeding control, transfemoral veins and femoral arteries are the primary interventional pathways for structural heart disease operations. However, these interventional pathways are far from the heart and have a tortuous travel path, thus not facilitating precise intracardiac interventions and limiting the indications for intervention. In the last decade, the epicardial route for intracardiac interventions has been widely used in clinical practice. Due to the closer proximity of the intervention to the heart and the precise visualization under ultrasound guidance, this technique has not only broadened the indications for the procedure, but also achieved better results than conventional interventions in the treatment of certain diseases such as ventricular septal defects. Therefore, the transcatheter route should be the most ideal interventional access for structural heart disease. The epicardial route can be formed by either incision of the chest wall or transthoracic puncture. Undoubtedly, transthoracic puncture is more advantageous, as it is less invasive and results no surgical incision scar. However, the key to this technique is to choose the precise puncture site and route to ensure a one-time success. Repeated punctures of the heart can lead to serious complications such as pericardial tamponade due to bleeding at the puncture site. For this reason, preoperative transthoracic ultrasound is required to plan the puncture path. However, during the actual clinical operation, the operator can only reconstruct the spatial position of the puncture needle in his mind based on his personal experience, and there is a high rate of miss-election in terms of puncture points and angles. Therefore, it is difficult to promote the application of this technique in clinical practice. A highly accurate navigation system has become an urgent need if transthoracic puncture interventions are to be realized. Transthoracic puncture intracardiac interventional therapy is an emerging interventional technology. Its interventional paths are short, straight, and close to lesions. It makes it easy for medical staff to precisely manipulate interventional instruments. It is suitable for a variety of operations for structural heart disease, especially for post-surgery complications. The key point of this technology is to select precise puncture sites and paths to ensure success at first try. If the heart is punctured repeatedly, serious complications such as pericardial tamponade due to bleeding from the puncture point(s) will result. For this reason, preoperative transthoracic ultrasound scan is needed to plan a puncture path and determine puncture sites and spatial position of the puncture needle. Generally speaking, after the ultrasound scan has determined the lesion's location, the best puncture path is along the axis of the fan-shaped ultrasound section that goes through the lesion. However, in reality, the operator can only reconstruct the spatial position of the puncture needle in their mind based on their personal experience. Therefore, there is a high rate of miss-election in terms of puncture points and angles, which leads to serious complications. In order to improve the success rate of puncturing, it is necessary to record and visualize the planned puncture path preoperatively for intraoperative reference. Therefore, a method and apparatus for obtaining the axis of an ultrasound probe as projected onto an ultrasound section has become an urgent need.

US 2010/0298704 A1 discloses an ultrasound system with an ultrasound transducer equipped with a position marker, and a needle also equipped with a position marker. These markers allow the determination of the position and orientation of the transducer and needle. A display shows an ultrasound image acquired via the transducer and a graphical element representative of a projection of the longitudinal axis of the needle onto a plane of the ultrasound image. The quality of the position and orientation information from the position markers is monitored, and if it falls below a quality threshold, the display indicates this fact.

US 6 216 029 B1 discloses a method for free-hand directing of a needle towards a target located within a body volume. The method includes producing and transmitting an image of the target via an imaging detector to a display screen that communicates with a position sensing controller. It involves sensing spatial orientation data of the imaging detector relative to a reference, displaying the spatial orientation data on the screen, positioning the needle to point at the target, transmitting the needle's spatial orientation data to the controller, and indicating on the display screen the trajectory for needle insertion into the body volume towards the target as shown on the screen.

CN 108210024 A discloses a surgery navigation method and system, involving optical positioning on an ultrasonic probe with a configured optical marking part and on a puncture needle with a similar optical marking part. The method includes the acquisition of ultrasonic imaging data by the probe and the generation of the position and trajectory of the puncture needle in the ultrasonic image based on this optical positioning information. The system includes

a positioning module, an ultrasonic imaging data acquisition module, and a puncture needle trajectory generating module. The disclosed method and system provide the advantage of real-time display of the puncture needle's position and trajectory within the ultrasonic image, thereby eliminating the risks associated with X-ray exposure and improving the clarity and nonvisual guidance over traditional ultrasonic guidance, allowing doctors to visually and efficiently determine the puncture direction during surgery.

CN 109805991 A relates to the field of vessel puncture, particularly providing a vessel puncture-assisting control method and device. The method involves acquiring ultrasonic images of the vascular minor axis section of a target region, identifying boundary information from these images, and extracting puncture path data with a puncture target point as a base. It requires obtaining a displacement parameter to adjust a puncture needle from its current position to the puncture path, and then driving the needle to the target point along this path. The invention aims to ensure accurate needle placement under the out-of-plane puncture technique, effectively reducing associated complications and increasing the one-time success rate of puncture procedures.

## CONTENT OF INVENTION

The invention is defined in the independent claims 1 and 11. In view of the above-described shortcomings of the prior art, the technical problem to be solved by the present invention is to provide a puncture needle positioning system and a computer-readable storage medium comprising instructions which, when executed by a computer, cause the computer to carry out a method for positioning a puncture needle.

To achieve these purposes, the present invention provides a puncture needle positioning system.

The present disclosure further describes a non-claimed device, including: a memory, on which computer programs are stored; and a processor, for executing the computer programs stored on the memory, wherein when executed the computer programs perform the puncture needle positioning method described herein.

The present invention further provides a computer-readable storage medium, comprising instructions which, when executed by a computer, cause the computer to carry out the puncture needle positioning method described herein.

The present disclosure further describes a non-claimed method for obtaining the axis of an ultrasound probe as projected onto an ultrasound section, comprising:

S201' obtaining the coordinates of three non-collinear sampling points on a virtual ultrasound section F1 in a virtual coordinate system, $P_1(p_x^1, p_y^1, p_z^1)$, $P_2(p_x^2, p_y^2, p_z^2)$, $P_3(p_x^3, p_y^3, p_z^3)$, and the coordinates of three non-collinear sampling points on an axial plane F2 of a virtual ultrasound probe, $Q_1(q_x^1, q_y^1, q_z^1)$, $Q_2(q_x^2, q_y^2, q_z^2)$, $Q_3(q_x^3, q_y^3, q_z^3)$;

S202' obtaining a transformation matrix $T_1$ corresponding to the transformation from the axial plane $F_2$ of the virtual ultrasound probe to a plane $F_2'$, wherein the plane F2' is obtained by translating the axial plane $F_2$ of the virtual ultrasound probe until any of the sampling points on the axial plane $F_2$ of the virtual ultrasound probe coincides with the origin of the coordinate system;

S203' obtaining a transformation matrix $T_2$ corresponding to the transformation from the plane $F_2'$ to a plane $F_2''$, wherein the plane $F_2''$ is obtained by rotating the plane $F_2'$ around the origin of the coordinate system until its unit normal vector coincides with the unit normal vector $\overrightarrow{n1}$ of the virtual ultrasound section $F_1$;

S204' obtaining a transformation matrix $T_3$ corresponding to the transformation from the plane $F_2''$ to a plane $F_2'''$, wherein the $F_2'''$ is obtained by translating the plane $F_2''$ until the sampling point coincident with the origin of the coordinate system at S202' returns to its initial position; and

S205' obtaining a transformation matrix $T_4$ corresponding to the transformation from the plane $F_2'''$ to a plane $F_2''''$, wherein the plane $F_2''''$ is obtained by first obtaining the distance between the plane $F_2'''$ and the virtual ultrasound section $F_1$, and then translating the plane $F_2'''$ along its unit normal vector by a corresponding vector $\vec{d}$ to coincide with the virtual ultrasound section $F_1$, wherein the coordinates of the projection of the axis of the ultrasound probe onto the ultrasound section can be obtained by using a coordinate transformation formula and the coordinates of the axis of the corresponding ultrasound probe on the axial plane $F_2$ of the virtual ultrasound probe.

The present disclosure further describes a non-claimed device for obtaining the axis of an ultrasound probe as projected onto an ultrasound section, comprising:

a sampling module, used to obtain the coordinates of three non-collinear sampling points on a virtual ultrasound section $F_1$ in a virtual coordinate system $P_1(p_x^1, p_y^1, p_z^1)$, $P_2(p_x^2, p_y^2, p_z^2)$, $P_3(p_x^3, p_y^3, p_z^3)$, and the coordinates of three non-collinear sampling points on an axial plane $F_2$ of a virtual ultrasound probe $Q_1(q_x^1, q_y^1, q_z^1)$, $Q_2(q_x^2, q_y^2, q_z^2)$, $Q_3(q_x^3, q_y^3, q_z^3)$;

a first translation module, used to obtain a transformation matrix $T_1$ corresponding to the transformation from the axial plane $F_2$ of the virtual ultrasound probe to a plane $F_2'$, wherein the plane F2' is obtained by translating the axial plane $F_2$ of the virtual ultrasound probe until any sampling point on $F_2$ is coincident with the origin of the virtual coordinate system;

a rotation module, used to obtain a transformation matrix $T_2$ corresponding to the transformation from the plane $F_2'$ to a plane $F_2''$, wherein the plane $F_2''$ is obtained by rotating the plane $F_2'$ around the origin of the coordinate system until its unit normal vector coincides with the unit normal vector $\overrightarrow{n1}$ of the virtual ultrasound section $F_1$;

a second translation module, used to obtain a transformation matrix $T_3$ corresponding to the transformation from the plane $F_2''$ to a plane $F_2'''$, wherein the $F_2'''$ is obtained by translating the plane $F_2''$ until the sampling point coincident with the origin of the coordinate system in the first translation module returns to its initial position; and

a third translation module, used to obtain a transformation matrix $T_4$ corresponding to the transformation from the plane $F_2'''$ to a plane $F_2''''$, wherein the plane $F_2''''$ is obtained by first obtaining the distance between the plane $F_2'''$ and the virtual ultrasound section $F_1$, and then translating the plane $F_2'''$ along its unit normal vector by a corresponding vector $\vec{d}$ to coincide with the virtual ultrasound section $F_1$, wherein the coordinates of the projection of the axis of the ultrasound probe onto the ultrasound section can be obtained by using a coordinate transformation formula and the coordinates of the axis of the corresponding ultrasound probe on the axial plane $F_2$ of the virtual ultrasound probe.

The present disclosure also describes a non-claimed computer-readable storage medium with computer programs stored thereon, that when executed by a processor, perform the method for obtaining the axis of an ultrasound probe as projected onto an ultrasound section as described herein.

The present disclosure further describes a non-claimed device, including: a memory, a processor; wherein a computer program is stored in the memory, and the processor is configured to execute the computer program stored in the memory. When the computer program is executed, the method for obtaining the axis of an ultrasound probe as projected onto an ultrasound section as described herein is realized.

The puncture needle positioning system provided by the present disclosure can greatly improve the accuracy of transthoracic puncture. Not only can the invention effectively avoid the complications caused by repeated puncture, but also greatly shorten the puncture time during surgery. Adopting the first positioning devices and the second positioning devices greatly improves the conventional single ultrasound positioning and navigation technique, and can digitally analyze all position information. Various kinds of position information in the magnetic field can be obtained through the first positioning devices and the second positioning devices, and transformed into three-dimensional information displayed in a three-dimensional coordinate system. The system provides accurate puncture path planning tailored for different individuals, and realizes individualized and precise medical treatment for patients, which will greatly reduce surgery-related complications and make it possible for transthoracic puncture interventional treatment to become a conventional treatment method.

The method of obtaining the axis of the virtual ultrasound probe as projected onto the ultrasound section provided by the present disclosure and being carried out by a computer, when the computer executes the instructions comprised on a computer-readable storage medium, can display the ultrasound section of the lesion and the plane where the positioning devices of the virtual ultrasound probe are located in the same virtual coordinate system, in order to obtain the planned path. The present disclosure provides a standardized technical scheme for transthoracic puncture intracardiac interventional therapy. Through the implementation of the scheme of the present disclosure, the preoperative planned puncture path can be accurately recorded and visualized, to provide a reliable guidance for the medical staff during operation, and effectively reduces the medical staff's error rate in terms of selecting the puncture sites and angles. The present disclosure can not only improve the puncture success rate, but also effectively prevent the occurrence of complications and improve the safety of the operation.

## DESCRIPTION OF THE DRAWINGS

Fig. 1 shows an application scenario of a puncture needle positioning system of the present disclosure;

Fig. 2 is a schematic diagram of the structure of a first probe of the present disclosure.

Fig. 3 is a schematic diagram of a fastener structure of an ultrasound probe of the present disclosure.

Fig. 4 is a schematic diagram of the structures of a puncture needle and a puncture needle fastener of the present disclosure.

Fig. 5 is a schematic flow chart illustrating a puncture needle positioning method of the present disclosure.

Fig. 6 is a flow chart illustrating a method for converting coordinates when projecting the axis of a first probe onto an ultrasound section according to some embodiments of the present disclosure.

Fig. 7 is a schematic diagram showing visual interfaces of a puncture needle positioning system according to some embodiments of the present disclosure.

Fig. 8 is a flow chart illustrating steps of determining if a virtual puncture needle and a planned path coincide according to some embodiments of the present disclosure.

Fig. 9 is a flow chart illustrating a method for obtaining coordinates of a projection of an axis of an ultrasound probe on an ultrasound section according to some embodiments of the present disclosure

Fig. 10 is a schematic diagram of the structure of an ultrasound probe according to some embodiments of the present disclosure.

**Reference Numerals**

| | |
|---|---|
| 1 | Ultrasound Unit |
| 11 | First probe |
| 12 | Second probe |
| 2 | Puncture needle unit |
| 3 | Processing and display unit |
| 4 | First positioning device |
| 5 | Second positioning device |
| 6 | Probe fastener |
| 7 | Puncture needle fastener |
| 8 | Ultrasound probe |
| 9 | Positioning device |

**SPECIFIC IMPLEMENTATIONS**

The following describes the implementation of the present disclosure through specific examples, and those skilled in the art can easily understand other advantages and effects of the present disclosure from the content disclosed in this specification. The present disclosure may also be implemented or applied through other different specific embodiments. It should be noted that the following embodiments and the features in the embodiments may be combined with each other if no conflict will result.

It should be noted that the drawings provided in this disclosure only illustrate the basic concept of the present disclosure in a schematic way, so the drawings only show the components related to the present disclosure. The drawings are not necessarily drawn according to the number, shape and size of the components in actual implementation; during the actual implementation, the type, quantity and proportion of each component may be changed as needed, and the components' layout may also be more complicated.

As shown in Fig. 1, the present invention provides a puncture needle positioning system. As shown in Fig. 1, the system includes: an ultrasound unit 1, a puncture needle unit 2, a processing and display unit 3. The ultrasound unit 1 includes a first probe 11 for providing an ultrasound section of a lesion. As shown in Fig. 2, the first probe 11 is provided with a plurality of first positioning devices 4 for providing coordinate information of the first probe 11. The puncture needle unit 2 includes a puncture needle. As shown in Fig. 4, the puncture needle is provided with a plurality of second positioning devices 5 for providing coordinate information of the puncture needle. The processing and display unit 3 is in communicative connection with the ultrasound unit 1, each of the first positioning devices 4, and each of the second positioning devices 5, and the processing and display unit 3 is used to: obtain the ultrasound section of the lesion and display it in a virtual coordinate system; obtain the coordinate information of the first probe 11 when it is acquiring the ultrasound section of the lesion, and display the coordinate information in the virtual coordinate system; determine a planned path L1 in the virtual coordinate system; obtain the coordinate information of the second positioning device 5 on the puncture needle in the virtual coordinate system; display the axis L2 of a virtual puncture needle and the apex C2 of the virtual puncture needle; in a virtual coordinate system, compare the axis L2 of the virtual puncture needle and the planned path L1 to see if they coincide. When the axis L2 of the virtual puncture needle coincides with the planned path L1, the puncture needle is

ready to be used for puncturing.

The ultrasound unit 1 may be an ultrasound machine with multiple probes. As shown in Fig. 2, the ultrasound unit 1 includes a first probe 11 for providing an ultrasound section of the lesion, and the first probe 11 is a transthoracic cardiac ultrasound probe. The first probe can be a conventional ultrasound probe, such as Philips EPIQ7C. When in use, the first probe 11 scans the intercostal space of the human body for any lesion such as a ventricular septal defect, to obtain an ultrasound section of the lesion. When the lesion appears on the ultrasound section then it is the ultrasound section of the lesion.

The first probe 11 is provided with a plurality of first positioning devices 4 for providing coordinate information of the first probe 11. The coordinate information of the first probe 11 includes the coordinate information of several position points on the first probe 11. The position points are selected to facilitate the determination of the axis of the first probe 11. In one embodiment, as shown in Fig. 2, the first probe 11 is provided with three first positioning devices 4, and the three first positioning devices 4 are located on the same plane which coincides with the axial plane of the first probe 11, wherein two of the first positioning devices 4 are on the cutting plane of a cross section of the first probe 11, the cross section is perpendicular to the axial plane, and the three first positioning devices 4 constitute the vertices of a right triangle. This arrangement is for determining the axis of the first probe 11 by each of the first positioning devices 4. The first positioning devices 4 are selected from sensors. Normally, the sensors are used in conjunction with a magnetic source and a magnetic locator, with the sensors in communicative connection with the magnetic locator, and the magnetic source in communicative connection with the magnetic locator. When in use, the magnetic source is located near an operating table. In one embodiment, 3DGuidance trakSTAR of the Canadian company Northern Digital Incorporated is used. The 3DGuidance trakSTAR device includes the sensor, magnetic source, and magnetic locator used in the present disclosure. Further, for the installation stability of the first probe 11 and the first positioning devices 4, the first probe 11 is provided with a probe fastener 6 that is matched with the first probe 11. The probe fastener 6 is shown in Fig. 3, and is provided with three first insertion holes 3 for installing the first positioning devices 4, and the positions of the three first insertion holes match those of the sensors $Q_1$, $Q_2$ and $Q_3$. The three sensors $Q_1$, $Q_2$ and $Q_3$ obtain their coordinate information, which is then recorded and provided as the coordinate information of the first probe 11. Since the three sensors are located at the position points of the first probe 11, the coordinate information of the first probe 11 can be obtained by them.

The ultrasound unit 1 also includes a second probe 12 for monitoring the whole puncturing process. The second probe 12 is in communicative connection with the processing and display unit 3, and the image scanned by the second probe 12 is displayed by the processing and display unit 3. The second probe 12 is a transesophageal ultrasound probe, and the second probe 12 can be an existing conventional ultrasound probe. For example, the second probe 12 can be Philips X7-2T. The second probe 12 provides real-time monitoring and evaluation for the puncturing, which further ensures the safety of intracardiac intervention.

The puncture needle unit 2 includes a puncture needle, and a plurality of second positioning devices 5 for providing coordinate information of the puncture needle are provided on the puncture needle. The coordinate information of the puncture needle includes the coordinate information of the position points on the puncture needle. The position points are selected to facilitate the determination of the position of the axis of the puncture needle. In one embodiment, the puncture needle is provided with two second positioning devices 5, and the two second positioning devices 5 are located at different positions on the axis of the puncture needle. This arrangement is for determining the axis of the puncture needle through the second positioning devices 5. The second positioning devices 5 are selected from sensors. Normally, the sensors are used in conjunction with a magnetic source and a magnetic locator, and the magnetic source and sensor are respectively communicative connected to the magnetic locator. When in use, the magnetic source is located near the operating table. In one embodiment, 3DGuidance trakSTAR can be used. 3DGuidance trakSTAR includes the sensor, magnetic source and magnetic locator used in the present disclosure. Further, for the installation stability of the puncture needle and the second positioning devices 5, the puncture needle is provided with a puncture needle fastener 7, matched with the puncture needle. As shown in Fig. 4, the puncture needle fastener 7 is provided with two second insertion holes for installing the second positioning device 5, and the positions of the two second insertion holes match those of sensor B1 and B2. It can be seen from the above that the two sensors B1 and B2 can obtain and record their own coordinate information, which is then provided as the coordinate information of the puncture needle. Since the two sensors B1 and B2 are located on the puncture needle at multiple locations, the coordinate information of the puncture needle can be obtained.

The processing and display unit 3 includes a processor and a display. The processor may be a server or a general-purpose processor, including a central processing unit (CPU), and a network processor (NP). It can also be a Digital Signal Processing (DSP), Application Specific Integrated Circuit (ASIC), Field-Programmable Gate Array (Field-Programmable Gate Array, FPGA), other programming logic devices, discrete gates or transistor logic devices, or discrete hardware components. The processor is in communicative connection with the display. The processor is respectively communicatively connected to the ultrasound unit 1, each of the first positioning devices 4, and each of the second positioning devices 5. The processor is communicatively connected to the display for display. More specifically, the processor is communicatively connected to the first probe 11 and the second probe 12.

More specifically, the processing and display unit 3 includes the following modules:

a first data acquisition module, used to acquire the ultrasound section of the lesion and display it in the virtual coordinate system, and used to acquire the coordinate information of the first probe 11 when the latter is acquiring the ultrasound section of the lesion and display the coordinate information of the first probe 11 in the virtual coordinate system;
a planned path determination module, used to determine the planned path L1 in the virtual coordinate system;
a second data acquisition module, used to acquire the coordinate information of the second positioning devices 5 on the puncture needle in the virtual coordinate system and display the axis L2 of the virtual puncture needle and the vertex C2 of the virtual puncture needle; and
a comparison module, used to compare the axis L2 of the virtual puncture needle and the planned path L1 in the virtual coordinate system to see if they are coincident.

Specifically, in the first data acquisition module, the ultrasound section of the lesion is obtained by scanning the lesion by the first probe 11, and when a lesion appears on the ultrasound section then it is the ultrasound section of the lesion.

The position of the ultrasound section of the lesion as displayed in the virtual coordinate system is not limited, and the selection of the origin of the virtual coordinate system is not limited. In order to facilitate data processing, in a preferred embodiment, the lesion ultrasound section is fan-shaped, and the vertex of the ultrasound section serves as the origin of the virtual coordinate system.

The first probe 11 can provide its own coordinate information through the first positioning devices 4. The coordinate information of the first positioning devices 4 matches coordinate information of the virtual coordinate system. The position of each first positioning device 4 in the virtual coordinate system can be used to represent the first probe 11 in the virtual coordinate system as the virtual first probe. In order to obtain the planned path more conveniently, the position of the axis of the first probe 11 in the virtual coordinate system needs to be determined first. In one embodiment, the first positioning devices 4 of the first probe 11 are all provided on a plane that coincides with the axis of the first probe 11 so that it is convenient to determine the coordinate information of the axis of the first probe 11. In the first probe 11 as shown in Fig. 2, the positions of the three sensors $Q_1$, $Q_2$ and $Q_3$ in the virtual coordinate system can be represented as the position of the first probe 11 in the virtual coordinate system, i.e., the virtual first probe. The axis of the first probe 11 lies in the plane containing Points $Q_1$, $Q_2$ and $Q_3$, and perpendicularly bisects Line Segment $Q_1$-$Q_2$.

In order to facilitate subsequent steps for determining the planned path, in a preferred embodiment, the point of the lesion to be punctured at and sampled from ("puncture sampling point") can be positioned on the axis of the ultrasound section of the lesion.

In the planed path determination module, according to the coordinate information of the first probe 11 in the virtual coordinate system, the position of the axis of the first probe 11 as projected to the ultrasound section of the lesion is obtained by planar matrix conversion, so as to determine the planned path L1.

The ultrasound section of the lesion is fan-shaped. In general, the line passing through the apex of the ultrasound section and the puncture sampling point of the lesion can serve as the actual planed path. When the puncture sampling point is on the axis of the ultrasound section of the lesion, the axis of the ultrasound section of the lesion can be used as the actual planned path. In the real world, the axis of the first probe 11 and the ultrasound section of the lesion should be on the same plane, the apexes of the first probe 11 and the ultrasound section of the lesion coincide, and the axis of the first probe 11 and the axis of the ultrasound section of the lesion coincide. Therefore, the planned path can be determined as soon as the position of the axis of the first probe 11 is determined.

However, the relative positions of the ultrasound section of the lesion coincide and the axis of the first probe 11 in the virtual coordinate system do not match the actual relative positions of the two, so it is needed to project the axis of the first probe 11 to the ultrasound section of the lesion coincide by conversion.

Specifically, the following transformation formula I may be used to perform planar matrix conversion, and obtain the transformed coordinate information of the projection of the axis of the first probe 11 onto the ultrasound section of the lesion:

$(x', y', z', 1) = T(x, y, z, 1)^T$ (transformation formula I), wherein $(x' y', z')^T$ is the transformed coordinate information, and $(x, y, z)^T$ is the coordinate information before the transformation.
wherein $T = T_4 T_3 T_2 T_1$, wherein T is the transformation matrix corresponding to the projection of the axial plane of the virtual first probe onto the ultrasound section of the lesion in the virtual coordinate system.

$$T_4 = \begin{bmatrix} 1 & 0 & 0 & d_x \\ 0 & 1 & 0 & d_y \\ 0 & 0 & 1 & d_z \\ 0 & 0 & 0 & 1 \end{bmatrix}$$

$$\vec{d} = \left(-\overrightarrow{P_1 Q_1'} \cdot \vec{n_1}\right)\vec{n_1},$$

$$\vec{d} = \left(d_x, d_y, d_z\right),$$

$$Q_1' = T_3 T_2 T_1 Q_1;$$

$$T_3 = \begin{bmatrix} 1 & 0 & 0 & q_x^1 \\ 0 & 1 & 0 & q_y^1 \\ 0 & 0 & 1 & q_z^1 \\ 0 & 0 & 0 & 1 \end{bmatrix}$$

$$T_2 = \begin{bmatrix} 1 & 0 & 0 & 0 \\ 0 & \cos\theta_x^1 & \sin\theta_x^1 & 0 \\ 0 & -\sin\theta_x^1 & \cos\theta_x^1 & 0 \\ 0 & 0 & 0 & 1 \end{bmatrix} \begin{bmatrix} \cos\theta_y^1 & 0 & \sin\theta_y^1 & 0 \\ 0 & 1 & 0 & 0 \\ -\sin\theta_y^1 & 0 & \cos\theta_y^1 & 0 \\ 0 & 0 & 0 & 1 \end{bmatrix} \begin{bmatrix} \cos\theta_y^2 & 0 & -\sin\theta_y^2 & 0 \\ 0 & 1 & 0 & 0 \\ \sin\theta_y^2 & 0 & \cos\theta_y^2 & 0 \\ 0 & 0 & 0 & 1 \end{bmatrix} \begin{bmatrix} 1 & 0 & 0 & 0 \\ 0 & \cos\theta_x^2 & -\sin\theta_x^2 & 0 \\ 0 & \sin\theta_x^2 & \cos\theta_x^2 & 0 \\ 0 & 0 & 0 & 1 \end{bmatrix}$$

$$\cos\theta_x^2 = \frac{n_z^2}{\sqrt{\left(n_y^2\right)^2 + \left(n_z^2\right)^2}}, \sin\theta_x^2 = \frac{n_y^2}{\sqrt{\left(n_y^2\right)^2 + \left(n_z^2\right)^2}};$$

$$\cos\theta_y^2 = \frac{\sqrt{\left(n_y^2\right)^2 + \left(n_z^2\right)^2}}{\sqrt{\left(n_x^2\right)^2 + \left(n_y^2\right)^2 + \left(n_z^2\right)^2}}, \sin\theta_y^2 = \frac{n_x^2}{\sqrt{\left(n_x^2\right)^2 + \left(n_y^2\right)^2 + \left(n_z^2\right)^2}};$$

$$\cos\theta_x^1 = \frac{n_z^1}{\sqrt{\left(n_y^1\right)^2 + \left(n_z^1\right)^2}}, \sin\theta_x^1 = \frac{n_y^1}{\sqrt{\left(n_y^1\right)^2 + \left(n_z^1\right)^2}}$$

$$\cos\theta_y^1 = \frac{\sqrt{\left(n_y^1\right)^2 + \left(n_z^1\right)^2}}{\sqrt{\left(n_x^1\right)^2 + \left(n_y^1\right)^2 + \left(n_z^1\right)^2}}, \sin\theta_y^1 = \frac{n_x^1}{\sqrt{\left(n_x^1\right)^2 + \left(n_y^1\right)^2 + \left(n_z^1\right)^2}} 。$$

$$\vec{n}_1 = \left(n_x^1, n_y^1, n_z^1\right), \quad \vec{n}_2 = \left(n_x^2, n_y^2, n_z^2\right)$$

$$\vec{n}_1 = \frac{\overrightarrow{P_1P_2} \times \overrightarrow{P_1P_3}}{\left|\overrightarrow{P_1P_2}\right|\left|\overrightarrow{P_1P_3}\right|}, \quad \vec{n}_2 = \frac{\overrightarrow{Q_1Q_2} \times \overrightarrow{Q_1Q_3}}{\left|\overrightarrow{Q_1Q_2}\right|\left|\overrightarrow{Q_1Q_3}\right|}$$

$$T_1 = \begin{bmatrix} 1 & 0 & 0 & -q_x^1 \\ 0 & 1 & 0 & -q_y^1 \\ 0 & 0 & 1 & -q_z^1 \\ 0 & 0 & 0 & 1 \end{bmatrix}$$

$P_1$, $P_2$, $P_3$ are three non-collinear sampling points on the virtual ultrasound section F1 of the lesion, and $Q_1$, $Q_2$, $Q_3$ are three non-collinear points on a plane containing the axis of a virtual first probe (hereinafter, the axial plane $F_2$ of the virtual first probe), $P_1\left(p_x^1, p_y^1, p_z^1\right)$, $P_2\left(p_x^2, p_y^2, p_z^2\right)$, $P_3\left(p_x^3, p_y^3, p_z^3\right)$, $Q_1\left(q_x^1, q_y^1, q_z^1\right)$, $Q_2\left(q_x^2, q_y^2, q_z^2\right)$, $Q_3\left(q_x^3, q_y^3, q_z^3\right)$ are the coordinates of $P_1$, $P_2$, $P_3$, $Q_1$, $Q_2$, and $Q_3$ respectively.

$\vec{n}_1$ is a unit normal vector of the virtual ultrasound section $F_1$, and $\vec{n}_2$ is a unit normal vector of the axial plane $F_2$ of the virtual first probe.

The axial plane $F_2$ of the virtual first probe is translated until $Q_1$ coincides with the origin of the virtual coordinate system, thereby obtaining the plane $F_2$'. Then the unit normal vector of the plane $F_2$' is rotated around the origin until it coincides with the unit normal vector $\vec{n}_1$ of the virtual ultrasound section $F_1$; the angle at which the unit normal vector of the plane $F_2$' has rotated counterclockwise around the x axis accordingly is $\theta_x^2$ or $2\pi - \theta_x^1$, and the angle at which the unit normal vector of the plane $F_2$' has rotated counterclockwise around they axis accordingly is $\theta_y^1$ or $2\pi - \theta_y^2$.

A planar matrix conversion device for projecting the axis of the first probe 11 onto the ultrasound section includes:

a sampling module, used to obtain the coordinates of three non-collinear sampling points on the virtual ultrasound section $F_1$ in a virtual coordinate system *OXYZ* $P_1\left(p_x^1, p_y^1, p_z^1\right)$, $P_2\left(p_x^2, p_y^2, p_z^2\right)$, $P_3\left(p_x^3, p_y^3, p_z^3\right)$, and the coordinates of three non-collinear sampling points on an axial plane $F_2$ of the virtual first probe, $Q_1\left(q_x^1, q_y^1, q_z^1\right)$, $Q_2\left(q_x^2, q_y^2, q_z^2\right)$, $Q_3\left(q_x^3, q_y^3, q_z^3\right)$;

a first translation module, used to obtain a transformation matrix $T_1$ corresponding to the transformation from the axial plane $F_2$ of the virtual first probe to a plane $F_2$', wherein the plane F2' is obtained by translating the axial plane $F_2$ of the virtual first probe until any sampling point on $F_2$ is coincident with the origin of the virtual coordinate system;

a rotation module, used to obtain a transformation matrix $T_2$ corresponding to the transformation from the plane $F_2$' to a plane $F_2$", wherein the plane $F_2$" is obtained by rotating the plane $F_2$' around the origin of the coordinate system until its

unit normal vector coincides with the unit normal vector of the virtual ultrasound section $F_1$;
a second translation module, used to obtain a transformation matrix $T_3$ corresponding to the transformation from the plane $F_2''$ to a plane $F_2'''$, wherein the $F_2'''$ is obtained by translating the plane $F_2''$ until the sampling point coincident with the origin of the coordinate system in the first translation module returns to its initial position;
a third translation module, used to obtain a transformation matrix $T_4$ corresponding to the transformation from the plane $F_2'''$ to a plane $F_2''''$, wherein the plane $F_2''''$ is obtained by first obtaining the distance between the plane $F_2'''$ and the virtual ultrasound section $F_1$, and then translating the plane $F_2'''$ along its unit normal vector (which is equal to the unit normal vector of $F_1$) by a corresponding vector $\vec{d}$ to coincide with the virtual ultrasound section $F_1$, wherein the coordinates of the projection of the axis of the ultrasound probe onto the virtual ultrasound section can be obtained by using a coordinate transformation formula and the coordinates of the axis of the ultrasound probe on the plane $F_2$.

Specifically:
In the sampling module, the virtual ultrasound section $F_1$ is a virtual plane displayed in the virtual coordinate system, which contains an ultrasound section of the lesion, obtained by scanning the lesion with the first probe 11. The axial plane $F_2$ of the virtual first probe is the axial plane of the first probe 11 as shown in the virtual coordinates system when the first probe 11 is scanning the lesion to obtain the ultrasound section of the lesion. The axial plane of the virtual first probe can be located according to the sensors on the first probe 11.

In a preferred embodiment, the virtual coordinate system also displays the apex C1 of the fan-shaped ultrasound section of the lesion to assist in determining the puncture point. Specifically, the virtual ultrasound section $F_1$ is fan-shaped, in which case the point where the apex of the first probe 11 is in contact with the patient's chest wall is represented in the virtual coordinate system as the apex C1 of the fan-shaped ultrasound section of the lesion.

In one embodiment, the axis and axial plane of the first probe 11 can be determined according to the positions of the first positioning devices 4 on the first probe 11. In a preferred embodiment, in order to facilitate the determination of the axis of the first probe 11, the first positioning devices 4 are located on the axial plane of the first probe 11.

The first positioning devices 4 may be sensors, and three non-collinear sensors $Q_1$, $Q_2$ and $Q_3$ as shown in Fig. 2 are provided on the axial plane of the first probe 11 to locate the axis and axial plane of the first probe 11. The three non-collinear sensors $Q_1$, $Q_2$ and $Q_3$ constitute the vertices of a right triangle for determining the axis of the first probe. The axis of the first probe 11 perpendicularly bisects Line Segment $Q_1$-$Q_2$.

In a preferred embodiment, the virtual three-dimensional coordinate system uses the vertex C1 of the fan-shaped section $F_1$ of the lesion as the origin of the virtual three-dimensional coordinate system *OXYZ.*

In another preferred embodiment, in the first translation module, the axial plane $F_2$ of the virtual first probe is translated until Point $Q_1$ coincides with the origin of the coordinate system.

The vertex C1 of the fan-shaped ultrasonic section of the lesion is taken as the origin of the coordinate system *OXYZ,* and the axial plane $F_2$ of the virtual first probe is translated until $Q_1$ coincides with the origin.

$$\begin{bmatrix} x' \\ y' \\ z' \\ 1 \end{bmatrix} = \begin{bmatrix} 1 & 0 & 0 & -q_x^1 \\ 0 & 1 & 0 & -q_y^1 \\ 0 & 0 & 1 & -q_z^1 \\ 0 & 0 & 0 & 1 \end{bmatrix} \begin{bmatrix} x \\ y \\ z \\ 1 \end{bmatrix}$$

wherein:

$$T_1 = \begin{bmatrix} 1 & 0 & 0 & -q_x^1 \\ 0 & 1 & 0 & -q_y^1 \\ 0 & 0 & 1 & -q_z^1 \\ 0 & 0 & 0 & 1 \end{bmatrix}.$$

The rotation module is used to rotate the unit normal vector of the plane $F_2'$ around the origin of the coordinate

system until the unit normal vector of the plane $F_2$' coincides with the unit normal vector $\overrightarrow{n1}$ of the virtual ultrasound section $F_1$, in order to obtain the plane $F_2$"; the corresponding transformation matrix $T_2$ is:

$$T_2 = \begin{bmatrix} 1 & 0 & 0 & 0 \\ 0 & \cos\theta_x^1 & \sin\theta_x^1 & 0 \\ 0 & -\sin\theta_x^1 & \cos\theta_x^1 & 0 \\ 0 & 0 & 0 & 1 \end{bmatrix} \begin{bmatrix} \cos\theta_y^1 & 0 & \sin\theta_y^1 & 0 \\ 0 & 1 & 0 & 0 \\ -\sin\theta_y^1 & 0 & \cos\theta_y^1 & 0 \\ 0 & 0 & 0 & 1 \end{bmatrix} \begin{bmatrix} \cos\theta_y^2 & 0 & -\sin\theta_y^2 & 0 \\ 0 & 1 & 0 & 0 \\ \sin\theta_y^2 & 0 & \cos\theta_y^2 & 0 \\ 0 & 0 & 0 & 1 \end{bmatrix} \begin{bmatrix} 1 & 0 & 0 & 0 \\ 0 & \cos\theta_x^2 & -\sin\theta_x^2 & 0 \\ 0 & \sin\theta_x^2 & \cos\theta_x^2 & 0 \\ 0 & 0 & 0 & 1 \end{bmatrix}$$

wherein:

$$\cos\theta_x^2 = \frac{n_z^2}{\sqrt{(n_y^2)^2 + (n_z^2)^2}}, \sin\theta_x^2 = \frac{n_y^2}{\sqrt{(n_y^2)^2 + (n_z^2)^2}};$$

$$\cos\theta_y^2 = \frac{\sqrt{(n_y^2)^2 + (n_z^2)^2}}{\sqrt{(n_x^2)^2 + (n_y^2)^2 + (n_z^2)^2}}, \sin\theta_y^2 = \frac{n_x^2}{\sqrt{(n_x^2)^2 + (n_y^2)^2 + (n_z^2)^2}};$$

$$\cos\theta_x^1 = \frac{n_z^1}{\sqrt{(n_y^1)^2 + (n_z^1)^2}}, \sin\theta_x^1 = \frac{n_y^1}{\sqrt{(n_y^1)^2 + (n_z^1)^2}}$$

$$\cos\theta_y^1 = \frac{\sqrt{(n_y^1)^2 + (n_z^1)^2}}{\sqrt{(n_x^1)^2 + (n_y^1)^2 + (n_z^1)^2}}, \sin\theta_y^1 = \frac{n_x^1}{\sqrt{(n_x^1)^2 + (n_y^1)^2 + (n_z^1)^2}}。$$

The axial plane $F_2$ of the virtual first probe is translated until $Q_1$ coincides with the origin of the virtual coordinate system, thereby obtaining the plane $F_2$'. Then the unit normal vector of the plane $F_2$' is rotated around the origin until it coincides with the unit normal vector $\overrightarrow{n_1}$ of the virtual ultrasound section $F_1$; the angle at which the unit normal vector of the plane $F_2$' has rotated counterclockwise around the x axis accordingly is $\theta_x^2$ or $2\pi - \theta_x^1$, and the angle at which the unit normal vector of the plane $F_2$' has rotated counterclockwise around they axis accordingly is $\theta_y^1$ or $2\pi - \theta_y^2$.

Through the rotation module, the plane $F_2$' can thus be rotated and transformed to obtain the plane $F_2$".

The second translation module is used to translate the plane $F_2$" until the sampling point coincident with the origin of the coordinate system in the first translation module returns to its initial position, in order to obtain the plane $F_2$"'. Taking the sampling point $Q_1$ as an example, the corresponding transformation matrix $T_3$ is:

$$\begin{bmatrix} x' \\ y' \\ z' \\ 1 \end{bmatrix} = T_3 \begin{bmatrix} x \\ y \\ z \\ 1 \end{bmatrix},$$

wherein transformation matrix $T_3$ is given by:

$$T_3 = \begin{bmatrix} 1 & 0 & 0 & q_x^1 \\ 0 & 1 & 0 & q_y^1 \\ 0 & 0 & 1 & q_z^1 \\ 0 & 0 & 0 & 1 \end{bmatrix}$$

The third translation module is used to obtain the distance d between the plane $F_2'''$ and the virtual ultrasound section $F_1$. The vector representing the translation required for the plane $F_2'''$ to coincide with the virtual ultrasound section $F_1$ is given by: $\vec{d} = \left(-\overrightarrow{P_1 Q_1'} \cdot \vec{n_1}\right)\vec{n_1}$ , wherein $Q_1' = T_3 T_2 T_1 Q_1$. The transformation matrix $T_4$ is:

$$T_4 = \begin{bmatrix} 1 & 0 & 0 & d_x \\ 0 & 1 & 0 & d_y \\ 0 & 0 & 1 & d_z \\ 0 & 0 & 0 & 1 \end{bmatrix}.$$

The plane $F_2'''$ translate along the vector $\vec{d}$ until it coincide with the virtual ultrasound section $F_1$, thereby obtaining the plane $F_2''''$.

In summary, the final transformation matrix is given by $T = T_4 T_3 T_2 T_1$.

For any point $(x, y, z)^T$ on the axial plane $F_2$ of the virtual first probe, $(x', y', z', 1) = T(x, y, z, 1)^T$, wherein $(x', y', z')^T$ is the coordinate information after the transformation, and $(x, y, z)^T$ is the coordinate information before the transformation.

Thus points on the virtual ultrasound section $F_1$ that correspond to the points on the axial plane $F_2$ of the virtual first probe after transformation can be found.

According to the above formulas, the points on the virtual ultrasound section $F_1$ that correspond to the points $Q_1$, $Q_2$, and $Q_3$ after transformation, where the points $Q_1$, $Q_2$, and $Q_3$ are on the axial plane of the virtual first probe $F_2$, can be found. So is the line that lies in the axial plane of the virtual first probe $F_2$ and perpendicularly bisects the Line Segment $Q_1Q_2$. A path coincident with this line is the planned path L1 in the virtual ultrasound section $F_1$.

In the second data acquisition module, according to the coordinate information of the second positioning devices 5 in the virtual coordinate system, the coordinate information of the axis L2 of the virtual puncture needle and the apex C2 of the virtual puncture needle as projected onto the virtual ultrasound section of the lesion is obtained (i.e., transformed coordinate information) through planar matrix conversion, and the axis L2 of the virtual puncture needle and the apex C2 of the virtual puncture needle are represented in the coordinate system based on the transformed coordinate information.

Specifically, in the virtual coordinate system, when the apex C1 of the fan-shaped ultrasound section of the lesion is taken as the origin, the transformation formula I is used for planar matrix conversion to obtain the transformed coordinate information of the corresponding axis L2 of the virtual puncture needle and apex C2 of the virtual puncture needle.

The comparison module is used in the virtual coordinate system to compare the apex C2 of the virtual puncture needle and the apex C1 of the fan-shaped ultrasound section of the lesion to see if they coincide, and compare the axis L2 of the virtual puncture needle and the planned path L1 to see if they coincide. When the apex C2 of the virtual puncture needle and the apex C1 of the fan-shaped ultrasound section of the lesion coincide, and the axis L2 of the virtual puncture needle and the planned path L1 coincide, a judgment of coincidence is returned.

More specifically, the procedure for judging if the axis L2 of the virtual puncture needle and the planned path L1 coincide is shown in Fig. 8, first, a determination is made as to whether the apex C2 of the virtual puncture needle and the apex C1 of the fan-shaped ultrasound section of the lesion coincide; if not, then a judgment of "not coincidence with the planned path L1" is returned; if yes, then another determination is made as to whether the axis L2 of the virtual puncture needle and the planed path L1 coincide. If the axis L2 of the virtual puncture needle and the planed path L1 coincide, then a judgment of coincidence is returned, and otherwise a judgment of "no coincidence" is returned. Corresponding signals indicating coincidence or the lack of it may be given. In one embodiment, the apex C2 of the virtual puncture needle serves as the center of rotation for the puncture needle to adjust its orientation, as shown in Fig. 7. Initially, the system's indicator light is red, and when the apex C2 of the virtual puncture needle and the apex C1 of the fan-shaped ultrasound section of

the lesion coincide, the indicator light turns blue; when the axis L2 of the puncture needle coincides with the planned path L1, the indicator light turns green. The medical staff monitors information related to the coincidence or the lack of it of the central axis L2 of the puncture needle and the planned path L1, and adjusts the puncture needle according to the color of the indicator light, which may be displayed on a virtual interface, during the movement of the puncture needle to assist the puncture procedure. The path suggested by a green indicator light is adopted to perform the puncturing.

Based on the transformation formula, a set of coordinate information can only correspond to one set of transformed coordinate information.

Similarly, there is only one line in the space whose transformed coordinate information corresponds to the axis of the ultrasound section. Only when the axis of the puncture needle coincides with this line, can the corresponding axis of the virtual puncture needle coincide with the axis of the ultrasound section (i.e., the planned path L1); when the axis of the puncture needle does not coincide with this line, the axis of the virtual puncture needle does not coincide with the planned path L1.

The working process of the puncture needle positioning system of the present disclosure:

The first probe 11 provided with the first positioning devices 4 scans the lesion to obtain an ultrasound section of the lesion, and the ultrasound section of the lesion and the first probe 11 are displayed in the virtual coordinate system of the processing and display unit 3. The ultrasound section of the lesion shows the fan-shaped area scanned by the first probe 11. The apex C1 of the fan-shaped ultrasound section of the lesion corresponds to the contact point between the first probe and the patient's skin, and this contact point is used as the point where the chest wall is to be punctured. i.e., the chest-wall puncture point. The axial plane of the first probe 11 is transformed to coincide with the ultrasound section of the lesion through a coordinate transformation formula, in order to obtain the planned path L1. The apex of the puncture needle is placed at the chest wall puncture point. The coordinate information of the puncture needle provided by the second positioning devices 5 are recorded, and the axis L2 of the puncture needle is displayed in the virtual coordinate system. The orientation of the puncture needle is adjusted with the chest wall puncture point as the center of rotation until the system indicates that the axis L2 of the virtual puncture needle and the planned path L1 coincide, and the puncture needle is then maintained at the position for the puncturing procedure. In a preferred embodiment, during the puncturing procedure, the system can indicate in real time whether the axis of the puncture needle coincides with the planned path L1, and ensure the procedure is carried out smoothly while monitored by the second probe 12.

The present invention also provides a computer-readable storage medium comprising instructions which, when executed by a computer, cause the computer to carry out a puncture needle positioning method, including;

S 100 acquiring the ultrasound section of the lesion and displaying it in the virtual coordinate system; acquiring the coordinate information of a first probe 11 when the latter is acquiring the ultrasound section of the lesion and displaying the first probe 11 in the virtual coordinate system;

S200 determining a planned path L1 in the virtual coordinate system;

S300 obtaining the coordinate information of second positioning devices 5 on a puncture needle in the virtual coordinate system and displaying the axis L2 of the virtual puncture needle and the apex C2 of the virtual puncture needle;

S400 comparing the axis L2 of the virtual puncture needle with the planned path L1 in the virtual coordinate system to see if they coincide.

Specifically, at S100, the ultrasound section of the lesion is obtained by scanning the lesion by the first probe 11, and when a lesion appears on an ultrasound section, the ultrasound section is the ultrasound section of the lesion.

The position of the lesion ultrasound section as displayed in the virtual coordinate system is not limited, and the selection of the origin of the virtual coordinate system is not limited. In order to facilitate data processing, in one embodiment, the ultrasound section of the lesion is fan-shaped and the vertex of the ultrasound section of the lesion serves as the origin of the virtual coordinate system.

The first probe 11 can provide its own coordinate information through the first positioning devices 4. The coordinate information of the first positioning devices 4 matches coordinate information of a virtual coordinate system. The position of each first positioning device 4 in the virtual coordinate system can be used to represent the first probe 11 in the virtual coordinate system. In order to obtain the planned path more conveniently, the position of the axis of the first probe 11 in the virtual coordinate system needs to be determined first. In one embodiment, the first positioning devices 4 of the first probe 11 are all provided on the axial plane of the first probe 11 so that it is convenient to determine the coordinate information of the axis of the first probe 11. In the first probe 11 as shown in Fig. 2, the positions of the three sensors $Q_1$, $Q_2$ and $Q_3$ in the virtual coordinate system can be represented as the position of the first probe 11 in the virtual coordinate system, i.e., the virtual first probe. The axis of the first probe 11 lies in the plane containing Points $Q_1$, $Q_2$ and $Q_3$, and perpendicularly bisects Line Segment $Q_1$-$Q_2$

In order to facilitate subsequent steps for determining the planned path, in one embodiment, the point of the lesion to be puncture at and sampled from (the puncture sampling point) can be positioned on the axis of the ultrasound section of

the lesion.

At S200, according to the coordinate information of the first probe 11 in the virtual coordinate system, the position of the axis of the first probe 11 as projected to the ultrasound section of the lesion is obtained by planar matrix conversion, so as to determine the planned path L1.

The ultrasound section of the lesion is fan-shaped. In general, the line passing through the apex of the ultrasound section and the puncture sampling point of the lesion can serve as the actual planed path. When the puncture sampling point is on the axis of the ultrasound section, the axis of the ultrasound section can be used as the actual planned path. In the real world, the axis of the first probe 11 and the ultrasound section of the lesion should be on the same plane, the apexes of the first probe 11 and the apexes of the ultrasound section of the lesion coincide, and the axis of the first probe 11 and the axis of the ultrasound section of the lesion coincide. Therefore, the planned path can be determined as soon as the position of the axis of the first probe 11 is determined.

However, the relative positions of the ultrasound section of the lesion and the axis of the first probe 11 in the virtual coordinate system do not match the actual relative positions of the two, so it is needed to project the axis of the first probe 11 onto the ultrasound section of the lesion in the virtual coordinate system by conversion.

Specifically, the following transformation formula I may be used to perform planar matrix conversion, and obtain the transformed coordinate information of the projection of the axis of the first probe 11 onto the ultrasound section of the lesion:

$(x', y', z', 1) = T(x, y, z, 1)^T$ (transformation formula 1), wherein $(x', y', z')^T$ is the transformed coordinate information, and $(x, y, z)^T$ is the coordinate information before the transformation.
wherein $T = T_4T_3T_2T_1$, and T is the transformation matrix corresponding to the projection of the axial plane of the virtual first probe onto the virtual ultrasound section in the virtual coordinate system.

$$T_4 = \begin{bmatrix} 1 & 0 & 0 & d_x \\ 0 & 1 & 0 & d_y \\ 0 & 0 & 1 & d_z \\ 0 & 0 & 0 & 1 \end{bmatrix}$$

$$\vec{d} = \left(-\overrightarrow{P_1Q_1'} \cdot \vec{n_1}\right)\vec{n_1},$$

$$\vec{d} = \left(d_x, d_y, d_z\right),$$

$$Q_1' = T_3T_2T_1Q_1$$

$$T_3 = \begin{bmatrix} 1 & 0 & 0 & q_x^1 \\ 0 & 1 & 0 & q_y^1 \\ 0 & 0 & 1 & q_z^1 \\ 0 & 0 & 0 & 1 \end{bmatrix}$$

$$T_2 = \begin{bmatrix} 1 & 0 & 0 & 0 \\ 0 & \cos\theta_x^1 & \sin\theta_x^1 & 0 \\ 0 & -\sin\theta_x^1 & \cos\theta_x^1 & 0 \\ 0 & 0 & 0 & 1 \end{bmatrix} \begin{bmatrix} \cos\theta_y^1 & 0 & \sin\theta_y^1 & 0 \\ 0 & 1 & 0 & 0 \\ -\sin\theta_y^1 & 0 & \cos\theta_y^1 & 0 \\ 0 & 0 & 0 & 1 \end{bmatrix} \begin{bmatrix} \cos\theta_y^2 & 0 & -\sin\theta_y^2 & 0 \\ 0 & 1 & 0 & 0 \\ \sin\theta_y^2 & 0 & \cos\theta_y^2 & 0 \\ 0 & 0 & 0 & 1 \end{bmatrix} \begin{bmatrix} 1 & 0 & 0 & 0 \\ 0 & \cos\theta_x^2 & -\sin\theta_x^2 & 0 \\ 0 & \sin\theta_x^2 & \cos\theta_x^2 & 0 \\ 0 & 0 & 0 & 1 \end{bmatrix}$$

$$\cos\theta_x^2 = \frac{n_z^2}{\sqrt{\left(n_y^2\right)^2 + \left(n_z^2\right)^2}}, \sin\theta_x^2 = \frac{n_y^2}{\sqrt{\left(n_y^2\right)^2 + \left(n_z^2\right)^2}};$$

$$\cos\theta_y^2 = \frac{\sqrt{\left(n_y^2\right)^2 + \left(n_z^2\right)^2}}{\sqrt{\left(n_x^2\right)^2 + \left(n_y^2\right)^2 + \left(n_z^2\right)^2}}, \sin\theta_y^2 = \frac{n_x^2}{\sqrt{\left(n_x^2\right)^2 + \left(n_y^2\right)^2 + \left(n_z^2\right)^2}};$$

$$\cos\theta_x^1 = \frac{n_z^1}{\sqrt{\left(n_y^1\right)^2 + \left(n_z^1\right)^2}}, \sin\theta_x^1 = \frac{n_y^1}{\sqrt{\left(n_y^1\right)^2 + \left(n_z^1\right)^2}}$$

$$\cos\theta_y^1 = \frac{\sqrt{\left(n_y^1\right)^2 + \left(n_z^1\right)^2}}{\sqrt{\left(n_x^1\right)^2 + \left(n_y^1\right)^2 + \left(n_z^1\right)^2}}, \sin\theta_y^1 = \frac{n_x^1}{\sqrt{\left(n_x^1\right)^2 + \left(n_y^1\right)^2 + \left(n_z^1\right)^2}} \circ$$

$$\vec{n}_1 = \frac{\overrightarrow{P_1P_2} \times \overrightarrow{P_1P_3}}{\left|\overrightarrow{P_1P_2}\right| \left|\overrightarrow{P_1P_3}\right|}, \quad \vec{n}_2 = \frac{\overrightarrow{Q_1Q_2} \times \overrightarrow{Q_1Q_3}}{\left|\overrightarrow{Q_1Q_2}\right| \left|\overrightarrow{Q_1Q_3}\right|}$$

$$\vec{n}_1 = \left(n_x^1, n_y^1, n_z^1\right), \quad \vec{n}_2 = \left(n_x^2, n_y^2, n_z^2\right)$$

$$T_1 = \begin{bmatrix} 1 & 0 & 0 & -q_x^1 \\ 0 & 1 & 0 & -q_y^1 \\ 0 & 0 & 1 & -q_z^1 \\ 0 & 0 & 0 & 1 \end{bmatrix}$$

$P_1$, $P_2$, $P_3$ are three non-collinear sampling points on the virtual ultrasound section $F_1$, and $Q_1$, $Q_2$, $Q_3$ are three non-collinear points on an the axial plane $F_2$ of the virtual first probe, $P_1\left(p_x^1, p_y^1, p_z^1\right)$, $P_2\left(p_x^2, p_y^2, p_z^2\right)$, $P_3\left(p_x^3, p_y^3, p_z^3\right)$, $Q_1\left(q_x^1, q_y^1, q_z^1\right)$, $Q_2\left(q_x^2, q_y^2, q_z^2\right)$, $Q_3\left(q_x^3, q_y^3, q_z^3\right)$ are the coordinates of $P_1$, $P_2$, $P_3$, $Q_1$, $Q_2$, and $Q_3$ respectively.

$\vec{n_1}$ is a unit normal vector of the virtual ultrasound section $F_1$, and $\vec{n_2}$ is a unit normal vector of the axial plane $F_2$ of

the virtual first probe.

The axial plane $F_2$ of the virtual first probe is translated until $Q_1$ coincides with the origin of the virtual coordinate system, thereby obtaining the plane $F_2'$. Then the unit normal vector of the plane $F_2'$ is rotated around the origin until it coincides with the unit normal vector $\overrightarrow{n_1}$ of the virtual ultrasound section $F_1$; the angle at which the unit normal vector of the plane $F_2'$ has rotated counterclockwise around the x axis accordingly is $\theta_x^2$ or $2\pi - \theta_x^1$, and the angle at which the unit normal vector of the plane $F_2'$ has rotated counterclockwise around they axis accordingly is $\theta_y^1$ or $2\pi - \theta_y^2$.

As shown in Fig. 6, the process of obtaining coordinate transformation formulas corresponding to the transformation of projecting the axis of the first probe 11 onto the ultrasound section of the lesion is as follows:

S201 obtaining the coordinates of three non-collinear sampling points on a virtual ultrasound section $F_1$ in a virtual coordinate system *OXYZ*, $P_1(p_x^1, p_y^1, p_z^1)$, $P_2(p_x^2, p_y^2, p_z^2)$, $P_3(p_x^3, p_y^3, p_z^3)$, and the coordinates of three non-collinear sampling points on an axial plane $F_2$ of the virtual first probe $Q_1(q_x^1, q_y^1, q_z^1)$, $Q_2(q_x^2, q_y^2, q_z^2)$, $Q_3(q_x^3, q_y^3, q_z^3)$;

S202 obtaining a transformation matrix $T_1$ corresponding to the transformation from the axial plane $F_2$ of the virtual first probe to a plane $F_2'$, wherein the plane F2' is obtained by translating the axial plane $F_2$ of the virtual first probe until any sampling point on $F_2$ is coincident with the origin of the virtual coordinate system;

S203 obtaining a transformation matrix $T_2$ corresponding to the transformation from the plane $F_2'$ to a plane $F_2''$, wherein the plane $F_2''$ is obtained by rotating the plane $F_2'$ around the origin of the coordinate system until its unit normal vector coincides with the unit normal vector $\overrightarrow{n1}$ of the virtual ultrasound section $F_1$;

S204 obtaining a transformation matrix $T_3$ corresponding to the transformation from the plane $F_2''$ to a plane $F_2'''$, wherein the $F_2'''$ is obtained by translating the plane $F_2''$ until the sampling point coincident with the origin of the coordinate system in the first translation module returns to its initial position;

S205 obtaining a transformation matrix $T_4$ corresponding to the transformation from the plane $F_2'''$ to a plane $F_2''''$, wherein the plane $F_2''''$ is obtained by first obtaining the distance between the plane $F_2'''$ and the virtual ultrasound section $F_1$, and then translating the plane $F_2'''$ along its unit normal vector (which is equal to the unit normal vector $\overrightarrow{n1}$ of the $F_1$) by a corresponding vector $\vec{d}$ to coincide with the virtual ultrasound section $F_1$, wherein the coordinates of the projection of the axis of the first probe onto the ultrasound section can be obtained by using a coordinate transformation formula and the coordinates of the axis of the first probe on the plane $F_2$.

Specifically, at S201, the virtual ultrasound section F1 is a virtual section displayed in the virtual coordinate system, obtained by scanning the lesion with the first probe 11. The axial plane F2 of the virtual first probe is the axial plane of the first probe 11 as shown in the virtual coordinates system when the first probe 11 is scanning the lesion to obtain the ultrasound section of the lesion. The axial plane of the virtual first probe can be located according to the sensors on the first probe 11.

In a preferred embodiment, the virtual coordinate system also displays the apex C1 of the fan-shaped ultrasound section of the lesion to assist in determining the puncture point. Specifically, the virtual ultrasound section $F_1$ is fan-shaped, in which case the point where the first probe 11 is in contact with the patient's chest wall is represented in the virtual coordinate system as the apex C1 of the fan-shaped ultrasound section of the lesion.

In one embodiment, the axis L and axial plane of the first probe 11 can be determined according to the positions of the first positioning devices 4 on the first probe 11. In one embodiment, in order to facilitate the determination of axis of the first probe 11, the first positioning devices 4 are located on the axial plane of the first probe 11.

The first positioning devices 4 may be sensors, and three non-collinear sensors $Q_1$, $Q_2$ and $Q_3$ as shown in Fig. 2 are provided on the axial plane of the first probe 11 to locate the axis and axial plane of the first probe 11.

The three non-collinear sensors $Q_1$, $Q_2$ and $Q_3$ constitute the vertices of a right triangle, and the axis of the first probe 11 perpendicularly bisects Line Segment $Q_1$-$Q_2$, for determining the coordinate of the axis of the first probe 11.

In a preferred embodiment, the virtual three-dimensional coordinate system OXYZ uses the vertex C1 of the fan-shaped ultrasound section $F_1$ as the origin of the coordinate system.

In another preferred embodiment, at S202, the axial plane $F_2$ of the virtual first probe is translated until Point $Q_1$ coincides with the origin of the coordinate system.

In one embodiment, the vertex C1 of the fan-shaped virtual ultrasonic section F1 is taken as the origin of the virtual

coordinate system *OXYZ,* and at S202, the axial plane $F_2$ of the virtual first probe is translated until $Q_1$ coincides with the origin.

The corresponding coordinate transformation is given by:

$$\begin{bmatrix} x' \\ y' \\ z' \\ 1 \end{bmatrix} = \begin{bmatrix} 1 & 0 & 0 & -q_x^1 \\ 0 & 1 & 0 & -q_y^1 \\ 0 & 0 & 1 & -q_z^1 \\ 0 & 0 & 0 & 1 \end{bmatrix} \begin{bmatrix} x \\ y \\ z \\ 1 \end{bmatrix}$$

wherein

$$T_1 = \begin{bmatrix} 1 & 0 & 0 & -q_x^1 \\ 0 & 1 & 0 & -q_y^1 \\ 0 & 0 & 1 & -q_z^1 \\ 0 & 0 & 0 & 1 \end{bmatrix}$$

At S203, the plane $F_2$" is obtained by rotating the plane $F_2$' around the origin of the coordinate system until its unit normal vector of $F_2$' coincides with the unit normal vector $\overrightarrow{n1}$ of the virtual ultrasound section $F_1$. The corresponding coordinate transformation is given by:

$$T_2 = \begin{bmatrix} 1 & 0 & 0 & 0 \\ 0 & \cos\theta_x^1 & \sin\theta_x^1 & 0 \\ 0 & -\sin\theta_x^1 & \cos\theta_x^1 & 0 \\ 0 & 0 & 0 & 1 \end{bmatrix} \begin{bmatrix} \cos\theta_y^1 & 0 & \sin\theta_y^1 & 0 \\ 0 & 1 & 0 & 0 \\ -\sin\theta_y^1 & 0 & \cos\theta_y^1 & 0 \\ 0 & 0 & 0 & 1 \end{bmatrix} \begin{bmatrix} \cos\theta_y^2 & 0 & -\sin\theta_y^2 & 0 \\ 0 & 1 & 0 & 0 \\ \sin\theta_y^2 & 0 & \cos\theta_y^2 & 0 \\ 0 & 0 & 0 & 1 \end{bmatrix} \begin{bmatrix} 1 & 0 & 0 & 0 \\ 0 & \cos\theta_x^2 & -\sin\theta_x^2 & 0 \\ 0 & \sin\theta_x^2 & \cos\theta_x^2 & 0 \\ 0 & 0 & 0 & 1 \end{bmatrix}$$

$$\cos\theta_x^2 = \frac{n_z^2}{\sqrt{\left(n_y^2\right)^2 + \left(n_z^2\right)^2}}, \sin\theta_x^2 = \frac{n_y^2}{\sqrt{\left(n_y^2\right)^2 + \left(n_z^2\right)^2}};$$

$$\cos\theta_y^2 = \frac{\sqrt{\left(n_y^2\right)^2 + \left(n_z^2\right)^2}}{\sqrt{\left(n_x^2\right)^2 + \left(n_y^2\right)^2 + \left(n_z^2\right)^2}}, \sin\theta_y^2 = \frac{n_x^2}{\sqrt{\left(n_x^2\right)^2 + \left(n_y^2\right)^2 + \left(n_z^2\right)^2}};$$

$$\cos\theta_x^1 = \frac{n_z^1}{\sqrt{\left(n_y^1\right)^2 + \left(n_z^1\right)^2}}, \sin\theta_x^1 = \frac{n_y^1}{\sqrt{\left(n_y^1\right)^2 + \left(n_z^1\right)^2}}$$

$$\cos\theta_y^1 = \frac{\sqrt{\left(n_y^1\right)^2 + \left(n_z^1\right)^2}}{\sqrt{\left(n_x^1\right)^2 + \left(n_y^1\right)^2 + \left(n_z^1\right)^2}}, \sin\theta_y^1 = \frac{n_x^1}{\sqrt{\left(n_x^1\right)^2 + \left(n_y^1\right)^2 + \left(n_z^1\right)^2}} 。$$

The axial plane $F_2$ of the virtual first probe is translated until $Q_1$ coincides with the origin of the virtual coordinate system, thereby obtaining the plane $F_2$'. Then the unit normal vector of the plane $F_2$' is rotated around the origin until it coincides with the unit normal vector $\overrightarrow{n_1}$ of the virtual ultrasound section $F_1$; the angle at which the unit normal vector of the plane $F_2$' has rotated counterclockwise around the x axis accordingly is $\theta_x^2$ or $2\pi - \theta_x^1$, and the angle at which the unit normal vector of the plane $F_2$' has rotated counterclockwise around they axis accordingly is $\theta_y^1$ or $2\pi - \theta_y^2$.

Through the above steps, the plane $F_2$' can be rotated and transformed to obtain the plane $F_2$".

At S204, the plane $F_2$''' is obtained by translating the plane $F_2$" until the sampling point coincident with the origin of the coordinate system in the first translation module returns to its initial position. Take $Q_1$ as example, the corresponding coordinate transformation is given by:

$$\begin{bmatrix} x' \\ y' \\ z' \\ 1 \end{bmatrix} = T_3 \begin{bmatrix} x \\ y \\ z \\ 1 \end{bmatrix},$$

wherein

$$T_3 = \begin{bmatrix} 1 & 0 & 0 & q_x^1 \\ 0 & 1 & 0 & q_y^1 \\ 0 & 0 & 1 & q_z^1 \\ 0 & 0 & 0 & 1 \end{bmatrix}$$

At S205, the distance between the plane $F_2$''' and the virtual ultrasound section $F_1$ is obtained. The plane $F_2$'''' is obtained by translating the plane $F_2$''' along its unit normal vector by a vector $\vec{d}$ based on the distance to coincide with the virtual ultrasound section $F_1$. And the corresponding coordinate transformation is given by:

$$\vec{d} = \left(-\overrightarrow{P_1 Q_1'} \cdot \overrightarrow{n_1}\right)\overrightarrow{n_1},$$

$$Q_1' = T_3 T_2 T_1 Q_1,$$

$$T_4 = \begin{bmatrix} 1 & 0 & 0 & d_x \\ 0 & 1 & 0 & d_y \\ 0 & 0 & 1 & d_z \\ 0 & 0 & 0 & 1 \end{bmatrix}.$$

In summary, the final transformation matrix is given by $T = T_4 T_3 T_2 T_1$.

For any point $(x, y, z)^T$ on the axial plane $F_2$ of the virtual first probe, $(x', y', z', 1) = T(x, y, z, 1)^T$, wherein $(x', y', z')^T$ is

the coordinate information after the transformation, and $(x, y, z)^T$ is the coordinate information before the transformation.

Thus points on the virtual ultrasound section $F_1$ that correspond to the points on the axial plane $F_2$ of the virtual first probe after transformation can be found.

According to the above formulas, the points on the virtual ultrasound section $F_1$ that correspond to the points $Q_1$, $Q_2$, and $Q_3$ after transformation, where the points $Q_1$, $Q_2$, and $Q_3$ are on the axial plane $F_2$ of the virtual first probe, can be found. So is the line that lies in the axial plane $F_2$ of the virtual first probe and perpendicularly bisects the Line Segment $Q_1Q_2$. A path coincident with this line is the planned path L1 in the virtual ultrasound section $F_1$.

At S300, according to the coordinate information of the second positioning devices 5 in the virtual coordinate system, the coordinate information of the axis L2 of the virtual puncture needle and the apex C2 of the virtual puncture needle as projected onto the ultrasound section of the lesion is obtained, i.e., transformed coordinate information, through planar matrix conversion, and the axis L2 of the virtual puncture needle and the apex C2 of the virtual puncture needle are represented in the virtual coordinate system based on the transformed coordinate information.

Specifically, in the virtual coordinate system, when the apex C1 of the fan-shaped ultrasound section of the lesion is taken as the origin, the transformation formula I is used for planar matrix conversion to obtain the corresponding axis L2 of the virtual puncture needle and apex C2 of the virtual puncture needle.

At S400, in the virtual coordinate system, the apex C2 of the virtual puncture needle and the apex C1 of the fan-shaped ultrasound section of the lesion are compared to see if they coincide, and the axis L2 of the virtual puncture needle and the planned path L1 are compared to see if they coincide. When the apex C2 of the virtual puncture needle and the apex C1 of the fan-shaped ultrasound section of the lesion coincide, and the axis L2 of the virtual puncture needle and the planned path L1 coincide, a judgment of coincidence is returned.

More specifically, the procedure for judging if the axis L2 of the virtual puncture needle and the planned path L1 coincide is shown in Fig. 8, first, a determination is made as to whether the apex C2 of the virtual puncture needle and the apex C1 of the fan-shaped ultrasound section of the lesion coincide; if not, then a judgment of "not coincidence with the planned path L1" is returned; if yes, then another determination is made as to whether the axis L2 of the virtual puncture needle and the planed path L1 coincide. If the axis L2 of the virtual puncture needle and the planed path L1 coincide, then a judgment of coincidence is returned, and otherwise a judgment of "no coincidence" is returned. Corresponding signals indicating coincidence or the lack of it may be given. In one embodiment, the apex C2 of the virtual puncture needle serves as the center of rotation for the puncture needle to adjust its orientation, as shown in Fig. 7. Initially, the system's indicator light is red, and when the apex C2 of the virtual puncture needle and the apex C1 of the fan-shaped ultrasound section of the lesion coincide, the indicator light turns blue; when the axis L2 of the puncture needle coincides with the planned path L1, the indicator light turns green. The medical staff monitors information related to the coincidence or the lack of it of the axis L2 of the puncture needle and the planned path L1, and adjusts the puncture needle according to the color of the indicator light, which may be displayed on a virtual interface, during the movement of the puncture needle to assist the puncture procedure. The path suggested by a green indicator light is adopted to perform the puncturing.

Based on the transformation formula, a set of coordinate information can only correspond to one set of transformed coordinate information.

Similarly, there is only one line in the space whose transformed coordinate information corresponds to the axis of the ultrasound section. Only when the axis of the puncture needle coincides with this line, can the corresponding axis of the virtual puncture needle coincide with the axis of the ultrasound section (i.e., the planned path L1); when the axis of the puncture needle does not coincide with this line, the axis of the virtual puncture needle does not coincide with the planned path L1.

The present disclosure further describes a non-claimed device, including: a memory, a processor; wherein a computer program is stored in the memory, and the processor is configured to execute the computer program stored in the memory. When the computer program is executed, the puncture needle positioning method described in the present disclosure is realized.

The memory may include random access memory (RAM), and may also include non-volatile memory, for example, at least one disk memory.

The processor may be a general-purpose processor, including a central processing unit (CPU), a network processor (NP), etc.; it may also be a digital signal processor (DSP), Application Specific Integrated Circuit (ASIC), Field-Programmable Gate Array (Field-Programmable Gate Array, FPGA), other programming logic devices, discrete gates or transistor logic devices, or discrete hardware components.

The present disclosure also provides a computer-readable storage medium with computer programs stored thereon, that when executed by a processor, perform the puncture needle positioning method described herein.

The computer-readable storage medium, as a person of ordinary skilled in the art can understand, perform all or part of the steps of the above-mentioned method's embodiments by computer program-related hardware. The aforementioned computer program can be stored in a computer-readable storage medium, which, when executed, performs the steps including the above-mentioned method's embodiments; and the computer-readable storage medium may be ROM, RAM, magnetic disks, optical disks , or other media that can store program codes.

As shown in Fig. 9, the present disclosure also provides a method for obtaining the axis of an ultrasound probe as projected onto an ultrasound section, comprising:

S201' obtaining the coordinates of three non-collinear sampling points on a virtual ultrasound section $F_1$ in a virtual coordinate system *OXYZ*, $P_1\left(p_x^1, p_y^1, p_z^1\right)$, $P_2\left(p_x^2, p_y^2, p_z^2\right)$, $P_3\left(p_x^3, p_y^3, p_z^3\right)$, and the coordinates of three non-collinear sampling points on an axial plane $F_2$ of a virtual ultrasound probe $Q_1\left(q_x^1, q_y^1, q_z^1\right)$, $Q_2\left(q_x^2, q_y^2, q_z^2\right)$, $Q_3\left(q_x^3, q_y^3, q_z^3\right)$;

S202' obtaining a transformation matrix $T_1$ corresponding to the transformation from the axial plane $F_2$ of the virtual ultrasound probe to a plane $F_2$', wherein the plane F2' is obtained by translating the axial plane $F_2$ of the virtual ultrasound probe until any sampling point on $F_2$ is coincident with the origin of the virtual coordinate system;

S203' obtaining a transformation matrix $T_2$ corresponding to the transformation from the plane $F_2$' to a plane $F_2$", wherein the plane $F_2$" is obtained by rotating the plane $F_2$' around the origin of the coordinate system until its unit normal vector coincides with the unit normal vector $\vec{n1}$ of the virtual ultrasound section $F_1$;

S204' obtaining a transformation matrix $T_3$ corresponding to the transformation from the plane $F_2$" to a plane $F_2$‴, wherein the $F_2$‴ is obtained by translating the plane $F_2$" until the sampling point coincident with the origin of the coordinate system in the first translation module returns to its initial position;

S205' obtaining a transformation matrix $T_4$ corresponding to the transformation from the plane $F_2$‴ to a plane $F_2$⁗, wherein the plane $F_2$⁗ is obtained by first obtaining the distance between the plane $F_2$‴ and the virtual ultrasound section $F_1$, and then translating the plane $F_2$‴ along its unit normal vector by a corresponding vector $\vec{d}$ to coincide with the virtual ultrasound section $F_1$, wherein the coordinates of the projection of the axis of the ultrasound probe onto the virtual ultrasound section $F_1$ can be obtained by using a coordinate transformation formula and the coordinates of the axis of the ultrasound probe on the plane $F_2$.

Specifically, at S201', the virtual ultrasound section $F_1$ is a virtual plane of the ultrasound section of the lesion displayed in the virtual coordinate system, obtained by scanning the lesion with the ultrasound probe 8. The axial plane $F_2$ of the virtual ultrasound probe is the axial plane of the virtual ultrasound probe as shown in the virtual coordinates system when the ultrasound probe 8 is scanning the lesion to obtain the ultrasound section of the lesion. The axial plane of the virtual ultrasound probe can be located according to the sensors on the ultrasound probe 8.

In one embodiment, the virtual coordinate system also displays the apex of the fan-shaped ultrasound section of the lesion to assist in determining the puncture point. Specifically, the point where the ultrasound probe 8 is in contact with the patient's chest wall is represented in the virtual coordinate system as the apex of the fan-shaped ultrasound section of the lesion.

In one embodiment, the axis L and axial plane of the ultrasound probe 8 can be determined according to the positions of positioning devices 9 on the ultrasound probe 8. In one embodiment, in order to facilitate the determination of the axis L of the ultrasound probe 8, the positioning devices 9 are located on the axial plane of the ultrasound probe 8.

The positioning devices 9 may be sensors, and three non-collinear sensors $Q_1$, $Q_2$ and $Q_3$ as shown in Fig. 10 are provided on the axial plane of the ultrasound probe 8 to locate the axis and axial plane of the ultrasound probe 8.

The three non-collinear sensors $Q_1$, $Q_2$ and $Q_3$ constitute the vertices of a right triangle, and the axis of the ultrasound probe 8 perpendicularly bisects Line Segment $Q_1$-$Q_2$. This arrangement is for determining the coordinate of the axis of the ultrasound probe 8..

In one embodiment, the virtual three-dimensional coordinate system *OXYZ* uses the vertex of the fan-shaped virtual ultrasonic section $F_1$ of the lesion as the origin of the coordinate system.

In another embodiment, at S202', the axial plane $F_2$ of the virtual ultrasound probe is translated until Point $Q_1$ coincides with the origin of the coordinate system.

In one embodiment, in the virtual three-dimensional coordinate system *OXYZ*, the vertex of the fan-shaped ultrasonic section $F_1$ of the lesion is taken as the origin of the coordinate system, and at S202' the axial plane $F_2$ of the virtual ultrasound probe is translated until $Q_1$ coincides with the origin, in which case, the coordinate transformation formula at S205' is

$$\left(x', y', z', 1\right) = T(x, y, z, 1)^T$$

wherein $(x', y', z')^T$ is the transformed coordinate information, and $(x, y, z)^T$ is the coordinate information before the transformation.

$T=T_4T_3T_2T_1$, wherein T is the transformation matrix corresponding to the projection of the axial plane of the virtual ultrasound probe onto the virtual ultrasound section.

Specifically, at S202' the transformation matrix $T_1$ corresponding to the transformation from the axial plane $F_2$ of the virtual ultrasound probe to the plane $F_2'$ is given by:

$$T_1 = \begin{bmatrix} 1 & 0 & 0 & -q_x^1 \\ 0 & 1 & 0 & -q_y^1 \\ 0 & 0 & 1 & -q_z^1 \\ 0 & 0 & 0 & 1 \end{bmatrix}$$

At S203', the transformation matrix $T_2$ corresponding to the transformation from the plane $F_2'$ to the plane $F_2''$ is given by:

$$T_2 = \begin{bmatrix} 1 & 0 & 0 & 0 \\ 0 & \cos\theta_x^1 & \sin\theta_x^1 & 0 \\ 0 & -\sin\theta_x^1 & \cos\theta_x^1 & 0 \\ 0 & 0 & 0 & 1 \end{bmatrix} \begin{bmatrix} \cos\theta_y^1 & 0 & \sin\theta_y^1 & 0 \\ 0 & 1 & 0 & 0 \\ -\sin\theta_y^1 & 0 & \cos\theta_y^1 & 0 \\ 0 & 0 & 0 & 1 \end{bmatrix} \begin{bmatrix} \cos\theta_y^2 & 0 & -\sin\theta_y^2 & 0 \\ 0 & 1 & 0 & 0 \\ \sin\theta_y^2 & 0 & \cos\theta_y^2 & 0 \\ 0 & 0 & 0 & 1 \end{bmatrix} \begin{bmatrix} 1 & 0 & 0 & 0 \\ 0 & \cos\theta_x^2 & -\sin\theta_x^2 & 0 \\ 0 & \sin\theta_x^2 & \cos\theta_x^2 & 0 \\ 0 & 0 & 0 & 1 \end{bmatrix}$$

At S204', the transformation matrix $T_3$ corresponding to the transformation from the plane $F_2''$ to the plane $F_2'''$ is given by:

$$T_3 = \begin{bmatrix} 1 & 0 & 0 & q_x^1 \\ 0 & 1 & 0 & q_y^1 \\ 0 & 0 & 1 & q_z^1 \\ 0 & 0 & 0 & 1 \end{bmatrix}$$

At S205', the transformation matrix $T_4$ corresponding to the transformation from the plane $F_2'''$ to the plane $F_2''''$ is given by:

$$T_4 = \begin{bmatrix} 1 & 0 & 0 & d_x \\ 0 & 1 & 0 & d_y \\ 0 & 0 & 1 & d_z \\ 0 & 0 & 0 & 1 \end{bmatrix}$$

wherein,

$$\cos\theta_x^2 = \frac{n_z^2}{\sqrt{\left(n_y^2\right)^2 + \left(n_z^2\right)^2}}, \sin\theta_x^2 = \frac{n_y^2}{\sqrt{\left(n_y^2\right)^2 + \left(n_z^2\right)^2}};$$

$$\cos\theta_y^2 = \frac{\sqrt{\left(n_y^2\right)^2 + \left(n_z^2\right)^2}}{\sqrt{\left(n_x^2\right)^2 + \left(n_y^2\right)^2 + \left(n_z^2\right)^2}}, \sin\theta_y^2 = \frac{n_x^2}{\sqrt{\left(n_x^2\right)^2 + \left(n_y^2\right)^2 + \left(n_z^2\right)^2}};$$

$$\cos\theta_x^1 = \frac{n_z^1}{\sqrt{\left(n_y^1\right)^2 + \left(n_z^1\right)^2}}, \sin\theta_x^1 = \frac{n_y^1}{\sqrt{\left(n_y^1\right)^2 + \left(n_z^1\right)^2}}$$

$$\cos\theta_y^1 = \frac{\sqrt{\left(n_y^1\right)^2 + \left(n_z^1\right)^2}}{\sqrt{\left(n_x^1\right)^2 + \left(n_y^1\right)^2 + \left(n_z^1\right)^2}}, \sin\theta_y^1 = \frac{n_x^1}{\sqrt{\left(n_x^1\right)^2 + \left(n_y^1\right)^2 + \left(n_z^1\right)^2}}$$

$\vec{n_1}$ is a unit normal vector of the virtual ultrasound section $F_1$, and $\vec{n_2}$ is a unit normal vector of the axial plane $F_2$ of the virtual ultrasound probe.

$$\vec{n}_1 = \frac{\overrightarrow{P_1P_2} \times \overrightarrow{P_1P_3}}{\left|\overrightarrow{P_1P_2}\right|\left|\overrightarrow{P_1P_3}\right|}, \quad \vec{n}_2 = \frac{\overrightarrow{Q_1Q_2} \times \overrightarrow{Q_1Q_3}}{\left|\overrightarrow{Q_1Q_2}\right|\left|\overrightarrow{Q_1Q_3}\right|},$$

$$\vec{n_1} = \left(n_x^1, n_y^1, n_z^1\right), \quad \vec{n_2} = \left(n_x^2, n_y^2, n_z^2\right)$$

The plane $F_2'''$ is translated by the vector $\vec{d}$ to obtain the plane $F_2''''$, and $\vec{d}$ is given by:

$$\vec{d} = \left(-\overrightarrow{P_1Q_1'} \cdot \vec{n_1}\right)\vec{n_1},$$

$$\vec{d} = \left(d_x, d_y, d_z\right),$$

$$Q_1' = T_3T_2T_1Q_1.$$

The explanation of the above formulas is as follows:

At S202', the plane F2' is obtained by translating the axial plane $F_2$ of the virtual ultrasound probe until any sampling point on $F_2$ is coincident with the origin of the virtual coordinate system. In one embodiment, the axial plane $F_2$ of the virtual ultrasound probe is translated until Point $Q_1$ coincides with the origin of the virtual coordinate system, and the corresponding transformation matrix $T_1$ is given by:

$$T_1 = \begin{bmatrix} 1 & 0 & 0 & -q_x^1 \\ 0 & 1 & 0 & -q_y^1 \\ 0 & 0 & 1 & -q_z^1 \\ 0 & 0 & 0 & 1 \end{bmatrix}。$$

The coordinates of points on the plane $F_2$' are given by:

$$\begin{bmatrix} x' \\ y' \\ z' \\ 1 \end{bmatrix} = \begin{bmatrix} 1 & 0 & 0 & -q_x^1 \\ 0 & 1 & 0 & -q_y^1 \\ 0 & 0 & 1 & -q_z^1 \\ 0 & 0 & 0 & 1 \end{bmatrix} \begin{bmatrix} x \\ y \\ z \\ 1 \end{bmatrix}$$

At S203', the plane $F_2$" is obtained by rotating the plane $F_2$' around the origin of the coordinate system until its unit normal vector coincides with the unit normal vector $\overrightarrow{n1}$ of the virtual ultrasound section $F_1$; the corresponding transformation matrix $T_2$ is given by:

$$T_2 = \begin{bmatrix} 1 & 0 & 0 & 0 \\ 0 & \cos\theta_x^1 & \sin\theta_x^1 & 0 \\ 0 & -\sin\theta_x^1 & \cos\theta_x^1 & 0 \\ 0 & 0 & 0 & 1 \end{bmatrix} \begin{bmatrix} \cos\theta_y^1 & 0 & \sin\theta_y^1 & 0 \\ 0 & 1 & 0 & 0 \\ -\sin\theta_y^1 & 0 & \cos\theta_y^1 & 0 \\ 0 & 0 & 0 & 1 \end{bmatrix} \begin{bmatrix} \cos\theta_y^2 & 0 & -\sin\theta_y^2 & 0 \\ 0 & 1 & 0 & 0 \\ \sin\theta_y^2 & 0 & \cos\theta_y^2 & 0 \\ 0 & 0 & 0 & 1 \end{bmatrix} \begin{bmatrix} 1 & 0 & 0 & 0 \\ 0 & \cos\theta_x^2 & -\sin\theta_x^2 & 0 \\ 0 & \sin\theta_x^2 & \cos\theta_x^2 & 0 \\ 0 & 0 & 0 & 1 \end{bmatrix}$$

$$\cos\theta_x^2 = \frac{n_z^2}{\sqrt{(n_y^2)^2 + (n_z^2)^2}}, \sin\theta_x^2 = \frac{n_y^2}{\sqrt{(n_y^2)^2 + (n_z^2)^2}};$$

$$\cos\theta_y^2 = \frac{\sqrt{(n_y^2)^2 + (n_z^2)^2}}{\sqrt{(n_x^2)^2 + (n_y^2)^2 + (n_z^2)^2}}, \sin\theta_y^2 = \frac{n_x^2}{\sqrt{(n_x^2)^2 + (n_y^2)^2 + (n_z^2)^2}};$$

$$\cos\theta_x^1 = \frac{n_z^1}{\sqrt{(n_y^1)^2 + (n_z^1)^2}}, \sin\theta_x^1 = \frac{n_y^1}{\sqrt{(n_y^1)^2 + (n_z^1)^2}}$$

$$\cos\theta_y^1 = \frac{\sqrt{(n_y^1)^2 + (n_z^1)^2}}{\sqrt{(n_x^1)^2 + (n_y^1)^2 + (n_z^1)^2}}, \sin\theta_y^1 = \frac{n_x^1}{\sqrt{(n_x^1)^2 + (n_y^1)^2 + (n_z^1)^2}}。$$

The explanation of the above formulas is as follows:
The axial plane $F_2$ of the virtual ultrasound probe is translated until $Q_1$ coincides with the origin of the virtual coordinate system, thereby obtaining the plane $F_2$'. Then the unit normal vector of the plane $F_2$' is rotated around the origin until it coincides with the unit normal vector $\overrightarrow{n_1}$ of the virtual ultrasound section $F_1$; the angle at which the unit normal vector of the

plane $F_2$' has rotated counterclockwise around the *x* axis accordingly is $\theta_x^2$ or $2\pi - \theta_x^1$, and the angle at which the unit normal vector of the plane $F_2$' has rotated counterclockwise around the *y* axis accordingly is $\theta_y^1$ or $2\pi - \theta_y^2$.

At S204', the plane $F_2'''$ is obtained by translating the plane $F_2''$ until the sampling point coincident with the origin of the coordinate system at S202' returns to its initial position. When the sample point is $Q_1$, the corresponding transformation is given by:

$$\begin{bmatrix} x' \\ y' \\ z' \\ 1 \end{bmatrix} = T_3 \begin{bmatrix} x \\ y \\ z \\ 1 \end{bmatrix},$$

wherein

$$T_3 = \begin{bmatrix} 1 & 0 & 0 & q_x^1 \\ 0 & 1 & 0 & q_y^1 \\ 0 & 0 & 1 & q_z^1 \\ 0 & 0 & 0 & 1 \end{bmatrix}。$$

At S205', the distance between the plane $F_2'''$ and the virtual ultrasound section $F_1$ is first obtained, then the plane $F_2'''$ is translated along its unit normal vector by the corresponding vector $\vec{d}$ to coincide with the virtual ultrasound section $F_1$, thereby obtaining the plane $F_2''''$.The vector is given by:

$$T_4 = \begin{bmatrix} 1 & 0 & 0 & d_x \\ 0 & 1 & 0 & d_y \\ 0 & 0 & 1 & d_z \\ 0 & 0 & 0 & 1 \end{bmatrix}。$$

In summary, the final transformation matrix is given by $T = T_4T_3T_2T_1$.

For any point $(x, y, z)^T$ on the axial plane of the plane $F_2$, $(x', y', z', 1) = T(x, y, z, 1)^T$, wherein $(x', y', z')^T$ is the coordinate information after the transformation, and $(x, y, z)^T$ is the coordinate information before the transformation. Thus points on the virtual ultrasound section $F_1$ that correspond to the points on the axial plane $F_2$ of the virtual ultrasound probe after transformation can be found.

According to the above formulas, the points on the virtual ultrasound section $F_1$ that correspond to the points $Q_1$, $Q_2$, and $Q_3$ after transformation, where the $Q_1$, $Q_2$, and $Q_3$ are on the plane $F_2$, can be found. So the line that lies in the axial plane of the virtual ultrasound probe $F_2$ and perpendicularly bisects the Line Segment $Q_1Q_2$. A path coincident with this line is the planned path L1 in the virtual ultrasound section $F_1$.

Therefore, according to the coordinate information of the ultrasound probe 8 in the virtual coordinate system, the position of the axis of the virtual ultrasound probe 8 on the ultrasound section can be obtained by the method of the present disclosure, which can be used as a planned path for transthoracic puncture.

More on determining the planned path by the axis obtained by the method of the present disclosure:

The ultrasound section of the lesion is fan-shaped. Under normal circumstances, a line segment passing through the apex of the fan-shaped ultrasound section of the lesion and the puncture sampling point can be used as the planned

path. When the puncture sampling point is placed on the axis of the ultrasound section of the lesion, the axis of the ultrasound section of the lesion can be used as the planned path. In real situations, the axis L of the ultrasound probe 8 should be located on the ultrasound section of the lesion, and the apex of the ultrasound probe coincides with the apex of the ultrasound section of the lesion, and the axis L of the ultrasound probe 8 coincides with the axis of the fan-shaped ultrasound section of the lesion. Therefore, the planned path can be determined by determining the position of the ultrasound probe's axis. But in the virtual coordinate system, the relative positions of the ultrasound section of the lesion and the axis L of the ultrasound probe 8 do not match the actual relative positions of the two. Therefore, the coordinate transformation formulas described in the method of the present disclosure may be used for conversion, to project the axis L of the ultrasound probe 8 onto the ultrasound section of the lesion.

The present disclosure also provides a device for locating the axis of the ultrasound probe as projected onto an ultrasound section, including

a sampling module, used to obtain the coordinates of three non-collinear sampling points on the a virtual ultrasound section $F_1$ in a virtual coordinate system *OXYZ,* $P_1(p_x^1, p_y^1, p_z^1)$, $P_2(p_x^2, p_y^2, p_z^2)$, $P_3(p_x^3, p_y^3, p_z^3)$, and the coordinates of three non-collinear sampling points on an axial plane $F_2$ of the virtual ultrasound probe $Q_1(q_x^1, q_y^1, q_z^1)$, $Q_2(q_x^2, q_y^2, q_z^2)$, $Q_3(q_x^3, q_y^3, q_z^3)$;

a first translation module, used to obtain the transformation matrix $T_1$ corresponding to the transformation from the axial plane $F_2$ of the virtual ultrasound probe to the plane $F_2'$, wherein the plane F2' is obtained by translating the axial plane $F_2$ of the virtual ultrasound probe until any sampling point on $F_2$ is coincident with the origin of the coordinate system;

a rotation module, used to obtain the transformation matrix $T_2$ corresponding to the transformation from the plane $F_2'$ to the plane $F_2''$, wherein the plane $F_2''$ is obtained by rotating the plane $F_2'$ around the origin of the coordinate system until its unit normal vector coincides with the unit normal vector $\overrightarrow{n1}$ of the virtual ultrasound section $F_1$;

a second translation module, used to obtain the transformation matrix $T_3$ corresponding to the transformation from the plane $F_2''$ to the plane $F_2'''$, wherein the $F_2'''$ is obtained by translating the plane $F_2''$ until the sampling point coincident with the origin of the coordinate system in the first translation module returns to its initial position;

a third translation module, used to obtain the transformation matrix $T_4$ corresponding to the transformation from the plane $F_2'''$ to a plane $F_2''''$, wherein the plane $F_2''''$ is obtained by first obtaining the distance between the plane $F_2'''$ and the virtual ultrasound section $F_1$, and then translating the plane $F_2'''$ along its unit normal vector by the vector $\vec{d}$ to coincide with the virtual ultrasound section $F_1$, wherein the coordinates of the projection of the axis of the ultrasound probe onto the ultrasound section can be obtained by using a coordinate transformation formula and the coordinates of the axis of the ultrasound probe on the plane $F_2$.

Specifically, in the sampling module, the virtual ultrasound section $F_1$ is a virtual plane of the ultrasound section of the lesion displayed in the virtual coordinate system, obtained by scanning the lesion with the ultrasound probe 8. The axial plane $F_2$ of the virtual ultrasound probe is the axial plane of the virtual ultrasound probe as shown in the virtual coordinates system when the ultrasound probe 8 is scanning the lesion to obtain the ultrasound section of the lesion. The axial plane of the virtual ultrasound probe can be located according to the sensors on the ultrasound probe 8.

In one embodiment, the virtual coordinate system also displays the apex of the fan-shaped ultrasound section of the lesion to assist in determining the puncture point. Specifically, the point where the apex of the ultrasound probe 8 is in contact with the patient's chest wall is represented in the virtual coordinate system as the apex of the fan-shaped ultrasound section of the lesion.

In one embodiment, the axis and axial plane of the ultrasound probe 8 can be determined according to the positions of the positioning devices 9 on the ultrasound probe 8. In one embodiment, in order to facilitate the determination of the axis L of the ultrasound probe 8, the positioning devices 9 are located on the axial plane of the ultrasound probe 8.

The positioning devices 9 may be sensors, and the three non-collinear sensors $Q_1$, $Q_2$ and $Q_3$ as shown in Fig. 2 are provided on the axial plane of the ultrasound probe 8 to locate the axis and axial plane of the ultrasound probe 8.

The three non-collinear sensors $Q_1$, $Q_2$ and $Q_3$ constitute the vertices of a right triangle, and the axis of the ultrasound probe 8 perpendicularly bisects Line Segment $Q_1$-$Q_2$. This arrangement is for determining the coordinate of the axis of the ultrasound probe 8.

In one embodiment, the virtual three-dimensional coordinate system OXYZ uses the vertex of the fan-shaped ultrasonic section of the lesion $F_1$ as the origin of the coordinate system..

In another embodiment, in the first translation module, the axial plane $F_2$ of the virtual ultrasound probe is translated until Point $Q_1$ coincides with the origin of the coordinate system.

The vertex of the fan-shaped ultrasonic section F1 is taken as the origin of the virtual three-dimensional coordinate system OXYZ, and in the third translation module, the plane $F_2$ is translated until $Q_1$ coincides with the origin, thereby obtaining the plane $F_2$'. And the corresponding transformation in the third translation module is given by:

$$\left(x', y', z', 1\right) = T(x, y, z, 1)^T$$

wherein $(x', y', z')^T$ is coordinate information after the transformation, $(x, y, z)^T$ is coordinate information before the transformation;

$T = T_4T_3T_2T_1$, wherein T is the transformation matrix corresponding to the projection of the axial plane of the virtual ultrasound probe onto the virtual ultrasound section in the virtual coordinate system.

In the first translation module, the transformation matrix T1 corresponding to the transformation from the plane $F_2$ to the plane F2' is given by:

$$T_1 = \begin{bmatrix} 1 & 0 & 0 & -q_x^1 \\ 0 & 1 & 0 & -q_y^1 \\ 0 & 0 & 1 & -q_z^1 \\ 0 & 0 & 0 & 1 \end{bmatrix}。$$

In the rotation module, the transformation matrix $T_2$ corresponding to the transformation from the plane $F_2$' to the plane $F_2$" is given by:

$$T_2 = \begin{bmatrix} 1 & 0 & 0 & 0 \\ 0 & \cos\theta_x^1 & \sin\theta_x^1 & 0 \\ 0 & -\sin\theta_x^1 & \cos\theta_x^1 & 0 \\ 0 & 0 & 0 & 1 \end{bmatrix} \begin{bmatrix} \cos\theta_y^1 & 0 & \sin\theta_y^1 & 0 \\ 0 & 1 & 0 & 0 \\ -\sin\theta_y^1 & 0 & \cos\theta_y^1 & 0 \\ 0 & 0 & 0 & 1 \end{bmatrix} \begin{bmatrix} \cos\theta_y^2 & 0 & -\sin\theta_y^2 & 0 \\ 0 & 1 & 0 & 0 \\ \sin\theta_y^2 & 0 & \cos\theta_y^2 & 0 \\ 0 & 0 & 0 & 1 \end{bmatrix} \begin{bmatrix} 1 & 0 & 0 & 0 \\ 0 & \cos\theta_x^2 & -\sin\theta_x^2 & 0 \\ 0 & \sin\theta_x^2 & \cos\theta_x^2 & 0 \\ 0 & 0 & 0 & 1 \end{bmatrix}$$

In the second translation nodule, the transformation matrix $T_3$ corresponding to the transformation from the plane $F_2$" to the plane $F_2$''' is given by:

$$T_3 = \begin{bmatrix} 1 & 0 & 0 & q_x^1 \\ 0 & 1 & 0 & q_y^1 \\ 0 & 0 & 1 & q_z^1 \\ 0 & 0 & 0 & 1 \end{bmatrix}。$$

In the third translation module, the transformation matrix $T_4$ corresponding to the transformation from the plane $F_2$''' to the plane $F_2$'''' is given by:

$$T_4 = \begin{bmatrix} 1 & 0 & 0 & d_x \\ 0 & 1 & 0 & d_y \\ 0 & 0 & 1 & d_z \\ 0 & 0 & 0 & 1 \end{bmatrix}$$

wherein

$$\cos\theta_x^2 = \frac{n_z^2}{\sqrt{\left(n_y^2\right)^2 + \left(n_z^2\right)^2}}, \sin\theta_x^2 = \frac{n_y^2}{\sqrt{\left(n_y^2\right)^2 + \left(n_z^2\right)^2}};$$

$$\cos\theta_y^2 = \frac{\sqrt{\left(n_y^2\right)^2 + \left(n_z^2\right)^2}}{\sqrt{\left(n_x^2\right)^2 + \left(n_y^2\right)^2 + \left(n_z^2\right)^2}}, \sin\theta_y^2 = \frac{n_x^2}{\sqrt{\left(n_x^2\right)^2 + \left(n_y^2\right)^2 + \left(n_z^2\right)^2}};$$

$$\cos\theta_x^1 = \frac{n_z^1}{\sqrt{\left(n_y^1\right)^2 + \left(n_z^1\right)^2}}, \sin\theta_x^1 = \frac{n_y^1}{\sqrt{\left(n_y^1\right)^2 + \left(n_z^1\right)^2}}$$

$$\cos\theta_y^1 = \frac{\sqrt{\left(n_y^1\right)^2 + \left(n_z^1\right)^2}}{\sqrt{\left(n_x^1\right)^2 + \left(n_y^1\right)^2 + \left(n_z^1\right)^2}}, \sin\theta_y^1 = \frac{n_x^1}{\sqrt{\left(n_x^1\right)^2 + \left(n_y^1\right)^2 + \left(n_z^1\right)^2}}$$

$\overrightarrow{n_1}$ is a unit normal vector of the virtual ultrasound section $F_1$, and $\overrightarrow{n_2}$ is a unit normal vector of the axial plane $F_2$ of the virtual ultrasound probe.

$$\overrightarrow{n}_1 = \frac{\overrightarrow{P_1P_2} \times \overrightarrow{P_1P_3}}{\left|\overrightarrow{P_1P_2}\right|\left|\overrightarrow{P_1P_3}\right|}, \quad \overrightarrow{n}_2 = \frac{\overrightarrow{Q_1Q_2} \times \overrightarrow{Q_1Q_3}}{\left|\overrightarrow{Q_1Q_2}\right|\left|\overrightarrow{Q_1Q_3}\right|},$$

$$\overrightarrow{n}_1 = \left(n_x^1, n_y^1, n_z^1\right), \quad \overrightarrow{n}_2 = \left(n_x^2, n_y^2, n_z^2\right)$$

The plane $F_2'''$ is translated by the vector $\vec{d}$ to obtain the plane $F_2''''$, and $\vec{d}$ is given by:

$$\overrightarrow{d} = \left(-\overrightarrow{P_1Q_1'} \cdot \overrightarrow{n_1}\right)\overrightarrow{n_1},$$

$$\overrightarrow{d} = \left(d_x, d_y, d_z\right),$$

$$Q_1' = T_3T_2T_1Q_1.$$

The explanation of the above formulas is as follows:

In the first translation module, the plane F2' is obtained by translating the axial plane $F_2$ of the virtual ultrasound probe until any sampling point on $F_2$ is coincident with the origin of the virtual coordinate system. In one embodiment, the axial plane $F_2$

of the virtual ultrasound probe is translated until Point $Q_1$ coincides with the origin of the virtual coordinate system, and the corresponding transformation matrix $T_1$ is given by:

$$T_1 = \begin{bmatrix} 1 & 0 & 0 & -q_x^1 \\ 0 & 1 & 0 & -q_y^1 \\ 0 & 0 & 1 & -q_z^1 \\ 0 & 0 & 0 & 1 \end{bmatrix} 。$$

The coordinates of points on the plane $F_2$' are given by:

$$\begin{bmatrix} x' \\ y' \\ z' \\ 1 \end{bmatrix} = \begin{bmatrix} 1 & 0 & 0 & -q_x^1 \\ 0 & 1 & 0 & -q_y^1 \\ 0 & 0 & 1 & -q_z^1 \\ 0 & 0 & 0 & 1 \end{bmatrix} \begin{bmatrix} x \\ y \\ z \\ 1 \end{bmatrix}$$

In the rotation module, the plane $F_2$" is obtained by rotating the plane $F_2$' around the origin of the coordinate system until its unit normal vector coincides with the unit normal vector $\overrightarrow{n1}$ of the virtual ultrasound section $F_1$; the corresponding transformation matrix $T_2$ is given by:

$$T_2 = \begin{bmatrix} 1 & 0 & 0 & 0 \\ 0 & \cos\theta_x^1 & \sin\theta_x^1 & 0 \\ 0 & -\sin\theta_x^1 & \cos\theta_x^1 & 0 \\ 0 & 0 & 0 & 1 \end{bmatrix} \begin{bmatrix} \cos\theta_y^1 & 0 & \sin\theta_y^1 & 0 \\ 0 & 1 & 0 & 0 \\ -\sin\theta_y^1 & 0 & \cos\theta_y^1 & 0 \\ 0 & 0 & 0 & 1 \end{bmatrix} \begin{bmatrix} \cos\theta_y^2 & 0 & -\sin\theta_y^2 & 0 \\ 0 & 1 & 0 & 0 \\ \sin\theta_y^2 & 0 & \cos\theta_y^2 & 0 \\ 0 & 0 & 0 & 1 \end{bmatrix} \begin{bmatrix} 1 & 0 & 0 & 0 \\ 0 & \cos\theta_x^2 & -\sin\theta_x^2 & 0 \\ 0 & \sin\theta_x^2 & \cos\theta_x^2 & 0 \\ 0 & 0 & 0 & 1 \end{bmatrix}$$

$$\cos\theta_x^2 = \frac{n_z^2}{\sqrt{\left(n_y^2\right)^2 + \left(n_z^2\right)^2}}, \sin\theta_x^2 = \frac{n_y^2}{\sqrt{\left(n_y^2\right)^2 + \left(n_z^2\right)^2}};$$

$$\cos\theta_y^2 = \frac{\sqrt{\left(n_y^2\right)^2 + \left(n_z^2\right)^2}}{\sqrt{\left(n_x^2\right)^2 + \left(n_y^2\right)^2 + \left(n_z^2\right)^2}}, \sin\theta_y^2 = \frac{n_x^2}{\sqrt{\left(n_x^2\right)^2 + \left(n_y^2\right)^2 + \left(n_z^2\right)^2}};$$

$$\cos\theta_x^1 = \frac{n_z^1}{\sqrt{\left(n_y^1\right)^2 + \left(n_z^1\right)^2}}, \sin\theta_x^1 = \frac{n_y^1}{\sqrt{\left(n_y^1\right)^2 + \left(n_z^1\right)^2}}$$

$$\cos\theta_y^1 = \frac{\sqrt{\left(n_y^1\right)^2 + \left(n_z^1\right)^2}}{\sqrt{\left(n_x^1\right)^2 + \left(n_y^1\right)^2 + \left(n_z^1\right)^2}}, \sin\theta_y^1 = \frac{n_x^1}{\sqrt{\left(n_x^1\right)^2 + \left(n_y^1\right)^2 + \left(n_z^1\right)^2}} 。$$

The explanation of the above formulas is as follows:

The axial plane $F_2$ of the virtual ultrasound probe is translated until $Q_1$ coincides with the origin of the virtual coordinate system, thereby obtaining the plane $F_2'$. Then the unit normal vector of the plane $F_2'$ is rotated around the origin until it coincides with the unit normal vector $\vec{n}_1$ of the virtual ultrasound section $F_1$; the angle at which the unit normal vector of the plane $F_2'$ has rotated counterclockwise around the x axis accordingly is $\theta_x^2$ or $2\pi - \theta_x^1$, and the angle at which the unit normal vector of the plane $F_2'$ has rotated counterclockwise around the *y* axis accordingly is $\theta_y^1$ or $2\pi - \theta_y^2$.

Through the above-mentioned steps, the plane $F_2''$is obtained by rotating and transforming the plane $F_2'$.

In the second translation module, the plane $F_2'''$ is obtained by translating the plane $F_2''$ until the sampling point coincident with the origin of the coordinate system at S202' returns to its initial position. When the sample point is $Q_1$, the corresponding transformation $T_3$ is given by:

$$\begin{bmatrix} x' \\ y' \\ z' \\ 1 \end{bmatrix} = T_3 \begin{bmatrix} x \\ y \\ z \\ 1 \end{bmatrix},$$

wherein

$$T_3 = \begin{bmatrix} 1 & 0 & 0 & q_x^1 \\ 0 & 1 & 0 & q_y^1 \\ 0 & 0 & 1 & q_z^1 \\ 0 & 0 & 0 & 1 \end{bmatrix}。$$

In the third translation module, the distance between the plane $F_2'''$ and the virtual ultrasound section $F_1$ is first obtained, then the plane $F_2'''$ is translated along its unit normal vector by the corresponding vector $\vec{d}$ to coincide with the virtual ultrasound section $F_1$, thereby obtaining the plane $F_2'''$.The vector is given by:

$$\vec{d} = \left(-\overrightarrow{P_1 Q_1'} \cdot \vec{n_1}\right)\vec{n_1},$$

wherein

$$Q_1' = T_3 T_2 T_1 Q_1.$$

The corresponding transformation matrix T4 is given by:

$$T_4 = \begin{bmatrix} 1 & 0 & 0 & d_x \\ 0 & 1 & 0 & d_y \\ 0 & 0 & 1 & d_z \\ 0 & 0 & 0 & 1 \end{bmatrix}。$$

In summary, the final transformation matrix is given by $T = T_4T_3T_2T_1$.

For any point $(x, y, z)^T$ *on* the axial plane of the plane $F_2$, $(x', y', z', 1) = T(x, y, z, 1)^T$, wherein $(x', y', z')^T$ is the coordinate information of the axial plane of the plane $F_2$ after the transformation, and $(x, y, z)^T$ is the coordinate information of the axial plane of the plane $F_2$ before the transformation. Thus points on the virtual ultrasound section $F_1$ that correspond to the points on the axial plane $F_2$ of the virtual ultrasound probe after transformation can be found.

According to the above formulas, the points on the virtual ultrasound section $F_1$ that correspond to the points $Q_1$, $Q_2$, and $Q_3$ after transformation, where the $Q_1$, $Q_2$, and $Q_3$ are on the plane $F_2$, can be found. So is the line that lies in the plane $F_2$ and perpendicularly bisects the Line Segment $Q_1Q_2$. A path coincident with this line is the planned path L1 in the virtual ultrasound section $F_1$.

Therefore, according to the coordinate information of the ultrasound probe 8 in the virtual coordinate system, the position of the axis of the ultrasound probe 8 on the ultrasound section of the lesion can be obtained by the device of the present disclosure, which can be used as a planned path for transthoracic puncture.

More on determining the planned path by the axis of the ultrasound probe obtained by the method of the present disclosure:

The ultrasound section of the lesion is fan-shaped. Under normal circumstances, a line segment passing through the apex of the fan-shaped ultrasound section of the lesion and the puncture sampling point can be used as the planned path. When the puncture sampling point is placed on the axis of the ultrasound section of the lesion, the axis of the ultrasound section of the lesion can be used as the planned path. In real situations, the axis L of the ultrasound probe 8 should be located on the ultrasound section of the lesion, and the apex of the ultrasound probe coincides with the apex of the ultrasound section of the lesion, and the axis L of the ultrasound probe 8 coincides with the axis of the fan-shaped ultrasound section of the lesion. Therefore, the planned path can be determined by determining the position of the ultrasound probe's axis. But in the virtual coordinate system, the relative positions of the ultrasound section of the lesion and the axis L of the ultrasound probe 8 do not match the actual relative positions of the two. Therefore, the coordinate transformation formulas described in the method of the present disclosure may be used for conversion, to project the axis L of the ultrasound probe 8 onto the ultrasound section of the lesion. The present disclosure also provides a computer-readable storage medium with computer programs stored thereon, that when executed by a processor, perform the method for obtaining the axis of an ultrasound probe as projected onto the virtual ultrasound section as described herein.

The computer-readable storage medium, as a person of ordinary skilled in the art can understand, perform all or part of the steps of the above-mentioned method's embodiments by computer program-related hardware. The aforementioned computer program can be stored in a computer-readable storage medium, which, when executed, performs the steps including the above-mentioned method's embodiments; and the computer-readable storage medium may be ROM, RAM, magnetic disks, optical disks , or other media that can store program codes.

The present disclosure further describes a non-claimed device, including: a memory, a processor; wherein a computer program is stored in the memory, and the processor is configured to execute the computer program stored in the memory. When the computer program is executed, the method for obtaining the axis of an ultrasound probe as projected onto the virtual ultrasound section as described herein is realized.

The memory may include random access memory (RAM), and may also include non-volatile memory, for example, at least one disk memory.

The processor may be a general-purpose processor, including a central processing unit (CPU), a network processor (NP), etc.; it may also be a digital signal processor (DSP), Application Specific Integrated Circuit (ASIC), Field-Programmable Gate Array (FPGA), other programming logic devices, discrete gates or transistor logic devices, or discrete hardware components.

In summary, the puncture needle positioning system and method provided by the present disclosure can greatly improve the accuracy of transthoracic puncture. Not only can the invention effectively avoid the complications caused by repeated puncture, but also greatly shorten the puncture time during surgery. Adopting the first positioning device and the second positioning device greatly improves the conventional single ultrasonic positioning and navigation technique, and can digitally analyze all position information. Various kinds of position information in the magnetic field can be obtained through the first positioning devices and the second positioning devices, and transformed into three-dimensional information displayed in a three-dimensional coordinate system. The system provides accurate puncture path planning tailored for different individuals, and realizes individualized and precise medical treatment for patients, which will greatly reduce surgery-related complications and make it possible for transthoracic puncture interventional treatment to become a conventional treatment method. The method of obtaining the axis of the virtual ultrasound probe as projected onto the ultrasound section provided by the present disclosure can display the ultrasound section of the lesion and the plane where the positioning devices of the virtual ultrasound probe are located in the same virtual coordinate system, in order to obtain the planned path. The present disclosure provides a standardized technical scheme for transthoracic puncture intracardiac interventional therapy. Through the implementation of the scheme of the present disclosure, the preoperative planned puncture path can be accurately recorded and visualized, to provide a reliable guidance for the medical staff during operation, and effectively reduces the medical staff's error rate in terms of selecting the puncture point. The present

disclosure can not only improve the puncture success rate, but also effectively prevent the occurrence of complications and improve the safety of the operation.

In summary, the present invention effectively overcomes various shortcomings of the prior art and has high industrial value.

## Claims

1. A puncture needle positioning system, comprising:

    an ultrasound unit (1), comprising a first probe (11) for providing an ultrasound section of a lesion, wherein a plurality of first positioning devices (4) for providing coordinate information of the first probe (11) are provided on the first probe (11);
    a puncture needle unit (2), comprising a puncture needle, wherein a plurality of second positioning devices (5) for providing coordinate information of the puncture needle are provided on the puncture needle;
    a processing and display unit (3), communicatively connected with the ultrasound unit (1), each of the first positioning devices (4), and each of the second positioning devices (5) respectively, wherein the processing and display unit (3) is used for: acquiring the ultrasound section of the lesion, and displaying it in a virtual coordinate system; acquiring the coordinate information of the first probe (11) when the first probe is acquiring the ultrasound section of the lesion, and displaying the first probe in the virtual coordinate system as virtual first probe;
    determining a planned path L1 in the virtual coordinate system; obtaining the coordinate information of the puncture needle provided by each of the second positioning device (5) and displaying an axis L2 of a virtual puncture needle and a vertex C2 of the virtual puncture needle in the virtual coordinate system; and in the virtual coordinate system, comparing the axis L2 of the virtual puncture needle with the planned path L1 to see if they are coincident, **characterized in that** the planned path L1 in the virtual coordinate system is determined by obtaining the position of an axis (L) of the first probe (11) on the ultrasound section of the lesion through a planar matrix conversion according to the coordinate information of the first probe (11) in the virtual coordinate system.

2. The puncture needle positioning system according to claim 1, wherein the first probe (11) is provided with three first positioning devices (4), the three first positioning devices (4) are located on the same horizontal plane, which coincides with an axial plane of the first probe (11), two of the three first positioning devices (4) are located on the same cross section of the first probe (11), and the three first positioning devices (4) constitute the vertices of a right triangle.

3. The puncture needle positioning system according to claim 2, wherein the first probe (11) is provided with a probe fastener (6) that matches the first probe (11), the probe fastener (6) is provided with three first insertion holes for installing the first positioning devices (4), and the positions of the three first insertion holes match the three first positioning devices (4).

4. The puncture needle positioning system according to claim 1, wherein two second positioning devices (5) are arranged on the puncture needle, and the second positioning devices (5) are respectively arranged on the axis of the puncture needle.

5. The puncture needle positioning system according to claim 4, wherein the puncture needle is provided with a puncture needle fastener (7) matched with the puncture needle, and the puncture needle fastener (7) is provided with two second insertion holes for installing the second positioning devices (5), and the positions of the two second insertion holes match the two second positioning devices (5).

6. The puncture needle positioning system according to claim 1, wherein the processing and display unit (3) comprises:

    a first data acquisition module, used to acquire the ultrasound section of the lesion and display it in the virtual coordinate system, and used to acquire the coordinate information of the first probe (11) when the first probe is acquiring the ultrasound section of the lesion and to display the first probe in the virtual coordinate system;
    a planned path determination module, used to determine the planned path L1 in the virtual coordinate system;
    a second data acquisition module, used to acquire the coordinate information of the second positioning devices (5) on the puncture needle in the virtual coordinate system and display the axis L2 of the virtual puncture needle and the vertex C2 of the virtual puncture needle; and
    a comparison module, used to compare the axis L2 of the virtual puncture needle and the planned path L1 in the virtual coordinate system to see if they are coincident.

**7.** The puncture needle positioning system according to claim 6, wherein in the planned path determination module, according to the coordinate information of the first probe (11) in the virtual coordinate system, the position of a projection of the axis of the first probe (11) onto the ultrasound section of the lesion is obtained through the planar matrix conversion, and the planned path L1 is thereby determined.

**8.** The puncture needle positioning system according to claim 7, wherein the ultrasound section of the lesion is fan-shaped, the virtual coordinate system takes the apex C1 of the fan-shaped ultrasound section of the lesion as the origin, and the planar matrix conversion is performed according to a transformation formula I to obtain coordinate information of the projection of the axis of the first probe (11) onto the ultrasound section of the lesion;

wherein the transformation formula I is given by

$$\left(x', y', z', 1\right) = T(x, y, z, 1)^T$$

wherein $(x', y', z')^T$ represents the coordinate information of a point on the axis of the first probe after the transformation, $(x, y, z)^T$ represents the coordinate information of the point before the transformation, wherein

$$T = T_4 T_3 T_2 T_1$$

$$T_4 = \begin{bmatrix} 1 & 0 & 0 & d_x \\ 0 & 1 & 0 & d_y \\ 0 & 0 & 1 & d_z \\ 0 & 0 & 0 & 1 \end{bmatrix}.$$

$$\vec{d} = \left(-\overrightarrow{P_1 Q_1'} \cdot \vec{n_1}\right)\vec{n_1}$$

$$\vec{d} = \left(d_x, d_y, d_z\right),$$

$$Q_1' = T_3 T_2 T_1 Q_1;$$

$$T_3 = \begin{bmatrix} 1 & 0 & 0 & q_x^1 \\ 0 & 1 & 0 & q_y^1 \\ 0 & 0 & 1 & q_z^1 \\ 0 & 0 & 0 & 1 \end{bmatrix};$$

$$T_2 = \begin{bmatrix} 1 & 0 & 0 & 0 \\ 0 & \cos\theta_x^1 & \sin\theta_x^1 & 0 \\ 0 & -\sin\theta_x^1 & \cos\theta_x^1 & 0 \\ 0 & 0 & 0 & 1 \end{bmatrix} \begin{bmatrix} \cos\theta_y^1 & 0 & \sin\theta_y^1 & 0 \\ 0 & 1 & 0 & 0 \\ -\sin\theta_y^1 & 0 & \cos\theta_y^1 & 0 \\ 0 & 0 & 0 & 1 \end{bmatrix} \begin{bmatrix} \cos\theta_y^2 & 0 & -\sin\theta_y^2 & 0 \\ 0 & 1 & 0 & 0 \\ \sin\theta_y^2 & 0 & \cos\theta_y^2 & 0 \\ 0 & 0 & 0 & 1 \end{bmatrix} \begin{bmatrix} 1 & 0 & 0 & 0 \\ 0 & \cos\theta_x^2 & -\sin\theta_x^2 & 0 \\ 0 & \sin\theta_x^2 & \cos\theta_x^2 & 0 \\ 0 & 0 & 0 & 1 \end{bmatrix}$$

$$\cos\theta_x^2 = \frac{n_z^2}{\sqrt{\left(n_y^2\right)^2 + \left(n_z^2\right)^2}}, \sin\theta_x^2 = \frac{n_y^2}{\sqrt{\left(n_y^2\right)^2 + \left(n_z^2\right)^2}},$$

$$\cos\theta_y^2 = \frac{\sqrt{\left(n_y^2\right)^2 + \left(n_z^2\right)^2}}{\sqrt{\left(n_x^2\right)^2 + \left(n_y^2\right)^2 + \left(n_z^2\right)^2}}, \sin\theta_y^2 = \frac{n_x^2}{\sqrt{\left(n_x^2\right)^2 + \left(n_y^2\right)^2 + \left(n_z^2\right)^2}},$$

$$\cos\theta_x^1 = \frac{n_z^1}{\sqrt{\left(n_y^1\right)^2 + \left(n_z^1\right)^2}}, \sin\theta_x^1 = \frac{n_y^1}{\sqrt{\left(n_y^1\right)^2 + \left(n_z^1\right)^2}},$$

$$\cos\theta_y^1 = \frac{\sqrt{\left(n_y^1\right)^2 + \left(n_z^1\right)^2}}{\sqrt{\left(n_x^1\right)^2 + \left(n_y^1\right)^2 + \left(n_z^1\right)^2}}, \sin\theta_y^1 = \frac{n_x^1}{\sqrt{\left(n_x^1\right)^2 + \left(n_y^1\right)^2 + \left(n_z^1\right)^2}},$$

$$\vec{n}_1 = \left(n_x^1, n_y^1, n_z^1\right), \quad \vec{n}_2 = \left(n_x^2, n_y^2, n_z^2\right);$$

$$\vec{n}_1 = \frac{\overrightarrow{P_1P_2} \times \overrightarrow{P_1P_3}}{\left|\overrightarrow{P_1P_2}\right| \left|\overrightarrow{P_1P_3}\right|}, \quad \vec{n}_2 = \frac{\overrightarrow{Q_1Q_2} \times \overrightarrow{Q_1Q_3}}{\left|\overrightarrow{Q_1Q_2}\right| \left|\overrightarrow{Q_1Q_3}\right|},$$

$$T_1 = \begin{bmatrix} 1 & 0 & 0 & -q_x^1 \\ 0 & 1 & 0 & -q_y^1 \\ 0 & 0 & 1 & -q_z^1 \\ 0 & 0 & 0 & 1 \end{bmatrix}$$

$P_1$, $P_2$, $P_3$ are three non-collinear sampling points on the ultrasound section F1 of the lesion, and $Q_1$, $Q_2$, $Q_3$ are three non-collinear points on an axial plane $F_2$ of a virtual first probe, $P_1\left(p_x^1, p_y^1, p_z^1\right)$, $P_2\left(p_x^2, p_y^2, p_z^2\right)$, $P_3\left(p_x^3, p_y^3, p_z^3\right)$, $Q_1\left(q_x^1, q_y^1, q_z^1\right)$, $Q_2\left(q_x^2, q_y^2, q_z^2\right)$, $Q_3\left(q_x^3, q_y^3, q_z^3\right)$ are the coordinates of $P_1$, $P_2$, $P_3$, $Q_1$, $Q_2$, and $Q_3$,

$\vec{n}_1$ is a unit normal vector of the ultrasound section F1 of the lesion, and $\vec{n}_2$ is a unit normal vector of the axial plane $F_2$ of the virtual first probe.

9. The puncture needle positioning system according to claim 6, wherein in the second data acquisition module, according to the coordinate information of the second positioning devices (5) in the virtual coordinate system, a planar matrix conversion is performed to obtain transformed coordinate information of the axis L2 of the virtual puncture needle, the axis L2 of the virtual puncture needle is obtained by projecting the axis of the puncture needle onto the ultrasound section of the lesion, and a planar matrix conversion is performed to obtain transformed coordinate information of the vertex C2 of the virtual puncture needle, the vertex C2 of the virtual puncture needle is obtained by projecting the apex of the puncture needle onto the ultrasound section of the lesion, the axis and apex of the virtual puncture needle are then displayed in the virtual coordinate system according to their transformed coordinate information.

10. The puncture needle positioning system according to claim 6, wherein in the comparison module, in the virtual coordinate system, the vertex C2 of the virtual puncture needle is compared with the apex C1 of the ultrasonic section to see if they coincide, and the direction of the axis L2 of the virtual puncture needle is compared with the direction of the planned path L1 to see if they coincide, wherein when the vertex C2 of the virtual puncture needle and the apex C1 of the ultrasonic section coincide, and the direction of the axis L2 of the virtual puncture needle and the direction of the planned path L1 coincide, a judgment of coincidence is returned.

11. A computer-readable storage medium comprising instructions which, when executed by the processing and display unit of a system according to claims 1-10, cause the processing and display unit to carry out a method for positioning a puncture needle, wherein the method comprises:

S100 obtaining an ultrasound section of a lesion and displaying it in a virtual coordinate system; obtaining the coordinate information of a first probe (11) when the first probe (11) is acquiring the ultrasound section of the lesion, and displaying the first probe (11) in the virtual coordinate system;
S200 determining a planned path L1 in the virtual coordinate system;
S300 obtaining the coordinate information of second positioning devices (5) on the puncture needle in the virtual coordinate system and displaying the axis L2 of a virtual puncture needle and the vertex C2 of the virtual puncture needle; and
S400 in the virtual coordinate system, comparing the axis L2 of the virtual puncture needle with the planned path L1 to see if they coincide;
wherein at S200, according to the coordinate information of the first probe (11) in the virtual coordinate system, the position of an axis (L) of the first probe (11) on the ultrasound section of the lesion is obtained through a planar matrix conversion, and the planned path L1 is thereby determined.

12. The computer-readable storage medium according to claim 11, wherein the ultrasound section of the lesion is fan-shaped, the virtual coordinate system takes the apex C1 of the fan-shaped ultrasound section of the lesion as the origin, and the planar matrix conversion is performed according to a transformation formula I to obtain transformed coordinate information of a projection of the axis of the first probe (11) onto the ultrasound section of the lesion, wherein the transformation formula I is given by:

$$\left(x', y', z', 1\right) = T(x, y, z, 1)^T,$$

wherein $(x', y', z')^T$ represents the coordinate information of a point on the axis of the first probe after the transformation, $(x, y, z)^T$ represents the coordinate information of the point before the transformation, wherein

$$T = T_4 T_3 T_2 T_1$$

$$T_4 = \begin{bmatrix} 1 & 0 & 0 & d_x \\ 0 & 1 & 0 & d_y \\ 0 & 0 & 1 & d_z \\ 0 & 0 & 0 & 1 \end{bmatrix},$$

$$\overrightarrow{d} = \left(-\overrightarrow{P_1Q_1'} \cdot \overrightarrow{n_1}\right)\overrightarrow{n_1}.$$

$$\overrightarrow{d} = \left(d_x, d_y, d_z\right),$$

$$Q_1' = T_3T_2T_1Q_1.$$

$$T_3 = \begin{bmatrix} 1 & 0 & 0 & q_x^1 \\ 0 & 1 & 0 & q_y^1 \\ 0 & 0 & 1 & q_z^1 \\ 0 & 0 & 0 & 1 \end{bmatrix};$$

$$T_2 = \begin{bmatrix} 1 & 0 & 0 & 0 \\ 0 & \cos\theta_x^1 & \sin\theta_x^1 & 0 \\ 0 & -\sin\theta_x^1 & \cos\theta_x^1 & 0 \\ 0 & 0 & 0 & 1 \end{bmatrix} \begin{bmatrix} \cos\theta_y^1 & 0 & \sin\theta_y^1 & 0 \\ 0 & 1 & 0 & 0 \\ -\sin\theta_y^1 & 0 & \cos\theta_y^1 & 0 \\ 0 & 0 & 0 & 1 \end{bmatrix} \begin{bmatrix} \cos\theta_y^2 & 0 & -\sin\theta_y^2 & 0 \\ 0 & 1 & 0 & 0 \\ \sin\theta_y^2 & 0 & \cos\theta_y^2 & 0 \\ 0 & 0 & 0 & 1 \end{bmatrix} \begin{bmatrix} 1 & 0 & 0 & 0 \\ 0 & \cos\theta_x^2 & -\sin\theta_x^2 & 0 \\ 0 & \sin\theta_x^2 & \cos\theta_x^2 & 0 \\ 0 & 0 & 0 & 1 \end{bmatrix}$$

$$\cos\theta_x^2 = \frac{n_z^2}{\sqrt{\left(n_y^2\right)^2 + \left(n_z^2\right)^2}}, \sin\theta_x^2 = \frac{n_y^2}{\sqrt{\left(n_y^2\right)^2 + \left(n_z^2\right)^2}},$$

$$\cos\theta_y^2 = \frac{\sqrt{\left(n_y^2\right)^2 + \left(n_z^2\right)^2}}{\sqrt{\left(n_x^2\right)^2 + \left(n_y^2\right)^2 + \left(n_z^2\right)^2}}, \sin\theta_y^2 = \frac{n_x^2}{\sqrt{\left(n_x^2\right)^2 + \left(n_y^2\right)^2 + \left(n_z^2\right)^2}},$$

**19.12.2023**

$$\cos\theta_x^1 = \frac{n_z^1}{\sqrt{\left(n_y^1\right)^2 + \left(n_z^1\right)^2}}, \sin\theta_x^1 = \frac{n_y^1}{\sqrt{\left(n_y^1\right)^2 + \left(n_z^1\right)^2}},$$

$$\cos\theta_y^1 = \frac{\sqrt{\left(n_y^1\right)^2 + \left(n_z^1\right)^2}}{\sqrt{\left(n_x^1\right)^2 + \left(n_y^1\right)^2 + \left(n_z^1\right)^2}}, \sin\theta_y^1 = \frac{n_x^1}{\sqrt{\left(n_x^1\right)^2 + \left(n_y^1\right)^2 + \left(n_z^1\right)^2}},$$

$$\overrightarrow{n_1} = \frac{\overrightarrow{P_1P_2} \times \overrightarrow{P_1P_3}}{\left|\overrightarrow{P_1P_2}\right| \left|\overrightarrow{P_1P_3}\right|}, \quad \overrightarrow{n_2} = \frac{\overrightarrow{Q_1Q_2} \times \overrightarrow{Q_1Q_3}}{\left|\overrightarrow{Q_1Q_2}\right| \left|\overrightarrow{Q_1Q_3}\right|},$$

$$\overrightarrow{n_1} = \left(n_x^1, n_y^1, n_z^1\right), \ \overrightarrow{n_2} = \left(n_x^2, n_y^2, n_z^2\right);$$

$$T_1 = \begin{bmatrix} 1 & 0 & 0 & -q_x^1 \\ 0 & 1 & 0 & -q_y^1 \\ 0 & 0 & 1 & -q_z^1 \\ 0 & 0 & 0 & 1 \end{bmatrix},$$

$P_1$, $P_2$, $P_3$ are three non-collinear sampling points on the ultrasound section F1 of the lesion, and $Q_1$, $Q_2$, $Q_3$ are three non-collinear points on an axial plane $F_2$ of a virtual first probe,

$$P_1\left(p_x^1, p_y^1, p_z^1\right), \ P_2\left(p_x^2, p_y^2, p_z^2\right), \ P_3\left(p_x^3, p_y^3, p_z^3\right),$$

$$Q_1\left(q_x^1, q_y^1, q_z^1\right), \ Q_2\left(q_x^2, q_y^2, q_z^2\right), \ Q_3\left(q_x^3, q_y^3, q_z^3\right)$$ are the coordinates of $P_1$, $P_2$, $P_3$, $Q_1$, $Q_2$, and $Q_3$,

$\vec{n}_1$ is a unit normal vector of the ultrasound section F1 of the lesion, and $\vec{n}_2$ is a unit normal vector of the axial plane $F_2$ of the virtual first probe.

**13.** The computer-readable storage medium according to claim 11, wherein at S300, according to the coordinate information of the second positioning devices (5) in the virtual coordinate system, a planar matrix conversion is performed to obtain transformed coordinate information of the axis L2 of the virtual puncture needle, the axis L2 of the virtual puncture needle is obtained by projecting the axis of the puncture needle onto the ultrasound section of the lesion, a planar matrix conversion is performed to obtain transformed coordinate information of the vertex C2 of the virtual puncture needle, the vertex C2 of the virtual puncture needle is obtained by projecting the apex of the puncture needle onto the ultrasound section of the lesion, the axis and apex of the virtual puncture needle are then displayed in the virtual coordinate system according to their transformed coordinate information.

**14.** The computer-readable storage medium according to claim 11, wherein at S400, in the virtual coordinate system, the vertex C2 of the virtual puncture needle is compared with the apex C1 of the ultrasonic section to see if they coincide, and the direction of the axis L2 of the virtual puncture needle is compared with the direction of the planned path L1 to see if they coincide, wherein when the vertex C2 of the virtual puncture needle and the apex C1 of the ultrasonic section coincide, and the direction of the axis L2 of the virtual puncture needle and the direction of the planned path L1 coincide, a judgment of coincidence is returned.

## Patentansprüche

**1.** Punktionsnadel-Positionierungssystem, umfassend:

eine Ultraschalleinheit (1), umfassend eine erste Sonde (11) zum Bereitstellen eines Ultraschallabschnitts einer Läsion, wobei eine Vielzahl von ersten Positionierungsvorrichtungen (4) zum Bereitstellen von Koordinateninformationen der ersten Sonde (11) auf der ersten Sonde (11) bereitgestellt sind;

eine Punktionsnadeleinheit (2), umfassend eine Punktionsnadel, wobei eine Vielzahl von zweiten Positionierungsvorrichtungen (5) zum Bereitstellen von Koordinateninformationen der Punktionsnadel auf der Punktionsnadel bereitgestellt sind;

eine Verarbeitungs- und Anzeigeeinheit (3), die kommunikativ mit der Ultraschalleinheit (1), jeder der ersten Positionierungsvorrichtungen (4) und jeder der zweiten Positionierungsvorrichtungen (5) verbunden ist, wobei die Verarbeitungs- und Anzeigeeinheit (3) verwendet wird zum: Erfassen des Ultraschallabschnitts der Läsion und Anzeigen desselben in einem virtuellen Koordinatensystem; Erfassen der Koordinateninformationen der ersten Sonde (11), wenn die erste Sonde den Ultraschallabschnitt der Läsion erfasst, und Anzeigen der ersten Sonde in dem virtuellen Koordinatensystem als virtuelle erste Sonde; Bestimmen eines geplanten Pfads L1 in

dem virtuellen Koordinatensystem; Erhalten der Koordinateninformationen der Punktionsnadel, die von jeder der zweiten Positionierungsvorrichtungen (5) bereitgestellt werden, und Anzeigen einer Achse L2 einer virtuellen Punktionsnadel und eines Scheitels C2 der virtuellen Punktionsnadel in dem virtuellen Koordinatensystem; und in dem virtuellen Koordinatensystem, Vergleichen der Achse L2 der virtuellen Punktionsnadel mit dem geplanten Pfad L1, um zu sehen, ob sie zusammenfallen, **dadurch gekennzeichnet, dass** der geplante Pfad L1 in dem virtuellen Koordinatensystem durch Erhalten der Position einer Achse (L) der ersten Sonde (11) auf dem Ultraschallabschnitt der Läsion durch eine planare Matrixumwandlung gemäß den Koordinateninformationen der ersten Sonde (11) in dem virtuellen Koordinatensystem bestimmt wird.

**2.** Punktionsnadel-Positionierungssystem nach Anspruch 1, wobei die erste Sonde (11) mit drei ersten Positionierungsvorrichtungen (4) versehen ist, die drei ersten Positionierungsvorrichtungen (4) auf derselben horizontalen Ebene angeordnet sind, die mit einer axialen Ebene der ersten Sonde (11) zusammenfällt, zwei der drei ersten Positionierungsvorrichtungen (4) auf demselben Querschnitt der ersten Sonde (11) angeordnet sind und die drei ersten Positionierungsvorrichtungen (4) die Scheitel eines rechten Dreiecks bilden.

**3.** Punktionsnadel-Positionierungssystem nach Anspruch 2, wobei die erste Sonde (11) mit einem Sondenbefestigungsmittel (6) versehen ist, das zu der ersten Sonde (11) passt, das Sondenbefestigungsmittel (6) mit drei ersten Einführlöchern zum Installieren der ersten Positionierungsvorrichtungen (4) versehen ist und die Positionen der drei ersten Einführlöcher zu den drei ersten Positionierungsvorrichtungen (4) passen.

**4.** Punktionsnadel-Positionierungssystem nach Anspruch 1, wobei zwei zweite Positionierungsvorrichtungen (5) auf der Punktionsnadel angeordnet sind und die zweiten Positionierungsvorrichtungen (5) jeweils auf der Achse der Punktionsnadel angeordnet sind.

**5.** Punktionsnadel-Positionierungssystem nach Anspruch 4, wobei die Punktionsnadel mit einem Punktionsnadel-Befestigungsmittel (7) versehen ist, das zu der Punktionsnadel passt, und das Punktionsnadel-Befestigungsmittel (7) mit zwei zweiten Einführlöchern zum Installieren der zweiten Positionierungsvorrichtungen (5) versehen ist und die Positionen der zwei zweiten Einführlöcher zu den zwei zweiten Positionierungsvorrichtungen (5) passen.

**6.** Punktionsnadel-Positionierungssystem nach Anspruch 1, wobei die Verarbeitungs- und Anzeigeeinheit (3) umfasst:

ein erstes Datenerfassungsmodul, das verwendet wird, um den Ultraschallabschnitt der Läsion zu erfassen und ihn in dem virtuellen Koordinatensystem anzuzeigen, und das verwendet wird, um die Koordinateninformationen der ersten Sonde (11) zu erfassen, wenn die erste Sonde den Ultraschallabschnitt der Läsion erfasst, und um die erste Sonde in dem virtuellen Koordinatensystem anzuzeigen;
ein Modul zum Bestimmen des geplanten Pfads, das verwendet wird, um den geplanten Pfad L1 in dem virtuellen Koordinatensystem zu bestimmen;
ein zweites Datenerfassungsmodul, das verwendet wird, um die Koordinateninformationen der zweiten Positionierungsvorrichtungen (5) auf der Punktionsnadel in dem virtuellen Koordinatensystem zu erfassen und die Achse L2 der virtuellen Punktionsnadel und den Scheitel C2 der virtuellen Punktionsnadel anzuzeigen; und
ein Vergleichsmodul, das verwendet wird, um die Achse L2 der virtuellen Punktionsnadel und den geplanten Pfad L1 in dem virtuellen Koordinatensystem zu vergleichen, um zu sehen, ob sie übereinstimmen.

**7.** Punktionsnadel-Positionierungssystem nach Anspruch 6, wobei in dem Modul zum Bestimmen des geplanten Pfads gemäß den Koordinateninformationen der ersten Sonde (11) in dem virtuellen Koordinatensystem die Position einer Projektion der Achse der ersten Sonde (11) auf den Ultraschallabschnitt der Läsion durch die planare Matrixumwandlung erhalten wird und dadurch der geplante Pfad L1 bestimmt wird.

**8.** Punktionsnadel-Positionierungssystem nach Anspruch 7, wobei der Ultraschallabschnitt der Läsion fächerförmig ist, das virtuelle Koordinatensystem den Scheitelpunkt C1 des fächerförmigen Ultraschallabschnitts der Läsion als den Ursprung nimmt und die planare Matrixumwandlung gemäß einer Transformationsformel I durchgeführt wird, um Koordinateninformationen der Projektion der Achse der ersten Sonde (11) auf den Ultraschallabschnitt der Läsion zu erhalten,

wobei die Transformationsformel I angegeben ist durch

$$(x', y', z', 1) = T(x, y, z, 1)^T,$$

wobei $(x', y', z')^T$ die Koordinateninformationen eines Punkts auf der Achse der ersten Sonde nach der Transformation darstellt, $(x, y, z)^T$ die Koordinateninformationen des Punkts vor der Transformation darstellt, wobei

$$T = T_4T_3T_2T_1$$

$$T_4 = \begin{bmatrix} 1 & 0 & 0 & d_x \\ 0 & 1 & 0 & d_y \\ 0 & 0 & 1 & d_z \\ 0 & 0 & 0 & 1 \end{bmatrix},$$

$$\vec{d} = \left(-\overrightarrow{P_1Q_1'} \cdot \vec{n_1}\right)\vec{n_1},$$

$$\vec{d} = (d_x, d_y, d_z),$$

$$Q_1' = T_3T_2T_1Q_1;$$

$$T_3 = \begin{bmatrix} 1 & 0 & 0 & q_x^1 \\ 0 & 1 & 0 & q_y^1 \\ 0 & 0 & 1 & q_z^1 \\ 0 & 0 & 0 & 1 \end{bmatrix};$$

$$T_2 = \begin{bmatrix} 1 & 0 & 0 & 0 \\ 0 & \cos\theta_x^1 & \sin\theta_x^1 & 0 \\ 0 & -\sin\theta_x^1 & \cos\theta_x^1 & 0 \\ 0 & 0 & 0 & 1 \end{bmatrix} \begin{bmatrix} \cos\theta_y^1 & 0 & \sin\theta_y^1 & 0 \\ 0 & 1 & 0 & 0 \\ -\sin\theta_y^1 & 0 & \cos\theta_y^1 & 0 \\ 0 & 0 & 0 & 1 \end{bmatrix} \begin{bmatrix} \cos\theta_y^2 & 0 & -\sin\theta_y^2 & 0 \\ 0 & 1 & 0 & 0 \\ \sin\theta_y^2 & 0 & \cos\theta_y^2 & 0 \\ 0 & 0 & 0 & 1 \end{bmatrix} \begin{bmatrix} 1 & 0 & 0 & 0 \\ 0 & \cos\theta_x^2 & -\sin\theta_x^2 & 0 \\ 0 & \sin\theta_x^2 & \cos\theta_x^2 & 0 \\ 0 & 0 & 0 & 1 \end{bmatrix}$$

$$\cos\theta_x^2 = \frac{n_z^2}{\sqrt{(n_y^2)^2 + (n_z^2)^2}}, \sin\theta_x^2 = \frac{n_y^2}{\sqrt{(n_y^2)^2 + (n_z^2)^2}},$$

$$\cos\theta_y^2 = \frac{\sqrt{\left(n_y^2\right)^2 + \left(n_z^2\right)^2}}{\sqrt{\left(n_x^2\right)^2 + \left(n_y^2\right)^2 + \left(n_z^2\right)^2}}, \sin\theta_y^2 = \frac{n_x^2}{\sqrt{\left(n_x^2\right)^2 + \left(n_y^2\right)^2 + \left(n_z^2\right)^2}},$$

$$\cos\theta_x^1 = \frac{n_z^1}{\sqrt{\left(n_y^1\right)^2 + \left(n_z^1\right)^2}}, \sin\theta_x^1 = \frac{n_y^1}{\sqrt{\left(n_y^1\right)^2 + \left(n_z^1\right)^2}},$$

$$\cos\theta_y^1 = \frac{\sqrt{\left(n_y^1\right)^2 + \left(n_z^1\right)^2}}{\sqrt{\left(n_x^1\right)^2 + \left(n_y^1\right)^2 + \left(n_z^1\right)^2}}, \sin\theta_y^1 = \frac{n_x^1}{\sqrt{\left(n_x^1\right)^2 + \left(n_y^1\right)^2 + \left(n_z^1\right)^2}},$$

$$\vec{n}_1 = \left(n_x^1, n_y^1, n_z^1\right), \ \vec{n}_2 = \left(n_x^2, n_y^2, n_z^2\right);$$

$$\vec{n}_1 = \frac{\overrightarrow{P_1P_2} \times \overrightarrow{P_1P_3}}{\left|\overrightarrow{P_1P_2}\right| \left|\overrightarrow{P_1P_3}\right|}, \ \vec{n}_2 = \frac{\overrightarrow{Q_1Q_2} \times \overrightarrow{Q_1Q_3}}{\left|\overrightarrow{Q_1Q_2}\right| \left|\overrightarrow{Q_1Q_3}\right|},$$

$$T_1 = \begin{bmatrix} 1 & 0 & 0 & -q_x^1 \\ 0 & 1 & 0 & -q_y^1 \\ 0 & 0 & 1 & -q_z^1 \\ 0 & 0 & 0 & 1 \end{bmatrix}$$

$P_1$, $P_2$, $P_3$ drei nicht-kollineare Abtastpunkte auf dem Ultraschallabschnitt F1 der Läsion sind und $Q_1$, $Q_2$, $Q_3$ drei nicht-kollineare Punkte auf einer axialen Ebene $F_2$ einer virtuellen ersten Sonde sind, $P_1\left(p_x^1, p_y^1, p_z^1\right)$, $P_2\left(p_x^2, p_y^2, p_z^2\right)$, $P_3\left(p_x^3, p_y^3, p_z^3\right)$, $Q_1\left(q_x^1, q_y^1, q_z^1\right)$, $Q_2\left(q_x^2, q_y^2, q_z^2\right)$, $Q_3\left(q_x^3, q_y^3, q_z^3\right)$ die Koordinaten von $P_1$, $P_2$, $P_3$, $Q_1$, $Q_2$ und $Q_3$ sind,
$\vec{n}_1$ ein Einheitsnormalenvektor des Ultraschallabschnitts F1 der Läsion ist und $\vec{n}_2$ ein Einheitsnormalenvektor der axialen Ebene $F_2$ der virtuellen ersten Sonde ist.

**9.** Punktionsnadel-Positionierungssystem nach Anspruch 6, wobei in dem zweiten Datenerfassungsmodul gemäß den Koordinateninformationen der zweiten Positionierungsvorrichtungen (5) in dem virtuellen Koordinatensystem eine planare Matrixumwandlung durchgeführt wird, um transformierte Koordinateninformationen der Achse L2 der virtuellen Punktionsnadel zu erhalten, die Achse L2 der virtuellen Punktionsnadel durch Projizieren der Achse der Punktionsnadel auf den Ultraschallabschnitt der Läsion erhalten wird und eine planare Matrixumwandlung durchgeführt wird, um transformierte Koordinateninformationen des Scheitels C2 der virtuellen Punktionsnadel zu erhalten, der Scheitel C2 der virtuellen Punktionsnadel durch Projizieren des Scheitelpunkts der Punktionsnadel auf den Ultraschallabschnitt der Läsion erhalten wird, die Achse und der Scheitelpunkt der virtuellen Punktionsnadel dann in dem virtuellen Koordinatensystem gemäß ihren transformierten Koordinateninformationen angezeigt werden.

**10.** Punktionsnadel-Positionierungssystem nach Anspruch 6, wobei in dem Vergleichsmodul in dem virtuellen Koordinatensystem der Scheitel C2 der virtuellen Punktionsnadel mit dem Scheitelpunkt C1 des Ultraschallabschnitts

verglichen wird, um zu sehen, ob sie übereinstimmen, und die Richtung der Achse L2 der virtuellen Punktionsnadel mit der Richtung des geplanten Pfads L1 verglichen wird, um zu sehen, ob sie übereinstimmen, wobei, wenn der Scheitel C2 der virtuellen Punktionsnadel und der Scheitelpunkt C1 des Ultraschallabschnitts übereinstimmen und die Richtung der Achse L2 der virtuellen Punktionsnadel und die Richtung des geplanten Pfads L1 übereinstimmen, eine Übereinstimmungsbeurteilung zurückgegeben wird.

**11.** Computerlesbares Speichermedium, umfassend Anweisungen, die, wenn sie von der Verarbeitungs- und Anzeigeeinheit eines Systems nach den Ansprüchen 1 bis 10 ausgeführt werden, die Verarbeitungs- und Anzeigeeinheit veranlassen, ein Verfahren zum Positionieren einer Punktionsnadel auszuführen, wobei das Verfahren umfasst:

S100 Erhalten eines Ultraschallabschnitts einer Läsion und Anzeigen desselben in einem virtuellen Koordinatensystem; Erhalten der Koordinateninformationen einer ersten Sonde (11), wenn die erste Sonde (11) den Ultraschallabschnitt der Läsion erfasst, und Anzeigen der ersten Sonde (11) in dem virtuellen Koordinatensystem;
S200 Bestimmen eines geplanten Pfads L1 in dem virtuellen Koordinatensystem;
S300 Erhalten der Koordinateninformationen von zweiten Positionierungsvorrichtungen (5) auf der Punktionsnadel in dem virtuellen Koordinatensystem und Anzeigen der Achse L2 einer virtuellen Punktionsnadel und des Scheitels C2 der virtuellen Punktionsnadel; und
S400 in dem virtuellen Koordinatensystem, Vergleichen der Achse L2 der virtuellen Punktionsnadel mit dem geplanten Pfad L1, um zu sehen, ob sie übereinstimmen;
wobei bei S200 gemäß den Koordinateninformationen der ersten Sonde (11) in dem virtuellen Koordinatensystem die Position einer Achse (L) der ersten Sonde (11) auf dem Ultraschallabschnitt der Läsion durch eine planare Matrixumwandlung erhalten wird und dadurch der geplante Pfad L1 bestimmt wird.

**12.** Computerlesbares Speichermedium nach Anspruch 11, wobei der Ultraschallabschnitt der Läsion fächerförmig ist, das virtuelle Koordinatensystem den Scheitelpunkt C1 des fächerförmigen Ultraschallabschnitts der Läsion als den Ursprung nimmt und die planare Matrixumwandlung gemäß einer Transformationsformel I durchgeführt wird, um transformierte Koordinateninformationen einer Projektion der Achse der ersten Sonde (11) auf den Ultraschallabschnitt der Läsion zu erhalten,
wobei die Transformationsformel I angegeben ist durch:

$$\left(x^{'}, y^{'}, z^{'}, 1\right) = T(x, y, z, 1)^T,$$

wobei $(x', y', z')^T$ die Koordinateninformationen eines Punkts auf der Achse der ersten Sonde nach der Transformation darstellt, $(x, y, z)^T$ die Koordinateninformationen des Punkts vor der Transformation darstellt, wobei

$$T = T_4T_3T_2T_1$$

$$T_4 = \begin{bmatrix} 1 & 0 & 0 & d_x \\ 0 & 1 & 0 & d_y \\ 0 & 0 & 1 & d_z \\ 0 & 0 & 0 & 1 \end{bmatrix},$$

$$\vec{d} = \left(-\overrightarrow{P_1Q_1^{'}} \cdot \vec{n_1}\right)\vec{n_1},$$

$$\vec{d} = \left(d_x, d_y, d_z\right),$$

$$Q_1^{'} = T_3 T_2 T_1 Q_1 \ ;$$

$$T_3 = \begin{bmatrix} 1 & 0 & 0 & q_x^1 \\ 0 & 1 & 0 & q_y^1 \\ 0 & 0 & 1 & q_z^1 \\ 0 & 0 & 0 & 1 \end{bmatrix};$$

$$T_2 = \begin{bmatrix} 1 & 0 & 0 & 0 \\ 0 & \cos\theta_x^1 & \sin\theta_x^1 & 0 \\ 0 & -\sin\theta_x^1 & \cos\theta_x^1 & 0 \\ 0 & 0 & 0 & 1 \end{bmatrix} \begin{bmatrix} \cos\theta_y^1 & 0 & \sin\theta_y^1 & 0 \\ 0 & 1 & 0 & 0 \\ -\sin\theta_y^1 & 0 & \cos\theta_y^1 & 0 \\ 0 & 0 & 0 & 1 \end{bmatrix} \begin{bmatrix} \cos\theta_y^2 & 0 & -\sin\theta_y^2 & 0 \\ 0 & 1 & 0 & 0 \\ \sin\theta_y^2 & 0 & \cos\theta_y^2 & 0 \\ 0 & 0 & 0 & 1 \end{bmatrix} \begin{bmatrix} 1 & 0 & 0 & 0 \\ 0 & \cos\theta_x^2 & -\sin\theta_x^2 & 0 \\ 0 & \sin\theta_x^2 & \cos\theta_x^2 & 0 \\ 0 & 0 & 0 & 1 \end{bmatrix}$$

,

$$\cos\theta_x^2 = \frac{n_z^2}{\sqrt{\left(n_y^2\right)^2 + \left(n_z^2\right)^2}}, \sin\theta_x^2 = \frac{n_y^2}{\sqrt{\left(n_y^2\right)^2 + \left(n_z^2\right)^2}},$$

$$\cos\theta_y^2 = \frac{\sqrt{\left(n_y^2\right)^2 + \left(n_z^2\right)^2}}{\sqrt{\left(n_x^2\right)^2 + \left(n_y^2\right)^2 + \left(n_z^2\right)^2}}, \sin\theta_y^2 = \frac{n_x^2}{\sqrt{\left(n_x^2\right)^2 + \left(n_y^2\right)^2 + \left(n_z^2\right)^2}},$$

$$\cos\theta_x^1 = \frac{n_z^1}{\sqrt{\left(n_y^1\right)^2 + \left(n_z^1\right)^2}}, \sin\theta_x^1 = \frac{n_y^1}{\sqrt{\left(n_y^1\right)^2 + \left(n_z^1\right)^2}},$$

$$\cos\theta_y^1 = \frac{\sqrt{\left(n_y^1\right)^2 + \left(n_z^1\right)^2}}{\sqrt{\left(n_x^1\right)^2 + \left(n_y^1\right)^2 + \left(n_z^1\right)^2}}, \sin\theta_y^1 = \frac{n_x^1}{\sqrt{\left(n_x^1\right)^2 + \left(n_y^1\right)^2 + \left(n_z^1\right)^2}},$$

$$\overrightarrow{n_1} = \frac{\overrightarrow{P_1P_2} \times \overrightarrow{P_1P_3}}{\left|\overrightarrow{P_1P_2}\right| \left|\overrightarrow{P_1P_3}\right|}, \quad \overrightarrow{n_2} = \frac{\overrightarrow{Q_1Q_2} \times \overrightarrow{Q_1Q_3}}{\left|\overrightarrow{Q_1Q_2}\right| \left|\overrightarrow{Q_1Q_3}\right|},$$

$$\overrightarrow{n_1} = \left(n_x^1, n_y^1, n_z^1\right), \quad \overrightarrow{n_2} = \left(n_x^2, n_y^2, n_z^2\right);$$

$$T_1 = \begin{bmatrix} 1 & 0 & 0 & -q_x^1 \\ 0 & 1 & 0 & -q_y^1 \\ 0 & 0 & 1 & -q_z^1 \\ 0 & 0 & 0 & 1 \end{bmatrix},$$

$P_1$, $P_2$, $P_3$ drei nicht-kollineare Abtastpunkte auf dem Ultraschallabschnitt F1 der Läsion sind und $Q_1$, $Q_2$, $Q_3$ drei nicht-kollineare Punkte auf einer axialen Ebene $F_2$ einer virtuellen ersten Sonde sind, $P_1\left(p_x^1, p_y^1, p_z^1\right)$, $P_2\left(p_x^2, p_y^2, p_z^2\right)$, $P_3\left(p_x^3, p_y^3, p_z^3\right)$, $Q_1\left(q_x^1, q_y^1, q_z^1\right)$, $Q_2\left(q_x^2, q_y^2, q_z^2\right)$, $Q_3\left(q_x^3, q_y^3, q_z^3\right)$ die Koordinaten von $P_1$, $P_2$, $P_3$, $Q_1$, $Q_2$ und $Q_3$ sind,
$\overline{n}_1$ ein Einheitsnormalenvektor des Ultraschallabschnitts F1 der Läsion ist und $\vec{n}_2$ ein Einheitsnormalenvektor der axialen Ebene $F_2$ der virtuellen ersten Sonde ist.

**13.** Computerlesbares Speichermedium nach Anspruch 11, wobei bei S300 gemäß den Koordinateninformationen der zweiten Positionierungsvorrichtungen (5) in dem virtuellen Koordinatensystem eine planare Matrixumwandlung durchgeführt wird, um transformierte Koordinateninformationen der Achse L2 der virtuellen Punktionsnadel zu erhalten, die Achse L2 der virtuellen Punktionsnadel durch Projizieren der Achse der Punktionsnadel auf den Ultraschallabschnitt der Läsion erhalten wird, eine planare Matrixumwandlung durchgeführt wird, um transformierte Koordinateninformationen des Scheitels C2 der virtuellen Punktionsnadel zu erhalten, der Scheitel C2 der virtuellen Punktionsnadel durch Projizieren des Scheitelpunkts der Punktionsnadel auf den Ultraschallabschnitt der Läsion erhalten wird, die Achse und der Scheitelpunkt der virtuellen Punktionsnadel dann in dem virtuellen Koordinatensystem gemäß ihren transformierten Koordinateninformationen angezeigt werden.

**14.** Computerlesbares Speichermedium nach Anspruch 11, wobei bei S400 in dem virtuellen Koordinatensystem der Scheitel C2 der virtuellen Punktionsnadel mit dem Scheitelpunkt C1 des Ultraschallabschnitts verglichen wird, um zu sehen, ob sie übereinstimmen, und die Richtung der Achse L2 der virtuellen Punktionsnadel mit der Richtung des geplanten Pfads L1 verglichen wird, um zu sehen, ob sie übereinstimmen, wobei, wenn der Scheitel C2 der virtuellen Punktionsnadel und der Scheitelpunkt C1 des Ultraschallabschnitts übereinstimmen und die Richtung der Achse L2 der virtuellen Punktionsnadel und die Richtung des geplanten Pfads L1 übereinstimmen, eine Übereinstimmungsbeurteilung zurückgegeben wird.

## Revendications

**1.** Système de positionnement d'aiguille de ponction, comprenant :

une unité ultrasonore (1), comprenant une première sonde (11) pour fournir une section ultrasonore d'une lésion, dans lequel une pluralité de premiers dispositifs de positionnement (4) pour fournir des informations de coordonnées de la première sonde (11) sont fournis sur la première sonde (11) ;
une unité d'aiguille de ponction (2), comprenant une aiguille de ponction, dans lequel une pluralité de seconds dispositifs de positionnement (5) pour fournir des informations de coordonnées de l'aiguille de ponction sont fournis sur l'aiguille de ponction ;
une unité de traitement et d'affichage (3), connectée en communication avec l'unité ultrasonore (1), chacun des premiers dispositifs de positionnement (4), et chacun des seconds dispositifs de positionnement (5) respectivement, dans lequel l'unité de traitement et d'affichage (3) est utilisée pour : acquérir la section ultrasonore de la lésion, et l'afficher dans un système de coordonnées virtuel ; acquérir les informations de coordonnées de la première sonde (11) lorsque la première sonde est en train d'acquérir la section ultrasonore de la lésion, et afficher la première sonde dans le système de coordonnées virtuel en tant que première sonde virtuelle ; déterminer un chemin planifié L1 dans le système de coordonnées virtuel ; obtenir les informations de coordonnées de l'aiguille de ponction fournies par chacun des seconds dispositifs de positionnement (5) et afficher un axe L2 d'une aiguille de ponction virtuelle et un vertex C2 de l'aiguille de ponction virtuelle dans le

système de coordonnées virtuel ; et dans le système de coordonnées virtuel, comparer l'axe L2 de l'aiguille de ponction virtuelle avec le chemin planifié L1 pour voir s'ils coïncident, **caractérisé en ce que** le chemin planifié L1 dans le système de coordonnées virtuel est déterminé en obtenant la position d'un axe (L) de la première sonde (11) sur la section ultrasonore de la lésion par le biais d'une conversion de matrice planaire selon les informations de coordonnées de la première sonde (11) dans le système de coordonnées virtuel.

**2.** Système de positionnement d'aiguille de ponction selon la revendication 1, dans lequel la première sonde (11) est fournie de trois premiers dispositifs de positionnement (4), les trois premiers dispositifs de positionnement (4) sont situés sur le même plan horizontal, qui coïncide avec un plan axial de la première sonde (11), deux des trois premiers dispositifs de positionnement (4) sont situés sur la même section transversale de la première sonde (11), et les trois premiers dispositifs de positionnement (4) constituent les vertex d'un triangle droit.

**3.** Système de positionnement d'aiguille de ponction selon la revendication 2, dans lequel la première sonde (11) est fournie d'un élément de fixation de la sonde (6) qui correspond à la première sonde (11), l'élément de fixation de la sonde (6) est fourni de trois premiers trous d'insertion pour installer les premiers dispositifs de positionnement (4), et les positions des trois premiers trous d'insertion correspondent aux trois premiers dispositifs de positionnement (4).

**4.** Système de positionnement d'aiguille de ponction selon la revendication 1, dans lequel deux seconds dispositifs de positionnement (5) sont agencés sur l'aiguille de ponction, et les seconds dispositifs de positionnement (5) sont respectivement agencés sur l'axe de l'aiguille de ponction.

**5.** Système de positionnement d'aiguille de ponction selon la revendication 4, dans lequel l'aiguille de ponction est fournie d'un élément de fixation d'aiguille de ponction (7) correspondant à l'aiguille de ponction, et l'élément de fixation d'aiguille de ponction (7) est fourni de deux seconds trous d'insertion pour installer les seconds dispositifs de positionnement (5), et les positions des deux seconds trous d'insertion correspondent aux deux seconds dispositifs de positionnement (5).

**6.** Système de positionnement d'aiguille de ponction selon la revendication 1, dans lequel l'unité de traitement et d'affichage (3) comprend :

un premier module d'acquisition de données, utilisé pour acquérir la section ultrasonore de la lésion et l'afficher dans le système de coordonnées virtuel, et utilisé pour acquérir les informations de coordonnées de la première sonde (11) lorsque la première sonde est en train d'acquérir la section ultrasonore de la lésion et pour afficher la première sonde dans le système de coordonnées virtuel ;
un module de détermination de chemin planifié, utilisé pour déterminer le chemin planifié L1 dans le système de coordonnées virtuel ;
un second module d'acquisition de données, utilisé pour acquérir les informations de coordonnées des seconds dispositifs de positionnement (5) sur l'aiguille de ponction dans le système de coordonnées virtuel et afficher l'axe L2 de l'aiguille de ponction virtuelle et le vertex C2 de l'aiguille de ponction virtuelle ; et
un module de comparaison, utilisé pour comparer l'axe L2 de l'aiguille de ponction virtuelle et le chemin planifié L1 dans le système de coordonnées virtuel pour voir s'ils coïncident.

**7.** Système de positionnement d'aiguille de ponction selon la revendication 6, dans lequel, dans le module de détermination de chemin planifié, selon les informations de coordonnées de la première sonde (11) dans le système de coordonnées virtuel, la position d'une projection de l'axe de la première sonde (11) sur la section ultrasonore de la lésion est obtenue par le biais de la conversion matricielle planaire, et le chemin planifié L1 est ainsi déterminé.

**8.** Système de positionnement d'aiguille de ponction selon la revendication 7, dans lequel la section ultrasonore de la lésion est en forme d'éventail, le système de coordonnées virtuel prend le point de sommet C1 de la section ultrasonore en forme d'éventail de la lésion comme origine, et la conversion matricielle planaire est effectuée selon une formule de transformation I pour obtenir des informations de coordonnées de la projection de l'axe de la première sonde (11) sur la section ultrasonore de la lésion ;

dans lequel la formule de transformation I est donnée par

$$\left(x',y',z',1\right)=T(x,y,z,1)^T,$$

dans lequel $(x', y', z', 1)^T$ représente les informations de coordonnées d'un point sur l'axe de la première sonde après la transformation, $(x, y, z)^T$ représente les informations de coordonnées du point avant la transformation, dans lequel

$$T=T_4T_3T_2T_1$$

$$T_4=\begin{bmatrix}1&0&0&d_x\\0&1&0&d_y\\0&0&1&d_z\\0&0&0&1\end{bmatrix},$$

$$\overrightarrow{d}=\left(-\overrightarrow{P_1Q_1'}\cdot\overrightarrow{n_1}\right)\overrightarrow{n_1},$$

$$\overrightarrow{d}=\left(d_x,d_y,d_z\right),$$

$$Q_1'=T_3T_2T_1Q_1;$$

$$T_3=\begin{bmatrix}1&0&0&q_x^1\\0&1&0&q_y^1\\0&0&1&q_z^1\\0&0&0&1\end{bmatrix};$$

$$T_2=\begin{bmatrix}1&0&0&0\\0&\cos\theta_x^1&\sin\theta_x^1&0\\0&-\sin\theta_x^1&\cos\theta_x^1&0\\0&0&0&1\end{bmatrix}\begin{bmatrix}\cos\theta_y^1&0&\sin\theta_y^1&0\\0&1&0&0\\-\sin\theta_y^1&0&\cos\theta_y^1&0\\0&0&0&1\end{bmatrix}\begin{bmatrix}\cos\theta_y^2&0&-\sin\theta_y^2&0\\0&1&0&0\\\sin\theta_y^2&0&\cos\theta_y^2&0\\0&0&0&1\end{bmatrix}\begin{bmatrix}1&0&0&0\\0&\cos\theta_x^2&-\sin\theta_x^2&0\\0&\sin\theta_x^2&\cos\theta_x^2&0\\0&0&0&1\end{bmatrix}$$

$$\cos\theta_x^2=\frac{n_z^2}{\sqrt{\left(n_y^2\right)^2+\left(n_z^2\right)^2}},\sin\theta_x^2=\frac{n_y^2}{\sqrt{\left(n_y^2\right)^2+\left(n_z^2\right)^2}},$$

$$\cos\theta_y^2=\frac{\sqrt{\left(n_y^2\right)^2+\left(n_z^2\right)^2}}{\sqrt{\left(n_x^2\right)^2+\left(n_y^2\right)^2+\left(n_z^2\right)^2}},\sin\theta_y^2=\frac{n_x^2}{\sqrt{\left(n_x^2\right)^2+\left(n_y^2\right)^2+\left(n_z^2\right)^2}},$$

$$\cos\theta_x^1 = \frac{n_z^1}{\sqrt{\left(n_y^1\right)^2 + \left(n_z^1\right)^2}}, \sin\theta_x^1 = \frac{n_y^1}{\sqrt{\left(n_y^1\right)^2 + \left(n_z^1\right)^2}},$$

$$\cos\theta_y^1 = \frac{\sqrt{\left(n_y^1\right)^2 + \left(n_z^1\right)^2}}{\sqrt{\left(n_x^1\right)^2 + \left(n_y^1\right)^2 + \left(n_z^1\right)^2}}, \sin\theta_y^1 = \frac{n_x^1}{\sqrt{\left(n_x^1\right)^2 + \left(n_y^1\right)^2 + \left(n_z^1\right)^2}},$$

$$\vec{n}_1 = \left(n_x^1, n_y^1, n_z^1\right), \quad \vec{n}_2 = \left(n_x^2, n_y^2, n_z^2\right);$$

$$\vec{n}_1 = \frac{\overrightarrow{P_1P_2} \times \overrightarrow{P_1P_3}}{\left|\overrightarrow{P_1P_2}\right| \left|\overrightarrow{P_1P_3}\right|}, \quad \vec{n}_2 = \frac{\overrightarrow{Q_1Q_2} \times \overrightarrow{Q_1Q_3}}{\left|\overrightarrow{Q_1Q_2}\right| \left|\overrightarrow{Q_1Q_3}\right|},$$

$$T_1 = \begin{bmatrix} 1 & 0 & 0 & -q_x^1 \\ 0 & 1 & 0 & -q_y^1 \\ 0 & 0 & 1 & -q_z^1 \\ 0 & 0 & 0 & 1 \end{bmatrix}$$

$P_1$, $P_2$, $P_3$ sont trois points d'échantillonnage non-colinéaire sur la section ultrasonore F1 de la lésion, et $Q_1$, $Q_2$, $Q_3$ sont trois points non-colinéaire sur un plan axial $F_2$ d'une première sonde virtuelle,

$$P_1\left(p_x^1, p_y^1, p_z^1\right), \quad P_2\left(p_x^2, p_y^2, p_z^2\right),$$

$$P_3\left(p_x^3, p_y^3, p_z^3\right), \quad Q_1\left(q_x^1, q_y^1, q_z^1\right), \quad Q_2\left(q_x^2, q_y^2, q_z^2\right), \quad Q_3\left(q_x^3, q_y^3, q_z^3\right)$$

sont les coordonnées de $P_1$, $P_2$, $P_3$, $Q_1$, $Q_2$, et $Q_3$,

$\vec{n}_1$ est un vecteur normal unitaire de la section ultrasonore F1 de la lésion, et $\vec{n}_2$ est un vecteur normal unitaire du plan axial $F_2$ de la première sonde virtuelle.

**9.** Système de positionnement d'aiguille de ponction selon la revendication 6, dans lequel, dans le second module d'acquisition de données, selon les informations de coordonnées des seconds dispositifs de positionnement (5) dans le système de coordonnées virtuel, une conversion matricielle planaire est effectuée pour obtenir des informations de coordonnées transformées de l'axe L2 de l'aiguille de ponction virtuelle, l'axe L2 de l'aiguille de ponction virtuelle est obtenu en projetant l'axe de l'aiguille de ponction sur la section ultrasonore de la lésion, et une conversion matricielle planaire est effectuée pour obtenir des informations de coordonnées transformées du vertex C2 de l'aiguille de ponction virtuelle, le vertex C2 de l'aiguille de ponction virtuelle est obtenu en projetant le point de sommet de l'aiguille de ponction sur la section ultrasonore de la lésion, l'axe et le point de sommet de l'aiguille de ponction virtuelle sont ensuite affichés dans le système de coordonnées virtuel selon leurs informations de coordonnées transformées.

**10.** Système de positionnement d'aiguille de ponction selon la revendication 6, dans lequel, dans le module de comparaison, dans le système de coordonnées virtuel, le vertex C2 de l'aiguille de ponction virtuelle est comparé avec le point de sommet C1 de la section ultrasonore pour voir s'ils coïncident, et la direction de l'axe L2 de l'aiguille de ponction virtuelle est comparée avec la direction du chemin planifié L1 pour voir s'ils coïncident, dans lequel, lorsque le vertex C2 de l'aiguille de ponction virtuelle et le point de sommet C1 de la section ultrasonore coïncident, et la direction de l'axe L2 de l'aiguille de ponction virtuelle et la direction du chemin planifié L1 coïncident, un jugement de coïncidence est renvoyé.

**11.** Moyen de stockage lisible par ordinateur comprenant des instructions qui, lorsqu'elles sont exécutées par unité de traitement et d'affichage d'un système selon les revendications 1 à 10, amènent l'unité de traitement et d'affichage à effectuer un procédé pour positionner une aiguille de ponction, dans lequel le procédé comprend :

S100 obtenir une section ultrasonore d'une lésion et l'afficher dans un système de coordonnées virtuel ; obtenir les informations de coordonnées d'une première sonde (11) lorsque la première sonde (11) est en train d'acquérir la section ultrasonore de la lésion, et afficher la première sonde (11) dans le système de coordonnées virtuel ;
S200 déterminer un chemin planifié L1 dans le système de coordonnées virtuel ;
S300 obtenir les informations de coordonnées de seconds dispositifs de positionnement (5) sur l'aiguille de ponction dans le système de coordonnées virtuel et afficher l'axe L2 d'une aiguille de ponction virtuelle et le vertex C2 de l'aiguille de ponction virtuelle ; et
S400 dans le système de coordonnées virtuel, comparer l'axe L2 de l'aiguille de ponction virtuelle avec le chemin planifié L1 pour voir s'ils coïncident ;
dans lequel, à S200, selon les informations de coordonnées de la première sonde (11) dans le système de coordonnées virtuel, la position d'un axe (L) de la première sonde (11) sur la section ultrasonore de la lésion est obtenue par le biais d'une conversion matricielle planaire, et le chemin planifié L1 est ainsi déterminé.

**12.** Moyen de stockage lisible par ordinateur selon la revendication 11, dans lequel la section ultrasonore de la lésion est en forme d'éventail, le système de coordonnées virtuel prend le point de sommet C1 de la section ultrasonore en forme d'éventail de la lésion comme origine, et la conversion matricielle planaire est effectuée selon une formule de transformation I pour obtenir des informations de coordonnées transformées d'une projection de l'axe de la première sonde (11) sur la section ultrasonore de la lésion,
dans lequel la formule de transformation I est donnée par :

$$\left(x', y', z', 1\right) = T(x, y, z, 1)^T,$$

dans lequel $(x', y', z')^T$ représente les informations de coordonnées d'un point sur l'axe de la première sonde après la transformation, $(x, y, z)^T$ représente les informations de coordonnées du point avant la transformation, dans lequel

$$T = T_4T_3T_2T_1$$

$$T_4 = \begin{bmatrix} 1 & 0 & 0 & d_x \\ 0 & 1 & 0 & d_y \\ 0 & 0 & 1 & d_z \\ 0 & 0 & 0 & 1 \end{bmatrix},$$

$$\overrightarrow{d} = \left(-\overrightarrow{P_1Q_1'} \cdot \overrightarrow{n_1}\right)\overrightarrow{n_1},$$

$$\overrightarrow{d} = \left(d_x, d_y, d_z\right),$$

$$Q_1' = T_3T_2T_1Q_1;$$

$$T_3 = \begin{bmatrix} 1 & 0 & 0 & q_x^1 \\ 0 & 1 & 0 & q_y^1 \\ 0 & 0 & 1 & q_z^1 \\ 0 & 0 & 0 & 1 \end{bmatrix};$$

$$T_2 = \begin{bmatrix} 1 & 0 & 0 & 0 \\ 0 & \cos\theta_x^1 & \sin\theta_x^1 & 0 \\ 0 & -\sin\theta_x^1 & \cos\theta_x^1 & 0 \\ 0 & 0 & 0 & 1 \end{bmatrix} \begin{bmatrix} \cos\theta_y^1 & 0 & \sin\theta_y^1 & 0 \\ 0 & 1 & 0 & 0 \\ -\sin\theta_y^1 & 0 & \cos\theta_y^1 & 0 \\ 0 & 0 & 0 & 1 \end{bmatrix} \begin{bmatrix} \cos\theta_y^2 & 0 & -\sin\theta_y^2 & 0 \\ 0 & 1 & 0 & 0 \\ \sin\theta_y^2 & 0 & \cos\theta_y^2 & 0 \\ 0 & 0 & 0 & 1 \end{bmatrix} \begin{bmatrix} 1 & 0 & 0 & 0 \\ 0 & \cos\theta_x^2 & -\sin\theta_x^2 & 0 \\ 0 & \sin\theta_x^2 & \cos\theta_x^2 & 0 \\ 0 & 0 & 0 & 1 \end{bmatrix}$$

,

$$\cos\theta_x^2 = \frac{n_z^2}{\sqrt{(n_y^2)^2 + (n_z^2)^2}}, \sin\theta_x^2 = \frac{n_y^2}{\sqrt{(n_y^2)^2 + (n_z^2)^2}},$$

$$\cos\theta_y^2 = \frac{\sqrt{(n_y^2)^2 + (n_z^2)^2}}{\sqrt{(n_x^2)^2 + (n_y^2)^2 + (n_z^2)^2}}, \sin\theta_y^2 = \frac{n_x^2}{\sqrt{(n_x^2)^2 + (n_y^2)^2 + (n_z^2)^2}},$$

$$\cos\theta_x^1 = \frac{n_z^1}{\sqrt{(n_y^1)^2 + (n_z^1)^2}}, \sin\theta_x^1 = \frac{n_y^1}{\sqrt{(n_y^1)^2 + (n_z^1)^2}},$$

$$\cos\theta_y^1 = \frac{\sqrt{(n_y^1)^2 + (n_z^1)^2}}{\sqrt{(n_x^1)^2 + (n_y^1)^2 + (n_z^1)^2}}, \sin\theta_y^1 = \frac{n_x^1}{\sqrt{(n_x^1)^2 + (n_y^1)^2 + (n_z^1)^2}},$$

$$\vec{n}_1 = \frac{\overrightarrow{P_1P_2} \times \overrightarrow{P_1P_3}}{\left|\overrightarrow{P_1P_2}\right| \left|\overrightarrow{P_1P_3}\right|}, \quad \vec{n}_2 = \frac{\overrightarrow{Q_1Q_2} \times \overrightarrow{Q_1Q_3}}{\left|\overrightarrow{Q_1Q_2}\right| \left|\overrightarrow{Q_1Q_3}\right|},$$

$$\vec{n}_1 = (n_x^1, n_y^1, n_z^1), \quad \vec{n}_2 = (n_x^2, n_y^2, n_z^2);$$

$$T_1 = \begin{bmatrix} 1 & 0 & 0 & -q_x^1 \\ 0 & 1 & 0 & -q_y^1 \\ 0 & 0 & 1 & -q_z^1 \\ 0 & 0 & 0 & 1 \end{bmatrix},$$

$P_1$, $P_2$, $P_3$ sont trois points d'échantillonnage non-colinéaire sur la section ultrasonore F1 de la lésion, et $Q_1$, $Q_2$, $Q_3$ sont trois points non-colinéaire sur un plan axial $F_2$ d'une première sonde virtuelle,

$$P_1(p_x^1, p_y^1, p_z^1),\ P_2(p_x^2, p_y^2, p_z^2),$$

$$P_3(p_x^3, p_y^3, p_z^3),\quad Q_1(q_x^1, q_y^1, q_z^1),\quad Q_2(q_x^2, q_y^2, q_z^2),\quad Q_3(q_x^3, q_y^3, q_z^3)$$ sont les coordonnées de $P_1$, $P_2$, $P_3$, $Q_1$, $Q_2$, et $Q_3$,

$\vec{n}_1$ est un vecteur normal unitaire de la section ultrasonore F1 de la lésion, et $\vec{n}_2$ est un vecteur normal unitaire du plan axial $F_2$ de la première sonde virtuelle.

**13.** Moyen de stockage lisible par ordinateur selon la revendication 11, dans lequel, à S300, selon les informations de coordonnées des seconds dispositifs de positionnement (5) dans le système de coordonnées virtuel, une conversion matricielle planaire est effectuée pour obtenir des informations de coordonnées transformées de l'axe L2 de l'aiguille de ponction virtuelle, l'axe L2 de l'aiguille de ponction virtuelle est obtenu en projetant l'axe de l'aiguille de ponction sur la section ultrasonore de la lésion, une conversion matricielle planaire est effectuée pour obtenir des informations de coordonnées transformées du vertex C2 de l'aiguille de ponction virtuelle, le vertex C2 de l'aiguille de ponction virtuelle est obtenu en projetant le point de sommet de l'aiguille de ponction sur la section ultrasonore de la lésion, l'axe et le point de sommet de l'aiguille de ponction virtuelle sont ensuite affichés dans le système de coordonnées virtuel selon leurs informations de coordonnées transformées.

**14.** Moyen de stockage lisible par ordinateur selon la revendication 11, dans lequel, à S400, dans le système de coordonnées virtuel, le vertex C2 de l'aiguille de ponction virtuelle est comparé avec le point de sommet C1 de la section ultrasonore pour voir s'ils coïncident, et la direction de l'axe L2 de l'aiguille de ponction virtuelle est comparée avec la direction du chemin planifié L1 pour voir s'ils coïncident, dans lequel, lorsque le vertex C2 de l'aiguille de ponction virtuelle et le point de sommet C1 de la section ultrasonore coïncident, et la direction de l'axe L2 de l'aiguille de ponction virtuelle et la direction du chemin planifié L1 coïncident, un jugement de coïncidence est renvoyé.

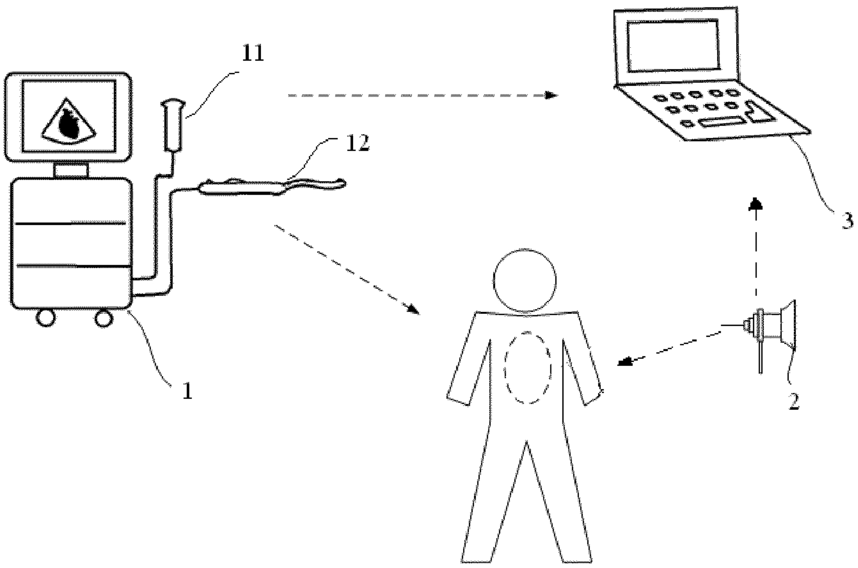


Fig. 1

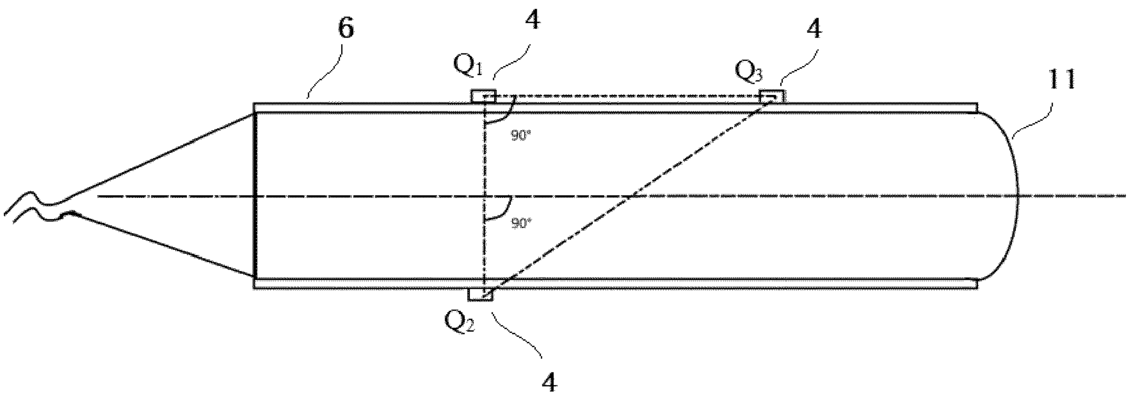


Fig. 2

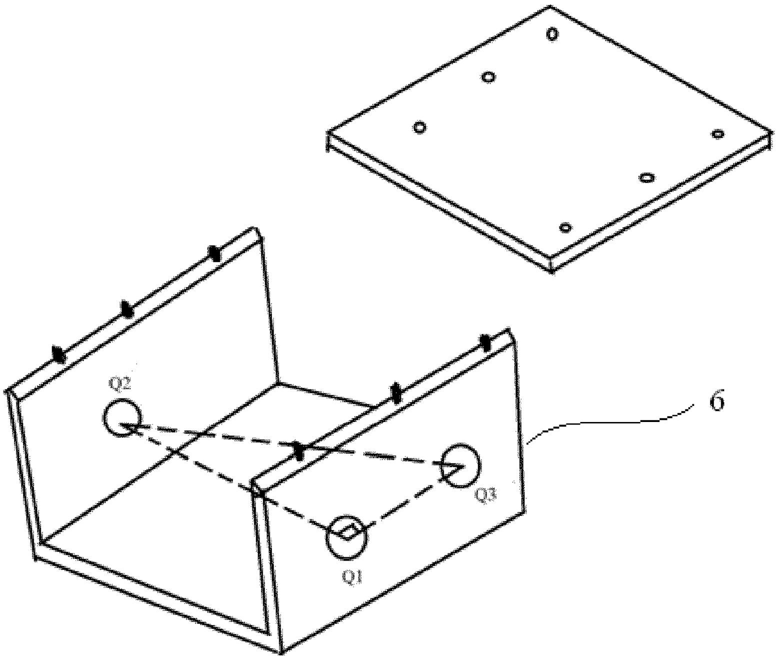


Fig. 3

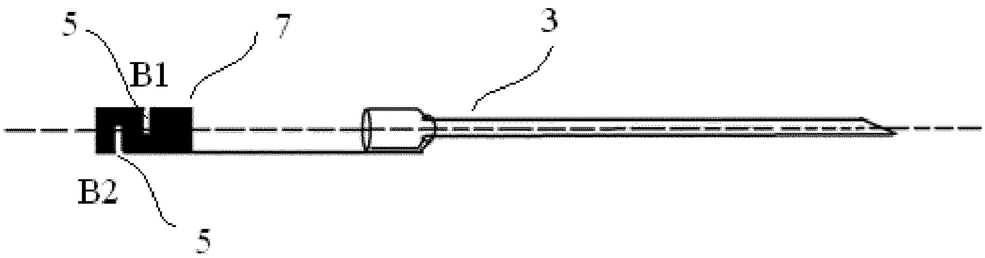


Fig. 4

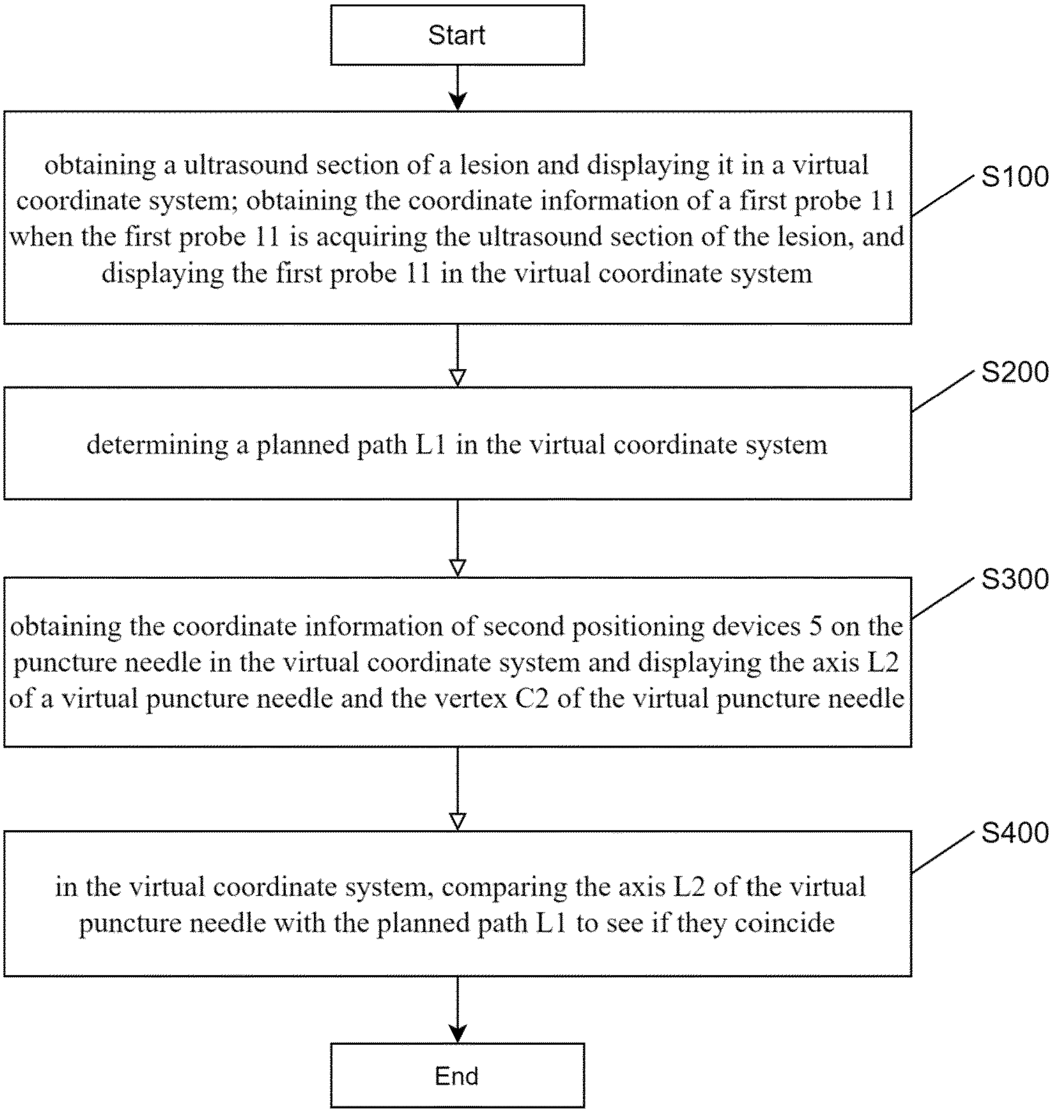
Start
obtaining a ultrasound section of a lesion and displaying it in a virtual coordinate system; obtaining the coordinate information of a first probe 11 when the first probe 11 is acquiring the ultrasound section of the lesion, and displaying the first probe 11 in the virtual coordinate system
S100
determining a planned path L1 in the virtual coordinate system
S200
obtaining the coordinate information of second positioning devices 5 on the puncture needle in the virtual coordinate system and displaying the axis L2 of a virtual puncture needle and the vertex C2 of the virtual puncture needle
S300
in the virtual coordinate system, comparing the axis L2 of the virtual puncture needle with the planned path L1 to see if they coincide
S400
End

Fig. 5

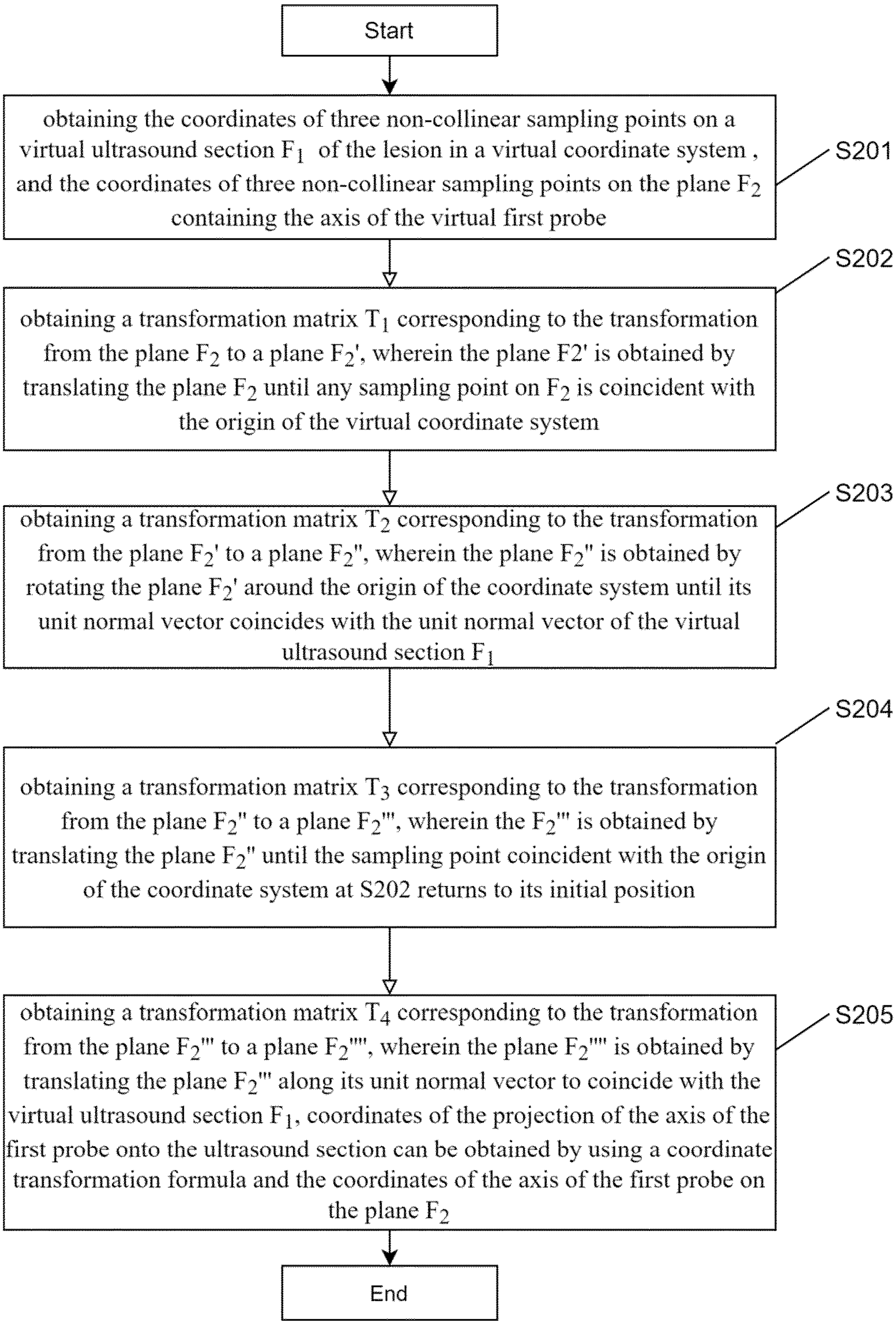
Start
obtaining the coordinates of three non-collinear sampling points on a virtual ultrasound section $F_1$ of the lesion in a virtual coordinate system , and the coordinates of three non-collinear sampling points on the plane $F_2$ containing the axis of the virtual first probe
S201
S202
obtaining a transformation matrix $T_1$ corresponding to the transformation from the plane $F_2$ to a plane $F_2$', wherein the plane F2' is obtained by translating the plane $F_2$ until any sampling point on $F_2$ is coincident with the origin of the virtual coordinate system
S203
obtaining a transformation matrix $T_2$ corresponding to the transformation from the plane $F_2$' to a plane $F_2$'', wherein the plane $F_2$'' is obtained by rotating the plane $F_2$' around the origin of the coordinate system until its unit normal vector coincides with the unit normal vector of the virtual ultrasound section $F_1$
S204
obtaining a transformation matrix $T_3$ corresponding to the transformation from the plane $F_2$'' to a plane $F_2$''', wherein the $F_2$''' is obtained by translating the plane $F_2$'' until the sampling point coincident with the origin of the coordinate system at S202 returns to its initial position
obtaining a transformation matrix $T_4$ corresponding to the transformation from the plane $F_2$''' to a plane $F_2$'''', wherein the plane $F_2$'''' is obtained by translating the plane $F_2$''' along its unit normal vector to coincide with the virtual ultrasound section $F_1$, coordinates of the projection of the axis of the first probe onto the ultrasound section can be obtained by using a coordinate transformation formula and the coordinates of the axis of the first probe on the plane $F_2$
S205
End

Fig. 6

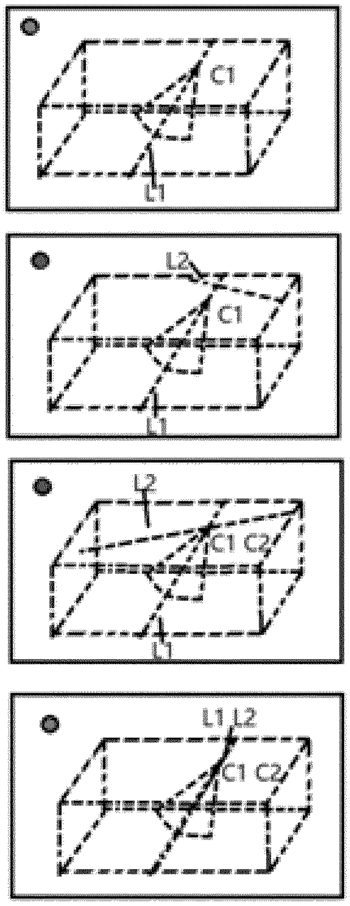
C1
L1
L2
C1
L1
L2
C1 C2
L1
L1 L2
C1 C2

Fig. 7

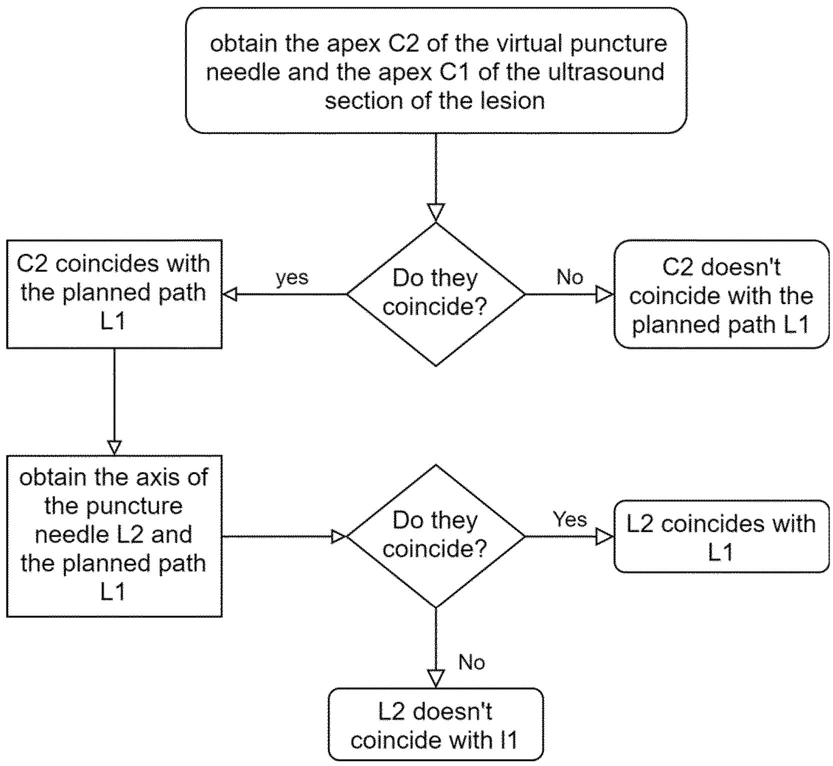
obtain the apex C2 of the virtual puncture needle and the apex C1 of the ultrasound section of the lesion
Do they coincide?
yes
C2 coincides with the planned path L1
No
C2 doesn't coincide with the planned path L1
obtain the axis of the puncture needle L2 and the planned path L1
Do they coincide?
Yes
L2 coincides with L1
No
L2 doesn't coincide with l1

Fig. 8

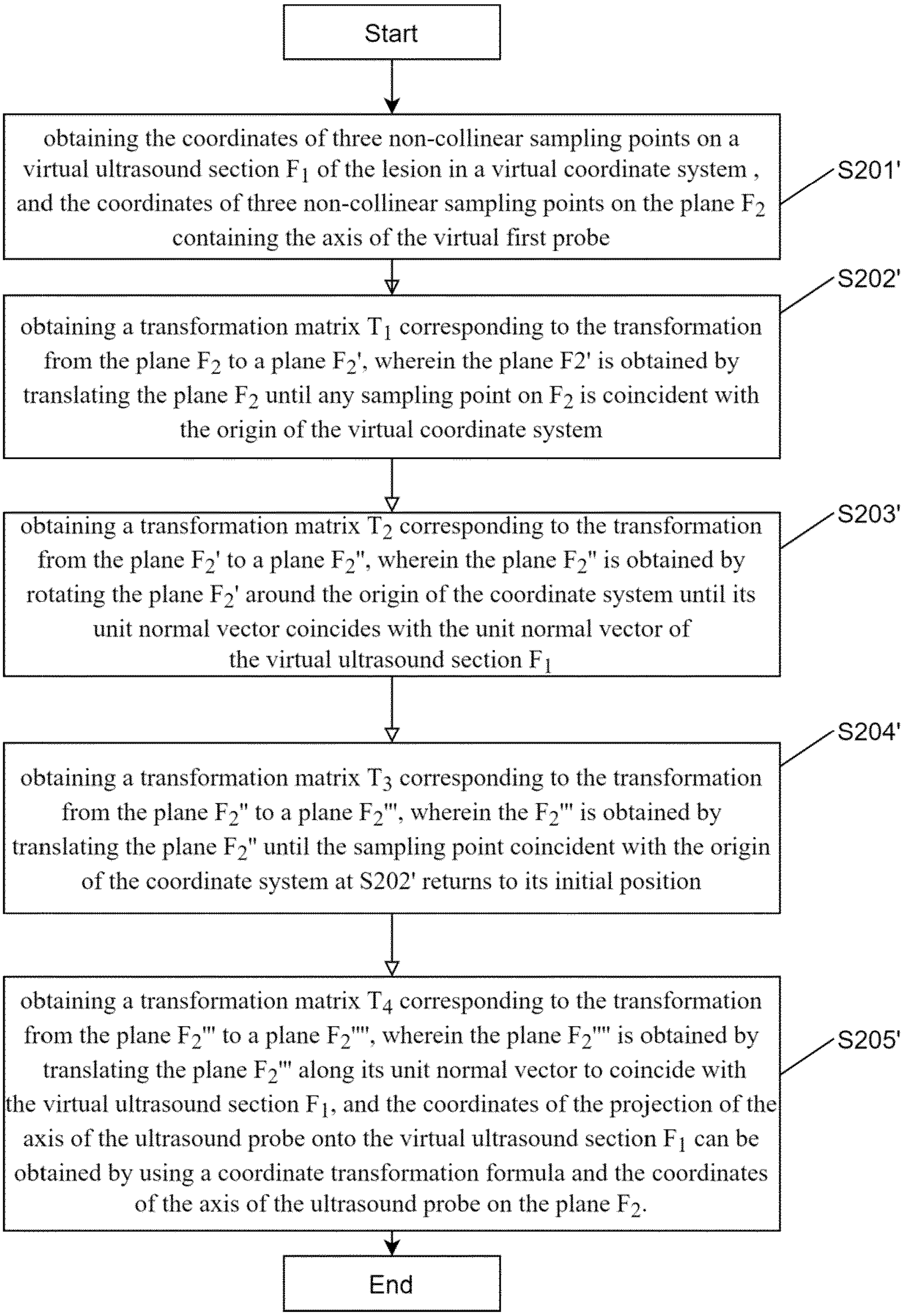
Start
obtaining the coordinates of three non-collinear sampling points on a virtual ultrasound section $F_1$ of the lesion in a virtual coordinate system , and the coordinates of three non-collinear sampling points on the plane $F_2$ containing the axis of the virtual first probe
S201'
obtaining a transformation matrix $T_1$ corresponding to the transformation from the plane $F_2$ to a plane $F_2'$, wherein the plane F2' is obtained by translating the plane $F_2$ until any sampling point on $F_2$ is coincident with the origin of the virtual coordinate system
S202'
obtaining a transformation matrix $T_2$ corresponding to the transformation from the plane $F_2'$ to a plane $F_2''$, wherein the plane $F_2''$ is obtained by rotating the plane $F_2'$ around the origin of the coordinate system until its unit normal vector coincides with the unit normal vector of the virtual ultrasound section $F_1$
S203'
obtaining a transformation matrix $T_3$ corresponding to the transformation from the plane $F_2''$ to a plane $F_2'''$, wherein the $F_2'''$ is obtained by translating the plane $F_2''$ until the sampling point coincident with the origin of the coordinate system at S202' returns to its initial position
S204'
obtaining a transformation matrix $T_4$ corresponding to the transformation from the plane $F_2'''$ to a plane $F_2''''$, wherein the plane $F_2''''$ is obtained by translating the plane $F_2'''$ along its unit normal vector to coincide with the virtual ultrasound section $F_1$, and the coordinates of the projection of the axis of the ultrasound probe onto the virtual ultrasound section $F_1$ can be obtained by using a coordinate transformation formula and the coordinates of the axis of the ultrasound probe on the plane $F_2$.
S205'
End

Fig. 9

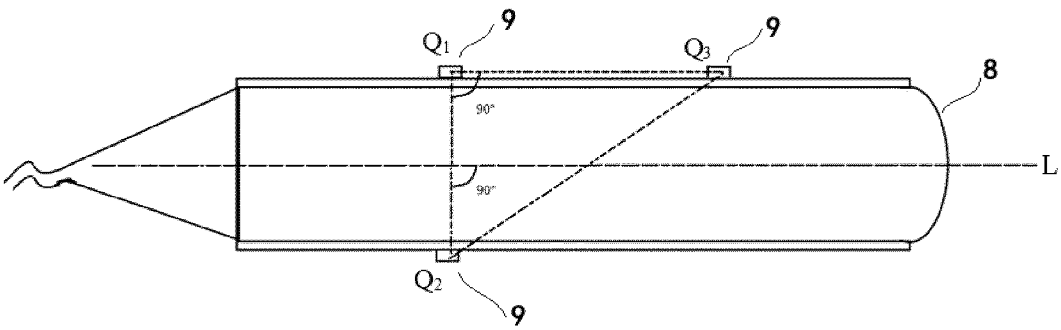


Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20100298704 A1
- US 6216029 B1
- CN 108210024 A
- CN 109805991 A